# EUROPÄISCHE PATENTSCHRIFT

(11) **EP 1 082 308 B1**
(45) Veröffentlichungstag und Bekanntmachung des Hinweises auf die Patenterteilung: **01.04.2009**
(21) Anmeldenummer: 99920710.3
(22) Anmeldetag: 20.04.1999
(51) Int. Cl.: C07D 213/82, C07D 239/42, C07D 317/60, C07D 405/12, C07D 295/155, C07D 401/04, C07D 215/50, A61K 31/495, A61K 31/505, A61K 31/455, A61K 31/44

(54) **HETEROCYCLISCH SUBSTITUIERTE AMIDE ALS CALPAINHEMMER**
HETEROCYCLICALLY SUBSTITUTED AMIDES USED AS CALPAIN INHIBITORS
AMIDES SUBSTITUES PAR VOIE HETEROCYCLIQUE ET UTILISES COMME INHIBITEURS DE CALPAINE

(30) Priorität: 20.04.1998 DE 19817462
(43) Veröffentlichungstag der Anmeldung: 14.03.2001
(73) Patentinhaber: Abbott GmbH & Co. KG, 65205 Wiesbaden (DE)
(72) Erfinder: LUBISCH, Wilfried, D-69115 Heidelberg (DE); MÖLLER, Achim, D-67269 Grünstadt (DE); TREIBER, Hans-Jörg, D-68782 Brühl (DE); KNOPP, Monika, D-67071 Ludwigshafen (DE)
(74) Vertreter: Grünecker, Kinkeldey, Stockmair & Schwanhäusser Anwaltssozietät
(86) Internationale Anmeldenummer: PCT/EP1999/002632
(87) Internationale Veröffentlichungsnummer: WO 1999/054305

(56) Entgegenhaltungen:
- EP-A- 0 520 336
- WO-A-98/25883
- WO-A-98/41506
- WO-A-99/17775
- WO-A-99/54304
- DE-A- 19 642 591

## Beschreibung

Die vorliegende Erfindung betrifft neuartige heterocyclisch substituierte Amide, die Inhibitoren von Enzyme, insbesondere Cystein-Proteasen, wie Calpain (= Calcium dependant cysteine proteases) und dessen Isoenzyme und Cathepsine, zum Beispiel B und L, darstellen.

Calpaine stellen intracelluläre, proteolytische Enzyme aus der Gruppe der sogenannten Cystein-Proteasen dar und werden in vielen Zellen gefunden. Das Enzym Calpain wird durch erhöhte Kalziumkonzentration aktiviert, wobei man zwischen Calpain I oder µ-Calpain, das durch µ-molare Konzentrationen von Kalzium-Ionen akiviert wird, und Calpain II oder m-Calpain, das durch m-molare Konzentrationen von Kalzium-Ionen aktiviert wird, unterscheidet (P.Johnson, Int.J.Biochem. 1990, 22(8), 811-22). Heute wird die Existenz weiterer Calpain-Isoenzyme postuliert (K.Suzuki et al., Biol.Chem. Hoppe-Seyler, 1995, 376(9),523-9).

Man vermutet, daß Calpaine in verschiedenen physiologischen Prozessen eine wichtige Rolle spielen. Dazu gehören Spaltungen von regulatorischen Proteinen wie Protein-Kinase C, Cytonkelett-Proteine wie MAP 2 und Spektrin, Muskelproteine, Proteinabbau in rheumatoider Arthritis, Proteine bei der Aktivierung von Plättchen, Neuropeptid-Metabolismus, Proteine in der Mitose und weitere, die in M.J.Barrett et al., Life Sci. 1991, 48, 1659-69 und K.K.Wang et al., Trends in Pharmacol.Sci., 1994, 15, 412-9 aufgeführt sind.

Bei verschiedenen pathophysiologischen Prozessen wurden erhöhte Calpain-Aktivität gemessen, zum Beispiel bei Ischämien des Herzens (z.B. Herzinfarkt), der Niere oder des Zentralnervensystems (z.B. "Stroke"), Entzündungen, Munkeldystrophien, Katarakten der Augen, Verletzungen des Zentralnervensystems (z.B.Trauma), Alzheimer Krankheit usw. (siehe K.K. Wang, oben). Man vermutet einen Zusammenhang dieser Krankheiten mit erhöhten und anhaltenden intrazellulären Kalziumspiegeln. Dadurch werden Kalzium-abhängige Prozesse überaktiviert und unterliegen nicht mehr der physiologischen Regelung. Dementsprechend kann eine Überaktivierung von Calpainen auch pathophysiologische Prozesse auslösen. Daher wurde postuliert, daß Inhibitoren der Calpain-Enzyme für die Behandlung dieser Krankheiten nützlich sein können. Verschiedene Untersuchungen bestätigen dies. So haben Seung-Chyul Hong et al., Stroke 1994, 25(3), 663-9 und R.T.Bartus et al., Neurological Res. 1995, 17, 249-58 eine neuroprotektive Wirkung von Calpain-Inhibitoren in akuten neurodegenerativen Störungen oder Ischämien, wie sie nach Hirnschlag auftreten, gezeigt. Ebenso nach experimentellen Gehirntraumata verbesserten Calpain-Inhibitoren die Erholung der auftretenden Gedächtnisleistungsdefizite und neuromotorischen Störungen (K.E.Saatman et al. Proc.Natl.Acad.Sci. USA, 1996, 93, 3428-3433). C.L.Edelstein et al., Proc.Natl.Acad.Sci. USA, 1995, 92, 7662-6, fand eine protektive Wirkung von Calpain-Inhibitoren auf durch Hypoxie geschädigten Nieren. Yoshida, Ken Ischi et al., Jap.Circ.J. 1995, 59(1), 40-8, konnten günstige Effekte von Calpain-Inhibitoren nach cardialen Schädigungen aufzeigen, die durch Ischämie oder Reperfusion erzeugt wurden. Da Calpain-Inhibitoren die Freisetzung von dem β-AP4-Protein hemmen, wurde eine potentielle Anwendung als Therapeutikum der Alzheimer Krankheit vorgeschlagen (J.Higaki et al., Neuron, 1995, 14, 651-59). Die Freisetzung von Interleukin-1α wurde ebenfalls durch Calpain-Inhibitoren gehemmt (N.Watanabe et al., Cytokine 1994, 6(6), 597-601). Weiterhin wurde gefunden, daß Calpain-Inhibitoren cytotoxische Effekte an Tumorzellen zeigen ( E.Shiba et al. 20th Meeting Int.Ass.Breast Cancer Res., Sendai Jp, 1994, 25.-28.Sept., Int.J.Oncol. 5(Supp1.), 1994, 381).

Weitere mögliche Anwendungen von Calpain-Inhibitoren sind in K.K.Wang, Trends in Pharmacol.Sci., 1994, 15, 412-8, aufgeführt. Calpain-Inhibitoren sind in der Literatur bereits beschrieben worden. Überwiegend sind dies jedoch entweder irreversible oder peptidische Inhibitoren. Irreversible Inhibitoren sind in der Regel alkylierende Substanzen und haben den Nachteil, daß sie im Organismus unselektiv reagieren oder instabil sind. So zeigen diese Inhibitoren oft unerwünschte Nebeneffekte, wie Toxizität, und sind danach in der Anwendung eingeschränkt oder nicht brauchbar. Zu den irreversiblen Inhibitoren kann man zum Beispiel die Epoxide E 64 (E.B.McGowan et al., Biochem.Biophys.Res.Commun. 1989, 158, 432-5), α-Halogenketone (H.Angliker et al., J.Med.Chem. 1992, 35, 216-20) oder Disulfide (R.Matsueda et al., Chem.Lett. 1990, 191-194) zählen.

Viele bekannte reversible Inhibitoren von Cystein-Proteasen wie Calpain stellen peptidische Aldehyde dar, insbesondere dipeptidische und tripepidische Aldehyde wie zum Beispiel Z-Val-Phe-H (MDL 28170) (S.Mehdi, Tends in Biol.Sci. 1991, 16, 150-3) und die Verbindungen aus EP 520336.

Es sind ebenfalls peptidische Keton-Derivate als Inhibitoren von Cystein-Proteasen und insbesondere Calpain gefunden worden. Allerdings sind nur Ketone, bei denen einerseits α-ständige Abgangsgruppen eine irreversible Hemmung verursachen und andererseits ein Carbonsäure-Derivat die Keto-Gruppe aktiviert, als wirksame Inhibitoren gefunden werden (siehe M.R.Angelastro et al., siehe oben; WO 92/11850; WO 92, 12140; WO 94/00095 und WO 95/00535). Jedoch sind von diesen Ketoamiden und Ketoestern bisher nur peptidische Derivate als wirksam beschrieben worden (Zhaozhao Li et al., J.Med.Chem. 1993, 36, 3472-80; S.L.Harbenson et al., J.Med.Chem. 1994, 37, 2918-29 und siehe oben M.R.Angelastro et al.).

Bisher ist noch nie gezeigt worden, daß nicht-peptidische Ketone ebenfalls potente, reversible Calpain-Inhibitoren darstellen. Ziel ist es somit, nicht-peptidische Inhibitoren zu erhalten, die sich von Carbonylverbindungen ableiten und die allgemeinen Probleme von Peptiden (metabolische Stabilität, schlechte Überwindung der Zellmembranen usw.) verbessern.

Ketobenzamide sind bereits in der Literatur bekannt. So wurde der Ketoester PhCO-Abu-COOCH₂CH₃ in WO 94/00095 und 92/11850 beschrieben. Das analoge Phenyl-Derivat Ph-CONH-CH(CH₂Ph)-CO-COCOOCH₃ wurde in M.R.Angelastro et al., J.Med.Chem. 1990, 33, 11-13 als jedoch nur schwacher Calpain-Inhibitor gefunden. Dieses Derivat ist auch in J.P.Burkhardt, Tetrahedron Lett., 1988, 3433-36 beschrieben. Die Bedeutung der heterocyclisch substituierten Amide ist jedoch bisher nie untersucht worden.

Andererseits gibt es Bemühungen, reversible nicht-peptidische Calpain-Inhibitoren zu finden. So sind in JP 8183759, JP 8183769, JP 8183771 und EP 520336 bereits von Dipeptiden abgeleitete Aldehyde beschrieben worden, wobei gesättigte carbocyclische Ringe, zum Beispiel Cyclohexane, oder gesättigte heterocyclische Ringe, zum Beispiel Piperidine, anstelle einer Aminosäure in diese peptidischen Inhibitoren eingebaut wurden und man neuartige Aldehyde als Calpain-Inhibitoren erhielt.

In DE-A-196 42 591 werden Piperidin-Ketocarbonsäure-Derivate und deren Herstellung beschrieben sowie ihre Verwendung als Inhibitoren von Cysteinproteasen.

In der vorliegenden Erfindung werden substituierte nicht-peptidische heterocyclisch substituierte Amid-Derivate beschrieben. Diese Verbindungen sind neu und zeigen überraschenderweise die Möglichkeit auf, durch Einbau von rigiden strukturellen Fragmenten potente nicht-peptidische Inhibitoren von Cystein-Proteasen, wie z.B. Calpain, zu erhalten.

Gegenstand der vorliegenden Erfindung sind heterocyclisch substituierte Amide der allgemeinen Formel I 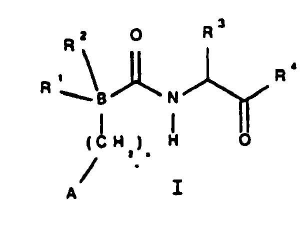 und ihre tautomeren und isomeren Formen, möglichen enantiomeren und diastereomeren Formen, sowie mögliche physiologisch verträgliche Salze, worin die Variablen folgende Bedeutung haben:
- A: Piperazin, Homopiperazin, Hexahydroazepin Piperidin und Pyrrolidin, die noch einen Rest R⁵ tragen können, bedeutet und
- B: einen Phenyl-, Pyridin-, Pyrimidin-, Pyrazin- oder Pyridazin-Ring darstellt und
- R¹ und R²: unabhängig voneinander Wasserstoff, C₁-C₆-Alkyl, verzweigt oder unverzweigt, O-C₁-C₆-Alkyl, verzweigt oder unverzweigt, OH, Cl, F, Br, J, CF₃, NO₂, NH₂, CN, COOH, COO-C₁-C₄-Alkyl, NHCO-C₁-C₄-Alkyl, NHCO-Phenyl, CONHR⁹, NHSO₂-C₁-C₄-Alkyl, NHSO₂-Phenyl, SO₂-C₁-C₄-Alkyl und SO₂-Phenyl bedeuten und R¹ und R² eine Kette -CH=CH-CH=CH- sein kann, die noch ein oder zwei Substituenten R⁶ tragen kann, und
- R³: C₁-C₆-Alkyl, verzweigt oder unverzweigt, das noch einen S-CH₃-Rest, Cyclohexyl, Cyclopentyl, Cycloheptyl, Phenyl-, Pyridyl-, Pyrimidyl-, Pyridazyl-, Pyrazyl-, Indolyl-, Thienyl oder Naphthyl-Ring tragen kann, wobei die Ringe mit maximal zwei Resten R⁷ substituiert sind und R⁷ Wasserstoff, C₁-C₄-Alkyl, verzweigt oder unverzweigt, O-C₁-C₄-Alkyl, OH, Cl, F, Br, J, CF₃, NO₂, NH₂, CN, COOH, COO-C₁-C₄-Alkyl, CONHR⁹, NHCO-C₁-C₄-Alkyl, NHCO-Phenyl, NHSO₂-C₁-C₄-Alkyl, NHSO₂-Phenyl, SO₂-C₁-C₄-Alkyl und SO₂-Phenyl bedeutet, und
- R⁴: Wasserstoff, -COR⁸ bedeutet, wobei R⁸ -OR⁹, und -NR⁹R¹⁰ sein kann und
- R⁵: Wasserstoff, C₁-C₆-Alkyl, verzweigt oder unverzweigt, das noch einen Substituenten R¹¹ tragen kann, oder R⁵ Phenyl-, Pyridyl-, Pyrimidyl-, Pyridazyl-, Pyrazinyl-, Pyrazyl-, Naphtyl, Thienyl, Piperidinyl, Pyrrolidinyl, Imidazyl-Ring sein kann, der noch einen oder zwei Substituenten R⁶ tragen kann, und
- R⁶: Wasserstoff, C₁-C₆-Alkyl, verzweigt oder unverzweigt, O-C₁-C₆-Alkyl, verzweigt oder unverzweigt, OH, Cl, F, Br, J, CF₃, NO₂, NH₂, CN, COOH, COO-C₁-C₄-Alkyl und C₁-C₄-Alkyl-NR⁹R¹³ bedeutet oder zwei Reste R⁶ eine Brücke OC(R⁹)₂O darstellen kann und
- R⁹: Wasserstoff, C₁-C₆-Alkyl, verzweigt und unverzweigt, bedeutet und
- R¹⁰: Wasserstoff, C₁-C₆-Alkyl, verzweigt oder unverzweigt, bedeutet, das noch mit einen Phenylring, der noch einen Rest R¹² tragen kann, und mit 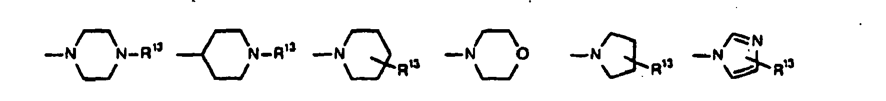 substituiert sein kann, und
- R¹¹: ein Phenyl-, Pyridyl-, Pyrimidyl-, Naphtyl, Thienyl, Furyl, Pyridazyl-, Pyrazinyl-, Pyrazyl-, Pyrrolyl-, Imidazyl-Ring sein kann, der noch einen oder zwei Substituenten R⁶ tragen kann, und
- R¹²: Wasserstoff, C₁-C₆-Alkyl, verzweigt oder unverzweigt, O-C₁-C₆-Alkyl, verzweigt oder unverzweigt, OH, Cl, F, Br, J, CF₃, NO₂, NH₂, CN, COOH, COO-C₁-C₄-Alkyl bedeutet und
- R¹³: Wasserstoff, eine C₁-C₄-Alkylkette und C₀-C₄-Alkylphenyl-, wobei der Phenylring noch ein oder zwei Resten R¹² tragen kann, bedeutet und
- x: eine Zahl 0, 1 und 2 bedeutet.

Die Verbindungen der Formel I können als Racemate oder als enantiomerenreine Verbindungen oder als Diastereomere eingesetzt werden. Werden enantiomerenreine Verbindungen gewünscht, kann man diese beispielweise dadurch erhalten, daß man mit einer geeigneten optisch aktiven Base oder Säure eine klassische Racematspaltung mit den Verbindungen der Formel I oder ihren Zwischenprodukten durchführt. Andererseits können die enantiomeren Verbindungen ebenfalls durch Einsatz von kommerziell erwerbbaren Verbindungen, zum Beispiel optisch aktiven Aminosäuren wie Phenylalanin, Tryptophan und Tyrosin, hergestellt werden.

Gegenstand der Erfindung sind auch zu Verbindungen der Formel I mesomere oder tautomere Verbindungen, beispielweise solche, bei denen die Ketogruppe der Formel I als Enol-Tautomeres vorliegt.

Die Erfindung umfaßt auch die Säureadditionssalze der Verbindungen der Formel I mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff., Birkhäuser Verlag, Basel und Stuttgart, 1966, beschrieben.

Die Herstellung der erfindungsgemäßen Amide I kann auf verschiedenen Wegen erfolgen, die in den Syntheseschemata 1, 2, 3 und 4 skizziert wurden.

Die Carbonsäureester III werden durch Umsetzung der Carbonsäureester II, wobei X eine Abgangsgruppe wie Chlorid, Bromid, Iodid oder Tosylat darstellt, mit den entsprechenden Piperazin bzw. Piperidinderivaten erhalten. Diese Reaktion wird unter üblicher Bedingungen aus dem Natrium- oder Kaliumamid der sekundären Amine in Lösungsmitteln wie THF, DMF, Toluol oder Benzol oder in Gegenwart eines Cu-Katalysators durchgeführt (s. C.R.Larock, Comprehensive Organic Transformations, VCH Publisher, 1989, S. 397). In den Beispielen, in denen B einen stickstoffhaltigen Aromaten darstellt, wird bevorzugt in Lösungsmitteln wie DMF oder THF gegebenenfalls in Gegenwart einer Base wie Triethylamin, NaH oder Kaliumcarbonat und einem Kronenether bei erhöhter Temperatur durchgeführt. In den Beispielen, in denen B einen Phenyl-Ring und A ein Piperidin-Derivat darstellt und die A-B-Bindung unter C-C-Bindungsknüpfung etabliert wird, werden Suzuki-Kupplungen (Suzuki et al., THL 1986, 27, 6369) oder Kupplungen mit Zinnorganylen durchgeführt (s. C.R.Larock, Comprehensive Organic Transformations, VCH Publisher, 1989, S. 64).

Die Karbonsäureester III werden mit Säuren oder Basen wie Lithiumhydroxid, Natriumhydroxid oder Kaliumhydroxid in wäßrigen Medium oder in Gemischen aus Wasser und organischen Lösungsmitteln wie Alkohole oder Tetrahydrofuran bei Raumtemperatur oder erhöhten Temperaturen, wie 25-100°C, in die Säuren IV überführt. Säuren IV werden mit einem α-Aminosäure-Derivat verknüpft, wobei man übliche Bedingungen benutzt, die zum Beispiel im Houben-Weyl, Methoden der organischen Chemie, 4.Aufl., E5, Kap. V, und C.R.Larock, Comprehensive Organic Transformations, VCH Publisher, 1989, Ch.9 aufgelistet sind.

Die Carbonsäuren IV werden in "aktivierte" Säure-Derivate R'-COOL überführt, wobei L eine Abgangsgruppe wie Cl, Imidazol und N-Hydroxybenzotriazol darstellt, und anschließend durch Umsatz mit einem Aminosäure-Derivat H₂N-CH(R³)-COOR in das Derivat V überführt. Diese Reaktion erfolgt in wasserfreien, inerten Lösungsmitteln wie Methylenchlorid, Tetrahydrofuran und Dimethylformamid bei Temperaturen von -20 bis +25°C.

Die Derivate V werden analog der oben beschriebenen Hydrolyse in die Ketokarbonsäuren VI überführt. In einer Dakin-West analogen Reaktion werden die Ketoester I' hergestellt, wobei nach einer Methode von ZhaoZhao Li et al. J.Med.Chem. 1993, 36, 3472-80 gearbeitet wird. Dabei werden eine Karbonsäuren wie VI bei erhöhter Temperatur (50-100°C) in Lösungsmitteln, wie zum Beispiel Tetrahydrofuran, mit Oxalsäuremonoesterchlorid umgesetzt und anschließend das so erhaltene Produkt mit Basen wie Natriumethanolat in Ethanol bei Temperaturen von 25-80°C zum erfindungsgemäßen Ketoester I' umgesetzt. Die Ketoester I' können, wie oben beschrieben, zum Beispiel zu erfindungsgemäßen Ketocarbonsäuren hydrolysiert werden.

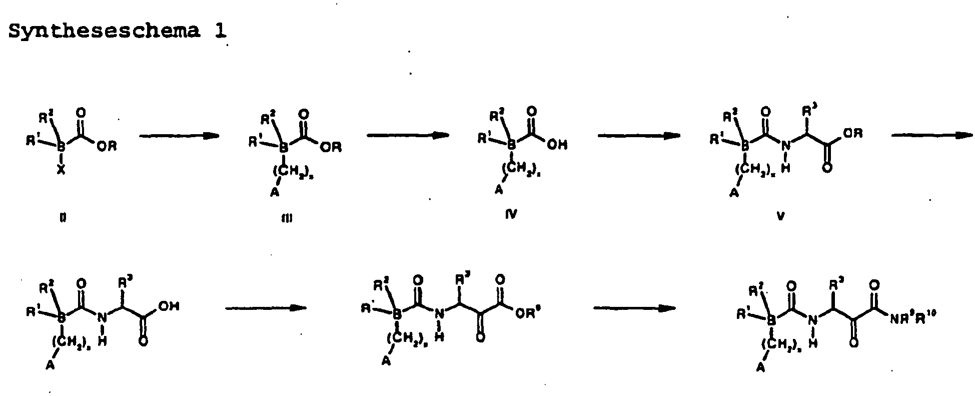

Die Umsetzung zu Ketoamiden I' erfolgt ebenfalls analog der Methode von ZhaoZhao Li et al. (s.oben). Die Ketogruppe in I' wird durch Zugabe von 1,2-Ethandithiol unter Lewissäure-Katalyse, wie zum Beispiel Bortrifluoridetherat, in inerten Lösungsmitteln, wie Methylenchlorid, bei Raumtemperatur geschützt, wobei ein Dithian anfällt. Diese Derivate werden mit Aminen R⁸-H in polaren Lösungsmitteln, wie Alkohole, bei Temperaturen von 0-80°C umgesetzt, wobei die Ketoamide I (R⁸ = NR⁹R¹⁰) anfallen.

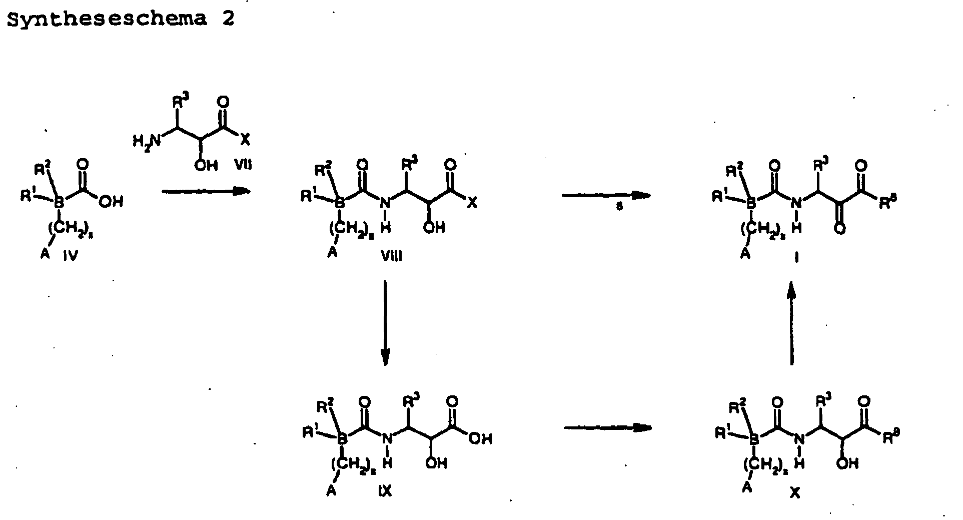

Eine alternative Methode ist im Schema 2 dargestellt. Die Carbonsäuren IV werden mit Aminohydroxykarbonsäure-Derivaten VII (Herstellung von IV siehe S.L.Harbenson et al., J.Med.Chem. 1994, 37, 2918-29) unter üblichen Peptid-Kupplungs-Methoden (siehe oben, Houben-Weyl) umgesetzt, wobei Amide VIII anfallen. Diese Alkohol-Derivate VIII können zu den erfindungsgemäßen Ketokarbonsäure-Derivaten I oxidiert werden. Dafür kann man verschiedene übliche Oxidationsreaktionen (siehe C.R.Larock, Comprehensive Organic Transformations, VCH Publisher, 1989, Seite 604 f.) wie zum Beispiel Swern- und Swern-analoge Oxidationen, bevorzugt Dimethylsulfoxid/Pyridin-Schwefeltrioxid-Komplex in Lösungsmitteln wie Methylenchorid oder Tetrahydrofuran, gegebenenfalls unter Zusatz von Dimethylsulfoxid, bei Raumtemperatur oder Temperaturen von -50 bis 25°C, (T.T.Tidwell, Synthesis 1990, 857-70) oder Natriumhypochlorid/TEMPO (S.L.Harbenson et al., siehe oben), benutzen.

Wenn VIII α-Hydroxyester darstellen (X = O-Alkyl), können diese zu Karbonsäuren IX hydrolysiert werden, wobei analog zu den obigen Methoden gearbeitet wird, bevorzugt aber mit Lithiumhydroxid in Wasser/Tetrahydrofuran-Gemischen bei Raumtemperatur. Die Herstellung von anderen Estern oder Amiden X erfolgt durch Umsetzung mit Alkoholen oder Aminen unter bereits beschriebenen Kupplungsbedingungen. Das Alkohol-Derivat X kann erneut zu erfindungsgemäßen Ketokarbonsäure-Derivaten I oxidiert werden.

Die erfindungsgemäßen Aldehyde der Formel I (R⁵ = Wasserstoff) können analog dem Syntheseschema 3 hergestellt werden.

Carbonsäure-Derivate IV werden mit geeigneten Aminoalkoholen XI zu den entsprechenden Amiden XII verknüpft. Dabei benutzt man übliche Peptid-Kupplungs-Methoden, die entweder im C.R.Larock, Comprehensive Organic Transformations, VCH Publisher, 1989, Seite 972f. oder im Houben-Weyl, Methoden der organischen Chemie, 4.Aufl., E5, Kap.V aufgeführt sind. Bevorzugt arbeitet man mit "aktivierten" Säurederivaten von III, wobei die Säuregruppe COOH in eine Gruppe COL überführt wird. L stellt eine Abgangsgruppe wie zum Beispiel Cl, Imidazol und N-Hydroxybenzotriazol dar. Diese aktivierte Säure wird anschließend mit Aminen zu den Amiden XII umgesetzt. Die Reaktion erfolgt in wasserfreien, inerten Lösungsmitteln wie Methylenchlorid, Tetrahydrofuran und Dimethylformamid bei Temperaturen von -20 bis +25°C.

Diese Alkohol-Derivate XII können zu den erfindungsgemäßen Aldehyd-Derivaten I oxidiert werden. Dafür kann man verschiedene übliche Oxidationsreaktionen (siehe C.R.Larock, Comrenhensiye Organic Transformations, VCH Publisher, 1989, Seite 604 f.) wie zum Beispiel Swern- und Swern-analoge Oxidationen (T.T.Tidwell, Synthesis 1990, 857-70), Natriumhypochlorid/TEMPO (S.L.Harbenson et al., siehe oben) oder Dess-Martin (J.Org.Chem. 1983, 48, 4155) benutzen. Bevorzugt arbeitet man hier in inerten aprotischen Lösungsmitteln wie Dimethylformamid, Tetrahydrofuran oder Methylenchorid mit Oxidationsmitteln wie DMSO/Pyridin x SO₃ oder DMSO/Oxalylchorid bei Temperaturen von -50 bis +25°C, je nach Methode (siehe obige Literatur).

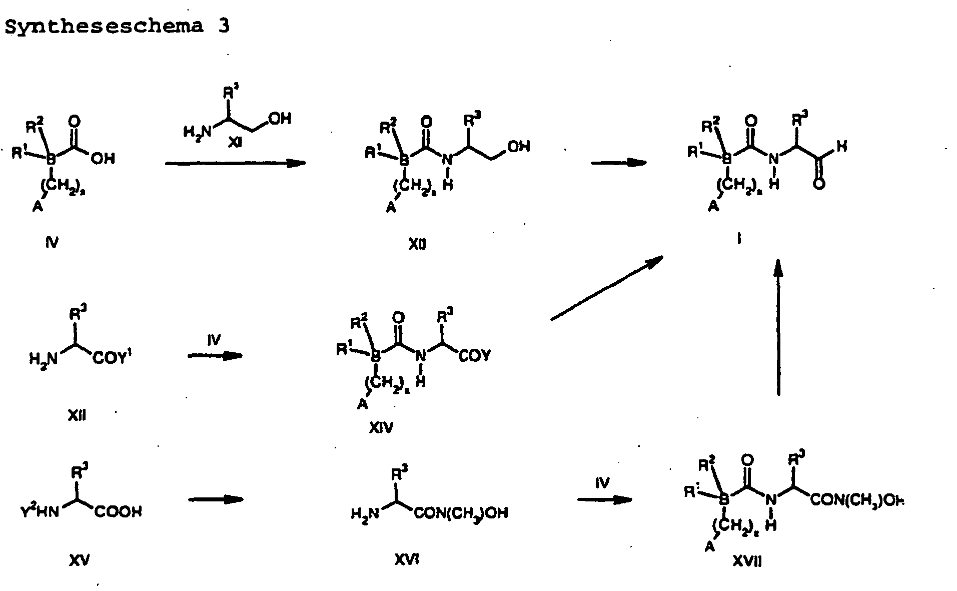

Alternativ kann man Carbonsäuren IV auch mit den Estern oder Amiden XIII umsetzen. Die resultierenden Amide XIV können durch Reduktion in die erfindungsgemäßen Aldehyde I überführt werden. Diese Verfahren sind in R.C.Larock, Comprehensive Organic Transformations, VCH Publisher, 1989, Seite 619-26 aufgelistet.

Analog zum letzten Verfahren kann man die Benzoesäure IV mit den Aminohydroxamsäure-Derivaten XVI zu den Amiden XVII umsetzten. Dabei bedient man sich der gleichen Reaktionsführung wie bei der Darstellung von XII. Die Hydroxam-Derivate XVI sind auch aus den geschützten Aminosäuren XV durch Umsatz mit Hydroxylamin erhältlich. Dabei benutzt auch hier die bereits beschriebenen Amidherstellungsverfahren. Die Abspaltung der Schutzgruppe Y², zum Beispiel Boc, erfolgt nach Standardverfahren, zum Beispiel mit Trifluoressigsäure in Methylenchlorid. Die so erhaltenen Benzamidhydroxamsäuren XVII können durch Reduktion in die erfindungsgemäßen Aldehyde I umgewandelt werden. Dabei benutzt man zum Beispiel Lithiumaluminiumhydrid als Reduktionsmittel bei Temperaturen von -60 bis 0°C in inerten Lösungsmitteln wie Tetrahydrofuran oder Ether.

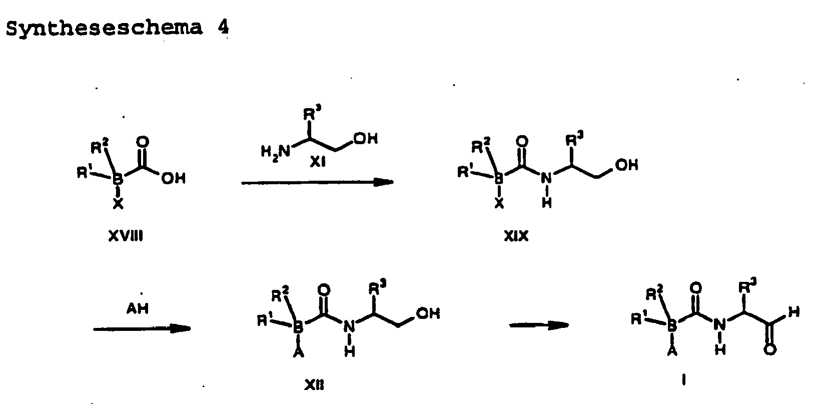

Eine weitere Alternative, die für die Beispiele geeignet ist, bei denen A ein Piperazinderivat und B ein Pyridinderivat darstellt, ist in Schema 4 beschrieben. Die einzelnen Syntheseschritte dieses sehr kurzen Weges sind bereits in den vorherigen Ausführungen ausführlich erläutert worden.

In der vorliegenden Erfindung enthaltenen Ketobenzamide I stellen Inhibitoren von Cystein-Proteasen dar, insbesondere Cystein-Proteasen wie die Calpaine I und II und Cathepsine B bzw. L. Die inhibitorische Wirkung der Ketobenzamide I wurde mit in der Literatur üblichen Enzymtests ermittelt, wobei als Wirkmaßstab eine Konzentration des Inhibitors ermittelt wurde, bei der 50% der Enzymaktivität gehemmt wird (= IC₅₀). Zum Teil wurde auch ein Kᵢ-Wert ermittelt. Die Benzamide I wurden in dieser weise auf Hemmwirkung von Calpain I, Calpain II und Cathepsin B gemessen.

### Cathepsin B-Test

Die Cathepsin B-Hemmung wurde analog einer Methode von S.Hasnain et al., J.Biol.Chem. 1993, 268, 235-40 bestimmt.

Zu 88µL Cathepsin B (Cathepsin B aus menschlicher Leber (Calbiochem), verdünnt auf 5 Units in 500µM Puffer) werden 2µL einer Inhibitor-Lösung, hergestellt aus Inhibitor und DMSO (Endkonzentrationen: 100µM bis 0,01µM). Dieser Ansatz wird für 60 Minuten bei Raumtemperatur (25°C) vorinkubiert und anschließend die Reaktion durch Zugabe von 10µL 10mM Z-Arg-Arg-pNA (in Puffer mit 10% DMSO) gestartet. Die Reaktion wird 30 Minuten bei 405nM im Mikrotiterplattenreader verfolgt. Aus den maximalen Steigungen werden anschließend die IC₅₀'s bestimmt.

### Calpain I und II Test

Die Testung der inhibitorischen Eigenschaften von Calpain-Inhibitoren erfolgt in Puffer mit 50 mM Tris-HCl, pH 7,5 ; 0,1 M NaCl; 1 mM Dithiotreithol; 0,11 mM CaCl₂, wobei das fluorogene Calpainsubstrat Suc-Leu-Tyr-AMC ( 25 mM gelöst in DMSO, Bachem/Schweiz) verwendet wird. Humanes µ-Calpain wird aus Erythrozyten isoliert und nach mehreren chromatographischen Schritten (DEAE-Sepharose, Phenyl-Sepharose, Superdex 200 und Blue-Sepharose) erhält man Enzym mit einer Reinheit >95%, beurteilt nach SDS-PAGE, Western Blot Analyse und N-terminaler Sequenzierung. Die Fluoreszenz des Spaltproduktes 7-Amino-4-methylcoumarin (AMC) wird in einem Spex-Fluorolog Fluorimeter bei λₑₓ = 380 nm und λₑₘ = 460 nm verfolgt. In einem Meßbereich von 60 min. ist die Spaltung des Substrats linear und die autokatalytische Aktivität von Calpain gering, wenn die Versuche bei Temperaturen von 12° C durchgeführt werden. Die Inhibitoren und das Calpainsubstrat werden in den Versuchsansatz als DMSO-Lösungen gegeben, wobei DMSO in der Endkonzentration 2% nicht überschreiten soll.

In einem Versuchsansatz werden 10 µl Substrat (250µM final) und anschließend 10 µl an µ-Calpain (2µg/ml final, d.h.18 nM) in eine 1 ml Küvette gegeben, die Puffer enthält. Die Calpain-vermittelte Spaltung des Substrats wird für 15 - 20 min. gemessen. Anschließend Zugabe von 10 µl Inhibitor (50 - 100 µM Lösung in DMSO) und Messung der Inhibition der Spaltung für weitere 40 min.

Kᵢ -Werte werden nach der klassischen Gleichung für reversible Hemmung bestimmt:

Ki = I / (v0/vi) - 1 ; wobei I= Inhibitorkonzentration, v0 = Anfangsgeschwindigkeit vor Zugabe des Inhibitors; vi = Reaktionsgeschwindigkeit im Gleichgewicht.

Die Geschwindigkeit wird errechnet aus v = Freisetzung AMC/ Zeit d.h. Höhe /Zeit.

Calpain ist eine intrazelluläre Cysteinprotease. Calpain-Inhibitoren müssen die Zellmembran passieren, um den Abbau von intrazellulären Pproteinen durch Calpain zu verhindern. Einige bekannte Calpain-Inhibitoren, wie zum Beispiel E 64 und Leupeptin, überwinden die Zellmembranen nur schlecht und zeigen dementsprechend, obwohl sie gute Calpain-Inhibitoren darstellen, nur schlechte Wirkung an Zellen. Ziel ist es, Verbindungen mit besserer Membrangängigkeit zu finden. Als Nachweis der Membrangängigkeit von Calpain-Inhibitoren wurden humane Blutplättchen benutzt.

### Calpain-vermittelter Abbau der Tyrosinkinase pp60src in Plättchen

Nach der Aktivierung von Plättchen wird die Tyrosinkinase pp60src durch Calpain gespalten. Dies wurde von Oda et al. in J. Biol. Chem., 1993, Vol 268, 12603-12608 eingehend untersucht. Hierbei wurde gezeigt, daß die Spaltung von pp60src durch Calpeptin, einen Inhibitor von Calpain, verhindert werden kann. In Anlehnung an diese Publikation wurde die zellulare Effektivität unserer Substanzen getestet. Frisches humanes, mit Zitrat versetztes Blut wurde 15 min. bei 200g zentrifugiert. Das Plättchen-reiche Plasma wurde gepoolt und mit Plättchenpuffer 1:1 verdünnt (Plättchenpuffer: 68 mM NaCl, 2,7 mM KCl, 0,5 mM MgCl₂ x 6 H₂O, 0,24 mM NaH₂PO₄ x H₂O, 12 mM NaHCO₃, 5,6 mM Glukose, 1 mM EDTA, pH 7,4). Nach einem Zentrifugations-und Waschschritt mit Plättchenpuffer wurden die Plättchen auf 10⁷Zellen/ml eingestellt. Die Isolierung der humanen Plättchen erfolgte bei RT.

Im Testansatz wurden isolierte Plättchen (2 x 10⁶) mit unterschiedlichen Konzentrationen an Inhibitoren (gelöst in DMSO) für 5 min. bei 37°C vorinkubiert. Anschließend erfolgte die Aktivierung der Plättchen mit 1µM Ionophor A23187 und 5 mM CaCl₂. Nach 5 min. Inkubation wurden die Plättchen kurz bei 13000 rpm zentrifugiert und das Pellet in SDS-Probenpuffer aufgenommen (SDS-Probenpuffer: 20 mM Tris-HCl, 5 mM EDTA, 5 mM EGTA, 1 mM DTT, 0,5 mM PMSF, 5 µg/ml Leupeptin, 10 µg/ml Pepstatin, 10% Glycerin und 1% SDS). Die Proteine wurden in einem 12%igen Gel aufgetrennt und pp60src und dessen 52-kDa und 47-kDa Spaltprodukte durch Western-Blotting identifiziert. Der verwendete polyklonale Kaninchen-Antikörper Anti-Cys-src (pp60^{c-src} ) wurde von der Firma Biomol Feinchemikalien (Hamburg) erworben. Dieser primäre Antikörper wurde mit einem HRP-gekoppelten zweiten Antikörper aus der Ziege (Boehringer Mannheim, FRG) nachgewiesen. Die Durchführung des Western-Blotting erfolgte nach bekannten Methoden.

Die Quantifizierung der Spaltung von pp60src erfolgte densitometrisch, wobei als Kontrollen nicht-aktivierte (Kontrolle 1: keine Spaltung) und mit Ionophor- und Kalzium-behandelte Plättchen (Kontrolle 2: entspricht 100% Spaltung) verwendet wurden. Der ED₅₀ -Wert entspricht der Konzentration an Inhibitor, bei der die Intensität der Farbreaktion um 50% reduziert wird.

### Glutamat induzierter Zelltod an corticalen Neuronen

Der Test wurde, wie bei Choi D. W., Maulucci-Gedde M. A. and Kriegstein A. R., "Glutamate neurotoxicity in cortical cell culture". J. Neurosci. 1989, 7, 357-368, durchgeführt.

Aus 15 Tage alten Mäuseembryos wurden die Cortexhälften präpariert und die Einzelzellen enzymatisch (Trypsin) gewonnen. Diese Zellen (Glia und corticale Neuronen) werden in 24 Well-Platten ausgesät. Nach drei Tagen (Laminin beschichteten Platten) oder sieben Tagen (Ornithin beschichteten Platten) wird mit FDU (5-Fluor-2-Desoxyuridine) die Mitosebehandlung durchgeführt. 15 Tage nach der Zellpräparation wird durch Zugabe von Glutamat (15 Minuten) der Zelltod ausgelöst. Nach der Glutamatentfernung werden die Calpaininhibitoren zugegeben. 24 Stunden später wird durch die Bestimmung der Lactatdehydrogenase (LDH) im Zellkulturüberstand die Zellschädigung ermittelt.

Man postuliert, daß Calpain auch eine Rolle im apoptotischen Zelltod spielt ( M.K.T.Squier et al. J.Cell.Physiol. 1994, 159, 229-237; T.Patel et al. Faseb Journal 1996, 590, 587-597 ). Deshalb wurde in einem weiteren Modell in einer humanen Zellinie der Zelltod mit Kalzium in Gegenwart eines Kalziumionophors ausgelöst. Calpain-Inhibitoren müssen in die Zelle gelangen und dort Calpain hemmen, um den ausgelösten Zelltod zu verhindern.

### Kalzium-vermittelter Zelltod in NT2 Zellen

In der humanen Zellinie NT2 (Stratagene GmbH) läßt sich durch Kalzium in Gegenwart des Ionophors A 23187 der Zelltod auslösen. 10⁵ Zellen/well wurden in Mikrotiterplatten 20 Stunden vor dem Versuch ausplattiert. Nach diesem Zeitraum wurden die Zellen mit verschiedenen Konzentrationen an Inhibitoren in Gegenwart von 2,5 µM Ionophor und 5 mM Kalzium inkubiert. Dem Reaktionsansatz wurden nach 5 Stunden 0,05 ml XTT (Cell Proliferation Kit II, Boehringer Mannnheim ) hinzugegeben. Die optische Dichte wird ungefähr 17 Stunden später, entsprechend den Angaben des Herstellers, in dem Easy Reader EAR 400 der Firma SLT bestimmt. Die optische Dichte, bei der die Hälfte der Zellen abgestorben sind, errechnet sich aus den beiden Kontrollen mit Zellen ohne Inhibitoren, die in Abwesenheit und Gegenwart von Ionophor inkubiert wurden.

Bei einer Reihe von neurologischen Krankheiten oder psychischen Störungen treten erhöhte Glutamat-Aktivitäten auf, die zu Zuständen von Übererregungen oder toxischen Effekten im zentralen Nervensystem (ZNS) führen. Glutamat vermittelt seine Effekte über verschiedene Rezeptoren. Zwei von diesen Rezeptoren werden nach den spezifischen Agonisten als NMDA-Rezeptor und AMPA-Rezeptor klassifiziert. Substanzen, die diese von Glutamat ausgelöstenen Effekte abschwächen, können somit zur Behandlung dieser Krankheiten eingesetzt werden, insbesondere zur therapeutischen Anwendung gegen neurodegenerativen Krankheiten wie Huntington und Parkinson, neurotoxischen Störungen nach Hypoxie, Anoxie, Ischämie und nach Läsionen, wie sie nach Schlaganfall und Trauma auftreten, oder auch als Antiepileptika (vgl. Arzneim.Forschung 1990, 40, 511-514; TIPS, 1990, 11, 334-338; Drugs of the Future 1989, 14, 1059-1071).

### Schutz gegen zerebrale Übererregung durch exzitatorische Aminosäuren (NMDA- bzw. AMPA-Antagonismus an der Maus)

Durch intrazerebrale Applikation von exzitatorischen Aminosäuren EAA (Excitatory Amino Acids) wird eine so massive Übererregung induziert, daß diese in kurzer Zeit zu Krämpfen und zum Tod der Tiere führt. Durch systemische, z.B. intraperitoneale, Gabe von zentral-wirksamen Wirkstoffen (EAA-Antagonisten) lassen sich diese Symptome hemmen. Da die exzessive Aktivierung von EAA-Rezeptoren des Zentralnervensystems in der Pathogenese verschiedener neurologischer Erkrankungen eine bedeutende Rolle spielt, kann aus dem nachgewiesenen EAA-Antagonismus in vivo auf eine mögliche therapeutische Verwendbarkeit der Substanzen gegen derartige ZNS-Erkrankungen geschlossen werden. Als Maß für die Wirksamkeit der Substanzen wurde ein ED₅₀-Wert bestimmt, bei dem 50% der Tiere durch eine festgelegte Dosis von entweder NMDA oder AMPA durch die vorangegangene ip.-Gabe der Meßsubstanz syptomfrei werden.

Es wurde bereits gezeigt, daß auch Calpain-Inhibitoren in Zellkulturen protektive Wirkung gegen den durch EAA ausgelösten Zelltod zeigen (H.Cauer et al., Brain Research 1993, 607, 354-356; Yu Cheg and A.Y. Sun, Neurochem. Res. 1994, 19, 1557-1564). Die in dieser Anmeldung enthaltenen Calpain-Inhibitoren sind überrascehnderweise sogar gegen die durch EAA (z.B. NMDA oder AMPA) ausgelösten Krämpfe *in vivo* (Maus) wirksam und zeigen damit eine mögliche therapeutische Verwendung in den oben genannten ZNS-Erkrankungen an.

Die heterozyklisch substituierten Amide I stellen Inhibitoren von Cystein-Derivate wie Calpain I bzw. II und Cathepsin B bzw. L dar und können somit zur Bekämpfung von Krankheiten, die mit einer erhöhten Enzymaktivität der Calpain-Enzyme oder Cathepsin-Enzyme verbunden sind, dienen. Die vorliegenden Amide I können danach zur Behandlung von neurodegenerativen Prozessen, die nach Ischämie, Trauma, Subarachnoidal-Blutungen und Schlaganfall auftreten, und von neurodegenerativen Krankheiten wie multipler Infarkt-Dementia, Alzheimer Krankheit, Huntington Krankheit und von Epilepsien und weiterhin zur Behandlung von Schädigungen des Herzens nach cardialen Ischämien, Schädigungen und Reperfusion nach Gefäßverschlüssen, Schädigungen der Nieren nach renalen Ischämien, Skelettmuskelschädigungen, Muskeldystrophien, Schädigungen, die durch Proliferation der glatten Muskelzellen entstehen, coronaren Vasospasmen, cerebralen Vasospasmen, Katarakten der Augen, Restenose der Blutbahnen nach Angioplastie dienen. Zudem können die Amide I bei der Chemotherapie von Tumoren und deren Metastasierung nützlich sein und zur Behandlung von Krankheiten, bei denen ein erhöhter Interleukin-1-Spiegel auftritt, wie bei Entzündungen und rheumatischen Erkrankungen, dienen.

Die erfindungsgemäßen Arzneimittelzubereitungen enthalten neben den üblichen Arneimittelhilfstoffen eine therapeutisch wirksame Menge der Verbindungen I.

Für die lokale äußere Anwendung, zum Beispiel in Puder, Salben oder Sprays, können die Wirkstoffe in den üblichen Konzentrationen enthalten sein. In der Regel sind die Wirkstoffe in einer Menge von 0,001 bis 1 Gew.-%, vorzugsweise 0,001 bis 0,1 Gew.-% enthalten.

Bei der inneren Anwendung werden die Präperationen in Einzeldosen verabreicht. In einer Einzeldosis werden pro kg Körpergewicht 0,1 bis 100 mg gegeben. Die Zubereitung kann täglich in einer oder mehreren Dosierungen je nach Art und Schwere der Erkrankungen verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes hydriertes Ricinusöl, Polyacrylsäure, Polyethylenglykol, Polyethylenglykostearat, ethoxylierte Fettalkohole, Paraffinöl, Vaseline und Wollfett, verwendet werden. Für die innere Anwendung eignen sich zum Beispiel Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wikstoff verträglich. Die Herstellung der Arzneimittelzubereitungen erfolgt in üblicher Weise, zum Beispiel durch Vermischung des Wirkstoffes mit anderen üblichen Trägerstoffen und Verdünnungsmitteln.

Die Arzneimittelzubereitungen können in verschiedenen Applikationsweisen verbreicht werden, zum Beispiel peroral, parenteral wie intravenös durch Infusion, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

### Beispiele

### Beispiel 1

### 2-(4-(Pyrid-4-yl)piperazin-1-yl)nikotinsäure-N-(3-phenylpropan-1-al-2-yl)amid

a) 2-(4-(Pyrid-4-yl)piperazin-1-yl)nikotinsäuremethylester
   3.4 g Chlornikotinsäuremethylester, 5.5 g Kaliumcarbonat, 3.3 g 4-Pyridylpiperazin und eine Spatelspitze 18-Krone-6 wurden in 75 ml DMF 5 h bei 100 °C erhitzt und anschließend 60 h bei Raumtemperatur gerührt. Das überschüssige Kaliumcarbonat wurde abfiltriert, das Filtrat wurde eingeengt und der Rückstand zwischen Wasser und Essigester verteilt. Nach Trocknen der organischen Phase über Magnesiumsulfat und Einengen des Lösungsmittels fielen 3.9 g (82 %) des Produkts an.
b) 2-(4-(Pyrid-4-yl)pipezazin-1-yl)nikotinsäure
   5.6 g der Zwischenverbindung 1a wurden in 100 ml THF vorgelegt und mit 1.4 g LiOH in 50 ml Wasser bei Raumtemperatur versetzt. Die trübe Lösung wurde durch Zugabe von 10 ml MeOH geklärt. Die Reaktionsmischung wurde 12 h bei Raumtemperatur gerührt und mit einer äquimolaren Menge 1 M HCl hydrolysiert. Die Reaktionsmischung wurde bis zur Trockne eingeengt und der Rückstand in Methanol/Toluol aufgenommen. Nach Entfernen des Lösungsmittels fielen 8.2 g des noch salzhaltigen Produkts an, die ohne weitere Reinigung in die nächste Stufe eingesetzt wurden.
c) 2-(4-(Pyrid-4-yl)piperazin-1-yl)nikotinsäure-N-(3-phenylpropan-1-ol-2-yl)amid
   8.2 g der Zwischenverbindung 1b und 5.2 g Triethylamin wurden in 200 ml Methylenchlorid und 50 ml DMF vorgelegt. Es wurden 5 g Natriumsulfat zugegeben und 30 min gerührt. 2.6 g Phenylalaninol, 2.3 g HOBT und 3.6 g EDC wurden nacheinander bei 0 °C zugegeben und über Nacht bei Raumtemperatur nachgerührt. Die Reaktionsmischung wurde auf destilliertes Wasser geschüttet, mit NaHCO₃ alkalisch gestellt, mit NaCl gesättigt und dreimal mit 100 ml Methylenchlorid extrahiert. Die organischen Phasen wurden zweimal mit Wasser gewaschen und über Magnesiumsulfat getrocknet. Nach Einengen des Lösungsmittels fielen 0.61 g (8%) des Produkt an.
d) 2-(4-(Pyrid-4-yl)piperazin-1-yl)nikotinsäure-N-(3-phenylpropan-1-al-2-yl)amid
   0.6 g der Zwischenverbindung 1c wurden in Gegenwart von 0.6 g Triethylamin in 20 ml DMSO vorgelegt und mit 0.9 g SO₃-Pyridin-Komplex versetzt. Es wurde über Nacht bei Raumtemperatur gerührt. Der Ansatz wurde auf 250 ml destilliertes Wasser geschüttet, mit NaHCO₃ alkalisch getsellt, mit NaCl gesättigt, mit 100 ml Methylenchlorid extrahiert und über Magnesiumsulfat getrocknet. Nach dem Einengen des Lösungsmittels wurde der Rückstand in THF gelöst und mit HCl in Dioxan das Hydrochlorid ausgefällt, Das Produkt wurde abgesaugt und mehrfach mit Ether gewaschen.
   Ausbeute: 0.08 g (11 %)
   MS: m/e = 488 (M⁺)

### Beispiel 2

### 2-(4-Methylpiperazin-1-yl)nikotinsäure-N-(3-phenylpropan-1-al-2-yl)amid

a) 2-(4-Methylpiperazin-1-yl)nikotinsäuremethylester
   3.4 g Chlornikotinsäuremethylester, 5.5 g Kaliumcarbonat und
   2.0 g N-Methylpiperazin wurden in 50 ml DMF analog Beispiel 1a umgesetzt, wobei 3.9 g (82 %) des Produkts anfielen.
b) 2-(4-Methylpiperazin-1-yl)nikotinsäure
   3.5 g der Zwischenverbindung 2a in 100 ml THF wurden analog Beispiel 1b mit 1.1 g LiOH in 50 ml Wasser und 10 ml MeOH bei Raumtemperatur umgesetzt, wobei 6.1 g des noch salzhaltigen Produkts anfielen, die ohne weitere Reinigung in die nächste Stufe eingesetzt wurden.
c) 2-(4-Methylpiperazin-1-yl)nikotinsäure-N-(3-phenylpropan-1-ol-2-yl)amid
   6.1 g Zwischenbverbindung 2b wurden analog Beispiel 1c mit
   2.1 g Phenylalaninol in 100 ml DMF in Gegenwart von 2.7 g EDC, 1.9 g HOBT und 5.0 g Triethylamin umgesetzt, wobei 1.2 g (23 %) des Produkts anfielen.
d) 2-(4-Methylpiperazin-1-yl)nikotinsäure-N-(3-phenylpropan-1-al-2-yl)amid
   1.0 g der Zwischenverbindung 2c wurden analog Beispiel 1d mit
   1.9 g SO₃-Pyriclin-Komplex in 25 ml DMSO in Gegenwart von 1.2 ml Triethylamin oxidiert, wobei 0.7 g (60 %) des Produkts in Form des Hydrochlorids anfielen.
   ¹H-NMR (d₆-DMSO): δ = 2.5 (3H), 2.7-3.9 (10H), 4.7 (1H), 6.9-7.9 (6H), 8.1-8.2 (2H), 9.7 (1H), 10.9 (1H) ppm.

### Beispiel 3

### 2-(4-(Pyrimid-2-yl)piperazin-1-yl)nikotinsäure-N-(3-phenylpropan-1-al-2-yl)amid

a) 2-(4-(Pyrimid-2-yl)piperazin-1-yl)nikotinsäuremethylester
   3.4 g Chlornikotinsäuremethylester, 11.1 g Kaliumcarbonat und
   4.7 g N-(2-Pyrimidyl)piperazin Dihydrochlorid wurden in 75 ml DMF analog Beispiel 1a umgesetzt, wobei 4.6 g (78 %) des Produkts anfielen.
b) 2-(4 -(Pyrimid-2-yl)piperazin-1-yl)nikotinsäure
   4.3 g der Zwischenverbindung 3a in 100 ml THF wurden analog Beispiel 1b mit 1.0 g LiOH in 50 ml Wasser und 10 ml MeOH bei Raumtemperatur umgesetzt, wobei 6.1 g des noch salzhaltigen Produkts anfielen, die ohne weitere Reinigung in die nächste Stufe eingesetzt wurden.
c) 2-(4-(Pyrimid-2-yl)piperazin-1-yl)nikotinsäure-N-(3-phenylpropan-1-ol-2-yl) amid
   3.8 g Zwischenbverbindung 3b wurden analog Beispiel 1c in 200 ml Methylenchlorid und 50 ml DMF in Gegenwart von 4.0 g Triethylamin und 5 g Natriumsulfat vorgelegt und nacheinander mit 2.0 g Phenylalaninol, 2.7 g EDC und 1.8 g HOBT versetzt, wobei 1.5 g (28 %) des Produkts anfielen.
d) 2-(4-(Pyrimid-2-yl)piperazin-1-yl)nikotinsäure-N-(3-phenylpropan-1-al-2-yl) amid
   1.3 g der Zwischenverbindung 3c wurden analog Beispiel 1d mit
   1.9 g SO₃-Pyridin-Komplex in 20 ml DMSO in Gegenwart von 1.2 ml Triethylamin oxidiert, wobei 0.7 g (48 %) des Produkts in Form des Hydrochlorids anfielen.
   ¹H-NMR (d₆-DMSO): δ =2.9 (1H), 3.2-3.9 (9H), 4.8 (1H), 6.7 (1H), 7.0-7.4 (5H), 7.8 (1H), 8.2 (1H), 8.3 (4H), 9.1 (1H), 9.6 (1H) ppm.

### Beispiel 4

### 2-(4-Henzylpiperazin-1-yl)nikotinsäure-N-(3-phenylpropan-1-al-2-yl)amid

a) 2-(4-Henzylpiperazin-1-yl)nikotinsäuremethylester 3.4 g 2-Chlornikotinsäuremethylester, 5.5 g Kaliumcarbonat und 3.5 g N-Benzylpiperazin wurden in 75 ml DMF analog Beispiel 1a umgesetzt, wobei 6.2 g (100 %) des Produkts anfielen.
b) 2-(4-Henzylpiperazin-1-yl)nikotinsäure
   6.2 g der Zwischenverbindung 4a in 100 ml THF wurden analog Beispiel 1b mit 1.4 g LiOH hydrolysiert, wobei 8.70 g des noch salzhaltigen Produkts anfielen, die ohne weitere Reinigung direkt in die nächste Stufe eingesetzt wurden.
c) 2-(A-Henzylpiperazin-1-yl)nikotinsäure-N-(3-phenylpropan-1-ol-2-yl)amid
   5.6 g der Zwischenverbindung 4b wurden analog Beispiel 1c in Gegenwart von 5.7 g Triethylamin und 5 g Natriumsulfat in 200 ml Methylenchlorid und 50 ml DMF vorgelegt, und nacheinander mit 2.9 g Phenylalaninol, 2.6 g HOBT und 4.0 g EDC versetzt, wobei 1.0 g (12 %) des Produkts anfielen.
d) 2-(4-Benzylpiperazin-1-yl)nikotinsäure-N-(3-phenylpropan-1-al-2-yl)amid
   0.9 g der Zwischenverbindung 4c wurden analog Beispiel 1d in 20 ml DMSO in Gegenwart von 0.8 g Triethylamin mit 1.2 g SO₃-Pyridin-Komplex oxidiert, wobei 0.7 g (72 %) des Produkts in Form des Hydrochlorids anfielen.
   ¹H-NMR (d₆-DMSO): δ = 2.8-3.5 (8H), 3.6 (1H), 3.8 (1H), 4.3 (2H), 4.7 (1H), 6.9-7.8 (12H), 8.3 (1H), 9.1 (1H), 9.7 (1H), 11.7 (2H) ppm.

### Beispiel 5

### 2-(4-(Picol-2-yl)piperazin-1-yl)nikotinsäure-N-(3-phenylpropan-1-al-2-yl) amid

a) 2-(4-(Picol-2-yl)piperazin-1-yl)nikotinsäuremethylester
   2.6 g 2-Chlornikotinsäuremethylester wurden in 50 ml DMF analog Beispiel 1a mit 2.7 g 2-Picolylpiperazin in Gegenwart von 4.2 g Kaliumcarbonat umgesetzt, wobei 1.7 g (36 %) des Produkts anfielen.
b) 2-(4-(Picol-2-yl)piperazin-1-yl)nikotinsäure
   1.7 g der Zwischenverbindung 5a wurden mit 20 ml 2 M NaOH 1 h auf 80 °C erhitzt. Sobald das gesamte Edukt gelöst ist, wurde die Reaktionsmischung eingeengt, der Rückstand mit 15 ml 4M HCl-Lösung in Dioxan versetzt und nach Entfernen des Dioxan der Rückstand in Methanol aufgenommen, vom Niederschlag abfiltriert. Das Filtrat wurde mit Methylenchlorid versetzt, über Natriumsulfat getrocknet, filtriert und eingeengt, wobei 2.9 g des noch salzhaltigen Produkts anfielen, die ohne weitere Reinigung in die nächste Stufe eingesetzt wurden.
c) 2-(4-(Picol-2-yl)piperazin-1-yl)nikotinsäure-N-(3-phenylpropan-1-ol-2-yl)amid
   3.0 g der Zwischenverbindung 5b wurden in 50 ml Methylenchlorid und 4 ml DMF in Gegenwart von 4.5 ml Triethylamin vorgelegt, nacheinander mit 0.8 g Phenylalaninol, 0.7 g HOBT und 1.2 g EDC versetzt und 12 h bei Raumtemperatur gerührt. Die Reaktionsmischung wurde über eine dünne Kieselgelschicht filtriert, das Filtrat mit 2M NaOH alkalisch gestellt und die organische Phase abgetrennt. Die organische Phase wurde mit 1H HCl-Lösung extrahiert, die wäßrige Phase mit NaOH neutraligiert und mit Methylenchlorid extrahiert. Die vereinigten organischen Phasen wurden über Magnesiumsulfat getrocknet, abfiltriert und eingeengt, wobei 1.3 g (56 %) des Produkts anfielen.
d) 2-(4-(Picol-2-yl)piperazin-1-yl)nikotiasäure-N-(3-phenylpropan-1-al-2-yl)amid
   1.3 g der Zwischenverbindung 5c wurden analog Beispiel 1d in 30 ml Methylenchlorid und 4 ml DMSO in Gegenwart von 3.7 g Triethylamin mit 2.6 g SO₃-Pyridin-Komplex oxidiert. Das Produkt wurde als Hydrochlorid ausgefällt, wobei 0.3 g (23 %) des Produkts in Form des Hydrochlorids anfielen.
   ¹H-NMR (d₆-DMSO): δ = 3.0-3.8 (10H), 4.5 (3H), 7.0-7.3 (4H), 7.4 (3H), 7.7 (2H), 8.0 (2H), 8.4 (1H), 8.7 (1H), 9.6 (1H) ppm.

### Beispiel 6

### 2-(4-(Picol-3-yl)piperazin-1-yl)nikotinsäure-N-(3-phenylpropan-1-al-2-yl) amid

a) 2-(4-(Picol-3-yl)piperazin-1-yl)nikotinsäuremethylester
   2.6 g 2-Chlornikotinsäuremethylester wurden in 50 ml DMF analog Beispiel 1a mit 2.7 g 3-Picolylpiperazin in Gegenwart von 4.2 g Kaliumcarbonat umgesetzt, wobei 1.2 g (25 %) des Produkts anfielen.
b) 2-(4-(Picol-3-yl)piperazin-1-yl)nikotinsäure
   1.2 g der Zwischenverbindung 6a wurden analog Beispiel 5b mit 20 ml 2 M NaOH bei 80 °C hydrolysiert, wobei 2.2 g des noch salzhaltigen Produkts anfielen, die ohne weitere Reinigung in die nächste Stufe eingesetzt wurden.
c) 2-(4-(Picol-3-yl)piperazin-1-yl)nikotinsäure-N-(3-phenylpropan-1-ol-2-yl)amid
   2.2 g der Zwischenverbindung 6b wurden analog Beispiel 5c in Gegenwart von 4 ml Triethylamin in 50 ml Methylenchlorid und 4 ml DMF gelöst, und nacheinander mit 0.6 g Phenylalaninol, 0.5 g HOBT und 0.9 g EDC versetzt, wobei 1.7 g (42 %) des Produkts anfielen.
d) 2-(4-(Picol-3-yl)piperazin-1-yl)nikotinsäure-N-(3-phenylpropan-1-al-2-yl)amid
   1.7 g der Zwischenverbindung 6c wurden analog Beispiel 1d in 6 ml DMSO und 30 ml Methylenchlorid in Gegenwart von 6.1 ml Triethylamin mit 4.1 g SO₃-Pyridin-Komplex oxidiert. Das Produkt wurde als Hydrochlorid ausgefällt, wobei 0.6 g (32 %) des Produkts anfielen.
   ¹H-NMR (d₆-DMSO): δ = 3.0-3.6 (10H), 4.5 (3H), 7.0-7.5 (7H), 8.0 (1H), 8.2 (1H), 8.4 (1H), 8.8 (1H), 9.0 (1H), 9.2 (1H), 9.6 (1H) ppm.

### Beispiel 7

### 2-(4-(Picol-4-yl)piperazin-1-yl)nikotinsäure-N-(3-phenylpropan-1-al-2-yl)amid

a) 2-(4-(Picol-4-yl)piperazin-1-yl)nikotinsäuremethylester
   3.7 g 2-Chlornikotinsäuremethylester wurden in 50 ml DMF analog Beispiel 1a mit 2.5 g 4-Picol-4-ylpiperazin in Gegenwart von 4.0 g Kaliumcarbonat umgesetzt, wobei 2.9 g (62 %) des Produkts anfielen.
b) 2-(4-(Picol-4-yl)piperazin-1-yl)nikotinsäure
   2.9 g der Zwischenverbindung 7a wurden analog Beispiel 5b mit 20 ml 2 M NaOH bei 60 °C hydrolysiert, wobei 3.4 g des noch salzhaltigen Produkts anfielen, die ohne weitere Reinigung in die nächste Stufe eingesetzt wurden.
c) 2-(4-(Picol-4-yl)piperazin-1-yl)nikotinsäure-N-(3-phenylpropan-1-ol-2-yl)amid
   3.4 g der Zwischenverbindung 7b wurden analog Beispiel 5c in Gegenwart von 4 ml Triethylamin und Molekularsieb in 30 ml Methylenchlorid vorgelegt, und nacheinander mit 1.4 g Phenylalaninol, 1.1 g HOBT und 2.0 g EDC versetzt, wobei 3.6 g (95 %) des Produkts anfielen.
d) 2-(4-(Picol-4-yl)piperazin-1-yl)nikotinsäure-N-(3-phenylpropan-1-al-2-yl)amid
   3.6 g der Zwischenverbindung 7c wurden analog Beispiel 1d in 11 ml DMSO und 80 ml Methylenchlorid in Gegenwart von 6 ml Triethylamin mit 4.4 g SO₃-Pyridin-Komplex oxidiert, wobei 0.9 g (25 %) des Produkts in Form des Hydrochlorids anfielen.
   ¹H-NMR (d₆-DMSO): δ = 2.7-3.9 (10H), 4.3-4.6 (3H), 7.0 (1H), 7.1-7.3 (3H), 7.4 (1H), 7.8-8.0 (2H), 8.3 (3H), 9.0 (3H), 9.7 (1H) ppm.

### Beispiel 8

### 2-(4-Benzylhomopiperazin-1-yl)nikotinsäure-N-(3-phenylpropan-1-al-2-yl)amid

a) 2-Chlornikotinsäure-N-(3-phenylpropan-1-ol-2-yl)amid
   10.0 g Chlornikotinsäure wurden mit 9.7 g Phenylalaninol in 250 ml CH₂Cl₂ in Gegenwart von 13.0 g EDC, 2.9 g HOBT und 9.6 g Triethylamin analog Beispiel 1c umgesetzt, wobei 17.3 g (94 %) des Produkts anfielen.
b) 2-(4-Benzylhomopiperazin-1-yl)nikotinsäure-N-(3-phenylpropan-1-ol-2-yl)amid
   5.0 g der Zwischenverbindung 8a wurden in 70 ml DMF mit 3.3 g Benzylhomopiperazin, 4.8 g Kaliumcarbonat und einer Spatelspitze 18-Krone-6 versetzt und 2 h zum Rückfluß erhitzt. Die Reaktionsmischung wurde auf Wasser gegossen und mit Ether extrahiert. Die vereinigten organischen Extrakte wurden mit NaCl-Lösung gewaschen, über Magnesiumsulfat getrocknet und eingeengt. Nach MPLC-Reinigung fielen 1.0 g (13 %) des Produkts an.
c) 2-(4-Benzylhomopiperazin-1-yl)nikotinsäure-N-(3-phenylpropan-1-al-2-yl)amid
   1.0 g der Zwischenverbindung 8b wurden analog Beispiel 1d in 20 ml DMSO in Gegenwart von 0.9 g Triethylamin mit 0.7 g SO₃-Pyridin-Komplex oxidiert, wobei 0.5 g (50 %) des Produkts in Form der freien Base anfielen.
   ¹H-NMR (CDCl₃): δ = 1.8 (2H), 2.6 (2H), 2.7 (2H), 3.2 (2H), 3.3 (2H), 3.4 (2H), 3.6 (2H), 4.9 (1H), 6.8 (1H), 7.1-7.3 (11H), 7.9 (1H), 8.3 (1H), 9.7 (1H) ppm.

### Beispiel 9

### 2-(4-(Picol-2-yl)homopiperazin-1-yl)nikotinsäure-N-(3-phenylpropan-1-al-2-yl)amid

a) 2-(4-(Picol-2-yl)homopiperazin-1-yl)nikotinsäuremethylester 2.4 g 2-Chlornikotinsäuremethylester wurden in 50 ml DMF analog Beispiel 1a mit 2.7 g 2-picolylhomopiperazin in Gegenwart von 3.8 g Kaliumcarbonat umgesetzt, wobei 3.7 g (81 %) des Produkts anfielen.
b) 2-(4-(Picol-2-yl)homopiperazin-1-yl)nikotinsäure
   3.7 g der Zwischenverbindung 9a wurden analog Beispiel 1b in 10 ml THF mit 30 ml 5M NaOH bei 80 °C hydrolysiert, wobei 3.0 g (86 %) des Produkts anfielen.
c) 2-(4-(Picol-2-yl)homopiperazin-1-yl)nikotinsäure-N-(3-phenylpropan-1-ol-2-yl)amid
   3.0 g der Zwischenverbindung 9b wurden analog Beispiel 1c in Gegenwart von 1.5 g Triethylamin in 60 ml Methylenchlorid gelöst, und nacheinander mit 1.5 g Phenylalaninol, 0.4 g HOBT und 2.0 g EDC versetzt, wobei 2.7 g (62 %) des Produkts anfielen.
d) 2-(4-(Picol-2-yl)homopiperazin-1-yl)nikotinsäure-N-(3-phenylpropan-1-al-2-yl)amid
   2.7 g der Zwischenverbindung 9c wurden analog Beispiel 1d in 10 ml DMSO in Gegenwart von 2.4 g Triethylamin mit 2.9 g SO₃-Pyridin-Komplex oxidiert, wobei 2.2 g (83 %) des Produkts in Form der freien Base anfielen.
   ¹H-NMR (CDCl₃) : δ = 1.8 (2H), 3.3-3.5 (8H), 3.7 (2H), 4.6 (1H), 6.7 (1H), 7.1-7.3 (2H), 7.4 (3H), 7.7 (2H), 8.1 (2H), 8.5 (2H), 8.8 (1H), 9.6 (1H) ppm.

### Beispiel 10

### 2-(4-(Picol-4-yl)homopiperazin-1-yl)nikotinsäure-N-(3-phenylpropan-1-al-2-yl)amid

a) 2-(4-(Picol-4-yl)homopiperazin-1-yl)nikotinsäuremethylester
   2.4 g 2-Chlornikotinsäuremethyleseter wurden in 50 ml DMF analog Beispiel 1a in Gegenwart von 3.8 g Kaliumcarbonat mit 2.6 g 4-Picolylhomopiperazin umgesetzt, wobei 3.9 g (88 %) des Produkts anfielen.
b) 2-(4-(Picol-4-yl)homopiperazin-1-yl)nikotinsäure
   3.9 g der Zwischenverbindung 10a wurden analog Beispiel 1b in 20 ml THF mit 40 ml 5 M NaOH bei 80 °C hydrolysiert, wobei 2.7 g (70 %) des Produkts anfielen.
c) 2-(4-(Picol-4-yl)homopiperazin-1-yl)nikotinsäure-N-(3-phenylpropan-1-ol-2-yl)amid
   2.6 g der Zwischenverbindung 10b wurden analog Beispiel 1c in Gegenwart von 1.7 g Triethylamin in 60 ml Methylenchlorid gelöst, und nacheinander mit 1.3 g Phenylalaninol, 0.4 g HOBT und 1.7 g EDC versetzt, wobei 1.7 g (46 %) des Produkts anfielen.
d) 2-(4-(Pieol-4-yl)homopiperazin-1-yl)nikotinsäure-N-(3-phenylpropan-1-al-2-yl)amid
   1.7 g der Zwischenverbindung 10c wurden analog Beispiel 1d in 20 ml DMSO in Gegenwart von 1.6 g Triethylamin mit 1.8 g SO₃-Pyridin-Komplex oxidiert, wobei 1.5 g (44 %) des Produkts in Form der freien Base anfielen.
   ¹H-NMR (CDCl₃): δ = 1.9 (2H), 2.6-2.8 (4H), 3.1-3.8 (8H), 4.7 (1H), 6.8 (1H), 7.0-7.4 (5H), 8.0 (1H), 8.2-8.3 (2H), 8.4-8.7 (4H), 9.8 (1H) ppm.

### Beispiel 11

### 2-(4-(2-(Pyrid-2-yl)-1-ethyl)piperazin-1-yl)nikotinsäure-N-(3-phenylpropan-1-al-2-yl)amid

a) 2-(4-(2-(Pyrid-2-yl)-1-ethyl)piperazin-1-yl)nikotinsäuremethylester
   3.3 g 2-Chlornikotinsäuremethylester wurden in 20 ml Butanol analog Beispiel 1a mit 3.0 g N-(2-(Pyrid-2-yl)ethyl)piperazin in Gegenwart von 4.3 g Kaliumcarbonat umgesetzt, wobei 3.8 g (72 %) des Produkts anfielen.
b) 2-(4-(2-(Pyrid-2-yl)ethyl)piperazin-1-yl)nikotinsäure
   3.8 g der Zwischenverbindung 11a wurden mit 20 ml 2 M NaOH 1 h bei 60 °C analog Beispiel 5b hydrolysiert, wobei 3.6 g des noch salzhaltigen Produkts anfielen, die ohne weitere Reinigung in die nächste Stufe eingesetzt wurden.
c) 2-(4-(2-(Pyrid-2-yl)ethyl)piperazin-1-yl)nikotinsäure-N-(3-phenylpropan-1-ol-2-yl)amid
   3.6 g der Zwischenverbindung 11b wurden in 50 ml Methylenchlorid und 4 ml DMF analog Beispiel 5c in Gegenwart von 5.1 ml Triethylamin und Molekularsieb vorgelegt, und nacheinander mit 1.7 g Phenylalaninol, 1.4 g HOBT und 2.5 g EDC versetzt, wobei 4.6 g (95 %) des Produkts anfielen.
d) 2-(4-(2-(Pyrid-2-yl)ethyl)piperazin-1-yl)nikotinsäure-N-(3-phenylpropan-1-al-2-yl)amid
   4.6 g der Zwischenverbindung 11c wurden analog Beispiel 1d in 30 ml Methylenchlorid und 15 ml DMSO in Gegenwart von 7.6 g Triethylamin mit 3.5 g SO₃-Pyridin-Komplex oxidiert. Das Produkt wurde als Hydrochlorid ausgefällt, wobei 0.9 g (20 %) des Produkts in Form des Hydrochlorids anfielen.
   ¹H-NMR (d₆-DMSO): δ = 3.0-3.4 (8H), 3.4-3.7 (4H), 3.9-4.4 (3H), 7.1-7.5 (5H), 7.8 (2H), 7.9 (2H), 8.4 (3H), 8.8 (2H), 9.7 (1H) ppm.

### Beispiel 12

### 2-(4-(2-Methoxybenzyl)piperazin-1-yl)nikotinsäure-N-(3-phenylpropan-1-al-2-yl)amid

a) 2-(4-(2-Methoxybenzyl)piperazin-1-yl)nikotinsäure-N-(3-phenylpropan-1-ol-2-yl)amid
   3.0 g Zwischenverbindung 8a wurden in 70 ml DMF mit 2.9 g N-(2-Methoxybenzyl)piperazin analog Beispiel 8b in Gegenwart von 5.7 g Kaliumcarbonat und einer Spatelspitze 18-Krone-6 umgesetzt, wobei 1.3 g (27 %) des Produkt anfielen.
b) 2 -(4-(2-Methoxybenzyl)piperazin-1-yl)nikotinsäure-N-(3-phenylpropan-1-al-2-yl)amid
   1.2 g Zwischenverbindung 12a wurden analog Beispiel 1d mit 1.2 g SO₃-Pyridin-Komplex in 20 ml DMSO in Gegenwart von 1.1 g Triethylamin oxidiert, wobei 0.7 g (58 %) des Produkts in Form der freien Base anfielen.
   MS: m/e = 458 (M⁺)

### Beispiel 13

### 2-(4-(3,4-Dioxomethylenbenzyl)piperazin-1-yl)nikotinsäure-N-(3-phenylpropan-1-al-2-yl)amid

a) 2-(4-(3,4-Dioxomethylenbenzyl)piperazin-1-yl)nikotinsäure-N-(3-phenylpropan-1-ol-2-yl)amid
   3.0 g Zwischenverbindung 8a wurden 70 ml DMF mit 2.3 g N-(3,4-Dioxomethylenbenzyl)piperazin analog Beispiel 8b in Gegenwart von 2.9 g Kaliumcarbonat und einer Spatelspitze 18-Krone-6 umgesetzt, wobei 1.3 g (27 %) des Produkt anfielen.
b) 2-(4-(3,4-Dioxomethylenbenzyl)piperazin-1-yl)nikotinsäure-N-(3-phenylpropan-1-al-2-yl)amid
   0.6 g Zwischenverbindung 13a wurden analog Beispiel 1d mit 0.6 g SO₃-Pyridin-Komplex in 20 ml DMSO in Gegenwart von 0.5 g Triethylamin oxidiert, wobei 0.3 g (55 %) des Produkts in Form der freien Base anfielen.
   ¹H-NMR (CDCl₃): δ = 2.3 (2H), 2.4 (2H), 3.0-3.2 (4H), 3.3-3.4 (4H), 4.9 (1H), 5.9 (2H), 6.7 (2H), 6.8 (1H), 7.1-7.3 (7H), 8.4 (2H), 9.8 (1H) ppm.

### Beispiel 14

### 2-(4-(1-Piperidinyl)piperidin-1-yl)nikotinsäure-N-(3-phenylpropan-1-al-2-yl)amid

a) 2-(4-(1-Piperidinyl)piperidin-1-yl)nikotinsäuremethylester
   3.4 g 2-Chloronikotinsäuremethylester wurden analog Beispiel 1a mit 3.4 g 4-Piperidinopideridin in 75 ml DMF in Gegenwart von 5.5 g Kaliumcarbonat und einer Spatelspitze 18-Krone-6 umgesetzt, wobei 5.9 g (97 %) des Produkts anfielen.
b) 2-(4-(1-Piperidinyl)piperidin-1-yl)nikotinsäure
   5.5 g der Zwischenverbindung 14a in 100 ml THF wurden analog Beispiel 1b mit 1.3 g LiOH in 50 ml Wasser und 10 ml MeOH umgesetzt, wobei 8.1 g des noch salzhaltigen Produkt anfielen, die ohne weitere Reinigung direkt in die nächste Stufe eingesetzt wurden.
c) 2-(4-(1-Piperidinyl)piperidin-1-yl)nikotinsäure-N-(3-phenylpropan-1-ol-2-yl)amid
   7.1 g der Zwischenverbindung 14b wurden analog Beispiel 1c in Gegenwart von 5.2 g Triethylamin und 5 g Natriumsulfat in 200 ml Methylenchlorid und 50 ml DMF vorgelegt und nacheinander mit 2.6 g Phenylalaninol 2.3 g HOBT und 3-6 g EDC versetzt, wobei 0.7g (10%) des Produkts anfielen.
d) 2-(4-(1-Piperidinyl)piperidin-1-yl)nikotinsäure-N-(3-phenylpropan-1-al-2-yl)amid
   0.6 g der Zwischenverbindung 14c wurden analog Beispiel 1d in 20 ml DMSO in Gegenwart von 0.6 g Triethylamin mit 1.0 g SO₃-Pyridin-Komplex oxidiert, wobei 0.1 g (19 %) des Produkts in Form der freien Base anfielen.
   MS: m/e = 420 (M⁺)

### Beispiel 15

### 2-(4-(4-N,N-Dimethylamino)benzylhomopiperazin-1-yl)nikotinsäure-N-(3-phenylpropan-1-al-2-yl)amid

a) 2-(Homopiperazin-1-yl)nikotinsäureethylester
   10.0 g 2-Chlornikotinsäureethylester wurden mit 21.6 g Homopiperazin in 150 ml Ethanol 2 h zum Rückfluß erhitzt. Nach Entfernen des Lösungsmittels wurde der Rückstand zwischen NaCl-Lösung und Essigester verteilt und die wäßrige Phase mehrmals mit Essigester extrahiert. Die vereinigten organischen Phasen wurden über Magnesiumsulfat getrocknet und eingeengt, wobei 11.1 g (83 %) des Produkts anfielen.
b) 2-(4-(4-N,N-Dimethylaminobenzyl)homopiperazin-1-yl)nikotinsäureethylester
   2.0 g der Zwischenverbindung 18a und 1.3 g 4-N,N-Dimethylaminobenzaldehyd wurden in 40 ml Ethanol vorgelegt und bei Raumtemperatur mit 1.1 ml Boran-Pyridin-Komplex versetzt. Es wurde 18 h bei Raumtemperatur nachgerührt. Nach Entfernen des Lösungsmittels wurde der Rückstand zwischen Wasser und Essigester verteilt. Die organische Phase wurde mit 2N HCl extrahiert, die wäßrige Phase zweimal mit Essigester gewaschen und mit 2N NaOH basisch gestellt. Das Produkt wurde mit Essigester extrahiert, die vereinigten Essigesterextrakte wurden über Magnesiumsulfat getrocknet und eingeengt, wobei 2.9 g (93%) des Produkt anfielen.
c) 2-(4-(4-N,N-Dimethylaminobenzyl)homopiperazin-1-yl)nikotinsäure
   2.8 g der Zwischenverbindung 18b wurden in 30 ml MeOH mit 30 ml 5N NaOH 2 h auf 60 °C erhitzt. Nach Einengen des Lösungsmittels wurde mit konz. HCl neutralisiert, mit EtOH und Triethylamin ausgesalzt, vom Salz abfiltriet und das Filtrat eingeengt, wobei 2.5 g (100 %) des Produkts anfielen.
d) 2-(4-(4-N,N-Dimethylaminobenzyl)homopiperazin-1-yl)nikotinsäure-N-(3-phenylpropan-1-ol-2-yl)amid
   2.3 g der Zwischenverbindung 18c wurden in 50 ml Methylenchlorid gelöst und nacheinander mit 0.8 g Triethylamin, 0.6 g Phenylalaninol, 0.2 g HOBT und 0.8 g EDC versetz. Es wurde bei Raumtemperatur 5h über Molsieb gerührt. Die Reaktionsmischung wurde mit Wasser und 2N NaOH gewaschen und das Produkt mit 1N HCl extrahiert. Die wäßrige Phase wurde mit Essigester gewaschen und mit 2N NaOH auf pH 9 gestellt. Das Produkt wurde mit Essigester extrahier. Die vereinigten organischen Phasen wurden über Magnesiumsulfat getrocknet und eingeengt, wobei 1.2 g (69 %) des Produkts anfielen.
e) 2-(4-(4-N,N-Dimethylaminobenzyl)homopiperazin-1-yl)nikotinsäure-N-(3-phenylpropan-1-al-2-yl)amid
   1.2 g Zwischenstufe 18d wurden in 40 ml Methylenchlorid mit 1.2 g SO₃-Pyridin-Komplex in Gegenwart von 1.0 g Triethylamin und 2 ml DMSO über Molsieb gerührt. Die Reaktionsmischung wurde mit Methylenchlorid verdünnt, dreimal mit NaCl gewaschen, über Magnesiumsulfat getrocknet und eingeengt, wobei 1.1 g (97 %) des Produkts anfielen.
   1H-NMR (CDCl₃): δ = 1.8 (2H), 3.0 (6H), 3.1 (4H), 3.2 (4H), 3.4 (2H), 4.4 (2H), 4.8 (1H), 6.6 (3H), 6.8 (1H), 7.2 (5H), 8.0 (1H), 8.2 (1H), 8.7 (1H), 9.8 (1H) ppm.

### Beispiel 16

### 2-(4-(2-Fluorobenzyl)piperazin-1-yl)nikotinsäure-N-(3-phenylpropan-1-al-2-yl)amid

a) 2-(Piperazin-1-yl)nikotinsäureethylester
   10.0 g 2-Chlornikotinsäureethylester wurden analog Beispiel 18a mit 27.8 g Piperazin in 400 ml Ethanol umgesetzt, wobei 6.9 g (54 %) des Produkts anfielen.
b) 2-(4-(2-Fluorobenzyl)piperazin-1-yl)nikotinsäureethylester
   1.0 g der Zwischenverbindung 19a wurden analog Beispiel 18b mit 2-Fluorobenzaldehyd und 0.5 g Boran-Pyridin-Komplex in 40 ml Ethanol umgesetzt, wobei 1.2 g (39 %) des Produkt anfielen.
c) 2-(4-(2-Fluorobenzyl)piperazin-1-yl)nikotinsäure
   1.2 g der Zwischenverbindung 19b wurden analog Beispiel 18c in 15 ml Methanol gelöst und mit 30 ml 5N NaOH hydrolysiert, wobei 0.7 g (63 %) des Produkts anfielen.
d) 2-(4-(2-Fluorobenzyl)piperazin-1-yl)nikotinsäure-N-(3-phenylpropan-1-ol-2-yl)amid
   0.7 g der Zwischenverbindung 19c wurden analog Beispiel 18d in 50 ml Methylenchlorid in Gegenwart von 0.5 g Triethylamin vorgelegt und nacheinander mit 0.3 g Phenylalaninol, 0.5 g EDC und 0.1 g HOBT versetzt, wobei 0.5 g (48 %) des Produkt anfielen.
e) 2-(4-(2-Fluorobenzyl)piperazin-1-yl)nikotinsäure-N-(3-phenylpropan-1-al-2-yl)amid
   0.5 g der Zwischenverbindung 19d wurden analog Beispiel 18e in 40 ml Methylenchlorid und 1 ml DMSO in Gegenwart von 0.4 g Triethylamin mit 0.5 g SO₃-Pyridin-Komplex oxidiert, wobei 0.5 g (100 %) des Produkts anfielen.
   MS: m/e = 446 (M⁺)

### Beispiel 17

### 2-(4-Phenylpiperazin-1-yl)nikotinsäure-N-(3-phenylpropan-1-al-2-yl)amid

a) 2-(4-Phenylpiperazin-1-yl)nikotinsäureethylester
   2.0 g 2-Chlornikotinsäureethylester wurden analog Beispiel 1a mit 1.8 g Phenylpiperazin in Gegenwart von 2.5 g Kaliumcarbonat und einer Spatelspitze 18-Krone-6 in 40 ml Butanol 2.5 h bei 80 °C umgesetzt, wobei 1.2 g (35 %) des Produkts anfielen.
b) 2-(4-Phenylpiperazin-1-yl)nikotinsäure
   1.2 g der Zwischenverbindung 20a wurden analog Beispiel 18c in 30 ml Methanol mit 30 ml 5M NaOH hydrolysiert, wobei 1.8 g des Rohprodukts anfielen, die ohne weitere Reinigung in die nächste Stufe eingesetzt wurden.
c) 2-(4-Phenylpiperäzin-1-yl)nikotinsäure-N-(3-phenylpropan-1-ol-2-yl)amid
   1.8 g der Zwischenverbindung 20b wurden analog Beispiel 18d in 50 ml Methylenchlorid in Gegenwart von 0.8 g Triethylamin vorgelegt und nacheinander mit 0.6 g Phenylalaninol, 0.8 g EDC und 0.2 g HOBT versetzt, wobei 1.1 g (72 %) des Produkt anfielen
d) 2-(4-Phenylpiperazin-1-yl)nikotinsäure-N-(3-phenylpropan-1-al-2-yl)amid
   1.1 g der Zwischenverbindung 20c wurden analog Beispiel 18e in 40 ml Methylenchlorid und 2 ml DMSO in Gegenwart von 1.1 g Triethylamin mit 1.2 g SO₃-Pyridin-Komplex oxidiert, wobei 0.8 g (72 %) des Produkts anfielen.
   MS: m/e = 414 (M⁺)

### Beispiel 18

### 2-(4-(Picol-2-yl)homopiperazin-1-yl)nikotinsäure-N-(1-carbamoyl-1-oxo-3-phenylpropan-2-yl)amid

a) 2-(4-(Picol-2-yl)homopiperazin-1-yl)nikotinsäure-N-(1-carbamoyl-1-ol-3-phenylpropan-2-yl)amid
   1.8 g der Zwischenverbindung 9b wurden analog Beispiel 18d in 50 ml Methylenchlorid in Gegenwart von 1.9 g Triethylamin vorgelegt und nacheinander mit 1.1 g 3-Amino-2-hydroxy-4-phenylbuttersäureamid-Hydrochlorid, 1.2 g EDC und 0.3 g HOBT versetzt, wobei 0.5 g (18 %) des Produkt anfielen.
b) 2-(4-(Picol-2-yl)homopiperazin-1-yl)nikotinsäure-N-(1-carbamoyl-1-oxo-3-phenylpropan-2-yl) amid
   0.5 g der Zwischenverbindung 21a wurden analog Beispiel 18e in 40 ml Methylenchlorid und 2.5 ml DMSO in Gegenwart von 0.4 g Triethylamin mit 0.4 g SO₃-Pyridin-Komplex oxidiert, wobei 0.2 g (54 %) des Produkts anfielen.
   MS: m/e = 486 (M⁺)

### Beispiel 19

### 2-(4-(4-Methoxybenzyl)piperazin-1-yl)nikotinsäure-N-(3-phenylpropan-1-al-2-yl)amid

a) 2-(4-(4-Methoxybenzyl)piperazin-1-yl)nikotinsäureethylester 2.0 g der Zwischenverbindung 19a wurden analog Beispiel 18b 6.0 ml Anisaldehyd und 1.1 ml Boran-Pyridin-Komplex in 40 ml Ethanol umgesetzt, wobei 3.0 g (94 %) des Produkts anfielen.
b) 2-(4-(4-Methoxybenzyl)piperazin-1-yl)nikotinsäure
   1.5 g der Zwischenverbindung 22a wurden analog Beispiel 18c in 10 ml Methanol mit 25 ml 5M NaOH hydrolysiert, wobei 1.6 g (61 %) des Produkts anfielen.
c) 2-(4-(4-Methoxybenzyl)piperazin-1-yl)nikotinsäure-N-(3-phenylpropan-1-ol-2-yl)amid
   1.0 g der Zwischenverbindung 22b wurden analog Beispiel 18d in 50 ml Methylenchlorid in Gegenwart von 1.4 ml Triethylamin vorgelegt und nacheinander mit 0.5 g Phenylalaninol, 0.8 g EDC und 0.5 g HOBT versetzt, wobei 1.2 g (88 %) des Produkt anfielen.
d) 2-(4-(4-Methoxybenzyl)piperazin-1-yl)nikotinsäure-N-(3-phenylpropan-1-al-2-yl)amid
   1.2 g der Zwischenverbindung 22c wurden analog Beispiel 18e in 50 ml Methylenchlorid und 3.5 ml DMSO in Gegenwart von 2.1 ml Triethylamin mit 3.0 g SO₃-Pyridin-Komplex oxidiert, wobei 0.5 g (46 %) des Produkts anfielen.
   MS: m/e = 458 (M⁺)

### Beispiel 20

### 2-(4-(4-Methoxybenzyl)homopiperazin-1-yl)nikotinsäure-N-(3-phenylpropan-1-al-2-yl)amid

a) 2-(4-(4-Methoxybenzyl)homopiperazin-1-yl)nikotinsäureethylester
   2.5 g der Zwischenverbindung 18a wurden analog Beispiel 18b 1.4 ml Anisaldehyd und 1.3 ml Boran-Pyridin-Komplex in 30 ml Ethanol umgesetzt, wobei 3.6 g (98 %) des Produkts anfielen.
b) 2-(4-(4-Methoxybenzyl)homopiperazin-1-yl)nikotinsäure
   3.6 g der Zwischenverbindung 23a wurden analog Beispiel 18c in 10 ml Methanol mit 20 ml 5M NaOH hydrolysiert, wobei 2.9 g (87 %) des Produkts anfielen.
c) 2-(4-(4-Methoxybenzyl)homopiperazin-1-yl)nikotinsäure-N-(3-phenylpropan-1-ol-2-yl)amid
   1.3 g der Zwischenverbindung 23b wurden analog Beispiel 18d in 50 ml Methylenchlorid in Gegenwart von 1.75 g Triethylamin vorgelegt und nacheinander mit 0.6 g Phenylalaninol, 0.9 g EDC und 0.6 g HOBT versetzt, wobei 1.3 g (69 %) des Produkt anfielen.
d) 2-(4-(4-Methoxybenzyl)homopiperazin-1-yl)nikotinsäure-N-(3-phenylpropan-1-al-2-yl)amid
   1.2 g der Zwischenverbindung 23c wurden analog Beispiel 18e in 50 ml Methylenchlorid und 3.5 ml DMSO in Gegenwart von 2.1 ml Triethylamin mit 3.0 g SO₃-Pyridin-Komplex oxidiert, wobei 0.6 g (46 %) des Produkts anfielen.
   MS: m/e = 472 (M⁺)

### Beispiel 21

### 2-(4-(4-n-Butoxybenzyl)piperazin-1-yl)nikotinsäure-N-(3-phenylpropan-1-al-2-yl)amid

a) 2-(4-(4-n-Butoxybenzyl)piperazin-1-yl)nikotinsäureethylester
   2.4 g der Zwischenverbindung 19a wurden analog Beispiel 18b 2.1 ml 4-Butoxybenzaldehyd und 1.3 ml Boran-Pyridin-Komplex in 30 ml Ethanol umgesetzt, wobei 3.5 g (89 %) des Produkts anfielen.
b) 2-(4-(4-n-Butoxybenzyl)piperazin-1-yl)nikotinsäure
   3.5 g der Zwischenverbindung 24a wurden analog Beispiel 18c in 30 ml Methanol mit 15 ml 5M NaOH hydrolysiert, wobei 3.2 g (97 %) des Produkts anfielen.
c) 2-(4-(4-n-Butoxybenzyl)piperazin-1-yl)nikotinsäure-N-(3-phenylpropan-1-ol-2-yl)amid
   1.5 g der Zwischenverbindung 24b wurden analog Beispiel 18d in 50 ml Methylenchlorid in Gegenwart von 1.9 ml Triethylamin vorgelegt und nacheinander mit 0.7 g Phenylalaninol, 1.0 g EDC und 0.7 g HOBT versetzt, wobei 1.3 g (62 %) des Produkt anfielen.
d) 2-(4-(4-n-Butoxybenzyl)piperazin-1-yl)nikotinsäure-N-(3-phenylpropan-1-al-2-yl)amid
   1.3 g der Zwischenverbindung 24c wurden analog Beispiel 18e in 40 ml Methylenchlorid und 3.5 ml DMSO in Gegenwart von 2.1 ml Triethylamin mit 2.0 g SO₃-Pyridin-Komplex oxidiert, wobei 0.8 g (52 %) des Produkts anfielen.
   MS: m/e = 500 (M⁺)

### Beispiel 22

### 3-(4-Benzylpiperazin-1-yl)benzoesäure-N-(1-carbamoyl-1-oxo-3-phenylpropan-2-yl)amid

a) 3-(4-Benzylpiperazin-1-yl)benzoesäure
   1.5 g 3-Benzylpiperazin-1-ylbenzonitril wurden 2 h in 14 ml konz. HCl zum Rückfluß erhitzt. Beim Abkühlen auf Raumtemperatur fallen 1.7 g (100 %) des Produkt aus, die abgesaugt wurden und gründlich mit Wasser gewaschen wurden.
b) 3-(4-Benzylpiperazin-1-yl)benzoesäure-N-(1-carbamoyl-1-ol-3-phenylpropan-2-yl)amid
   1.5 g der Zwischenverbindung 26a wurden in 50 ml DMF analog Beispiel 1c in Gegenwart von 2.9 ml Triethylamin vorgelegt und nacheinander mit 0.7 g HOBT, 1.2 g 3-Amino-2-hydroxy-4-phnylbuttersäureamid-Hydrochlorid und 1.1 g EDC versetzt, wobei 1.7 g (71 %) des Produkts anfielen.
c) 3-(4-Benzylpiperazin-1-yl)benzoesäure-N-(1-carbamoyl-1-oxo-3-phenylpropan-2-yl)amid
   1.5 g der Zwischenverbindung 26b wurden analog Beispiel 1d in 30 ml DMSO gelöst und in Gegenwart von 2 ml Triethylamin mit 1.5 g SO₃-Pyridin-Komplex oxidiert, wobei 0.5 g (33 %) des Produkts anfielen.
   MS: m/e = 470 (M⁺)

### Beispiel 23

### 2-(4-(2-Naphtylmethyl)piperazin-1-yl)nikotinsäure-N-(3-phenylpropan-1-al-2-yl)amid

a) 2-(4-Butoxycarbonylpiperazin-1-yl)nikotinsäure-N-(3-phenylpropan-1-ol-2-yl)amid
   13 g 2-(4-Butoxycarbonylpiperazin-1-yl)nikotinsäure wurden analog Beispiel 1c in 150 ml Methylenchlorid und 14.7 ml Triethylamin gelöst und nacheinander mit 6.4 g Phenylalaninol, 1.9 g HOBT und 8.11 g EDC versetzt, wobei 16.7 g (90 %) des Produkts anfielen.
b) 2-(Piperazin-1-yl)nikotinsäure-N-(3-phenylpropan-1-ol-2-yl)amid
   16.7 g der Zwischenverbindung 27a wurden in 300 ml Methylenchlorid gelöst und mit 30 ml Konz. Trifluoressigsäure versetzt. Es wurde 1.5 h bei Raumtemperatur nachgerührt. Die Reaktionsmischung wurde auf Eiswasser geschüttet, basisch getsellt und das Produkt mit Methylenchlorid extrahiert. Die vereinigten organischen Phasen wurden über Magnesiumsulfat getrocknet und eingeengt, wobei 12.8 g (98 %) des Produkts anfielen.
c) 2-(4-(2-Naphtylmethylpiperazin-1-yl)nikotinsäure-N-(3-phenylpropan-1-ol-2-yl)amid
   1.9 g der Zwischenverbindung 27b wurden in 75 ml Ethanol vorgelegt und mit 1.3 g 2-α-Brommethylnaphthalin und 0.8 g Kaliumcarbonat versetzt. Es wurde 18 h bei Raumtemperatur gerührt. Die Reaktionsmischung wurde eingeengt, der Rückstand zwischen Essigester und Wasser verteilt, und die organische Phase über Magnesiumsulfat getrocknet und eingeengt, wobei 2.3 g (86 %) des Produkts anfielen.
d) 2-(4-(2-Naphtylmethyl)-piperazin-1-yl)nikotinsäure-N-(3-phenylpropan-1-al-2-yl)amid
   1.5 g der Zwischenverbindung 27c wurden anlog Beispiel 1d in 25 ml DMSO in Gegenwart von 1.7 ml Triethylamin mit 1.5 g SO₃-Pyridinkomplex oxidiert, wobei 0.9 g (59 %) des Produkts anfielen.
   MS: m/e = 478 (M⁺)

### Beispiel 24

### 2-(4-(2-Tolyl)piperazin-1-yl)nikotinsäure-N-(3-phenylpropan-1-al-2-yl)amid

a) 2-(4-(2-Tolyl)piperazin-1-yl)nikotinsäure-N-(3-phenylpropan-1-ol-2-yl)amid
   2.0 g der Zwischenverbindung 27 wurden analog Beispiel 27c in 100 ml Ethanol vorgelegt und mit 1.1 g 2-(Brommethyl)toluol und 0.8 g Kaliumcarbonat versetzt, wobei 1.6 g (62 %) des Produkts anfielen.
b) 2-(4-(2-Tolyl)piperazin-1-yl)nikotinsäure-N-(3-phenylpropan-1-al-2-yl)amid
   1.4 g der Zwischenverbindung 28a wurden anlog Beispiel 1d in 25 ml DMSO in Gegenwart von 1.7 ml Triethylamin mit 1.0 g SO₃-Pyridinkomplex oxidiert, wobei 0.7 g (48 %) des Produkts anfielen.
   MS: m/e = 460 (M⁺+H₂O)

### Beispiel 25

### 2-(4-(3-Tolyl)piperazin-1-yl)nikotinsäure-N-(3-phenylpropan-1-al-2-yl)amid

a) 2-(4-(3-Tolyl)piperazin-1-yl)nikotinsäure-N-(3-phenylpropan-1-ol-2-yl)amid
   2.0 g der Zwischenverbindung 29b wurden analog Beispiel 27c in 80 ml Ethanol vorgelegt und mit 1.1 g 3(Brommethyl)toluol und 0.8 g Kaliumcarbonat versetzt, wobei 1.8 g (70 %) des Produkts anfielen.
b) 2-(4-(3-Tolyl)piperazin-1-yl)nikotinsäure-N-(3-phenylpropan-1-al-2-yl)amid
   1.6 g der Zwischenverbindung 29a wurden anlog Beispiel 1d in 25 ml DMSO in Gegenwart von 2.0 ml Triethylamin mit 1.2 g SO₃-Pyridinkomplex oxidiert, wobei 0.4 g (26 %) des Produkts anfielen.
   MS: m/e = 442 (M⁺)

### Beispiel 26

### 2-(4-(4-Tolyl)piperazin-1-yl)nikotinsäure-N-(3-phenylpropan-1-al-2-yl)amid

a) 2-(4-(4-Tolyl)piperazin-1-yl)nikotinsäure-N-(3-phenylpropan-1-ol-2-yl)amid
   1.6 g der Zwischenverbindung 27b wurden analog Beispiel 27c in 80 ml Ethanol vorgelegt und mit 1.1 g 4(Brommethyl)toluol und 0.8 g Kaliumcarbonat versetzt, wobei 1.8 g (69 %) des Produkts anfielen.
b) 2-(4-(4-Tolyl)piperazin-1-yl)nikotinsäure-N-(3-phenylpropan-1-al-2-yl)amid
   1.6 g der Zwischenverbindung 30a wurden anlog Beispiel 1d in 25 ml DMSO in Gegenwart von 2.0 ml Triethylamin mit 1.2 g SO₃-Pyridinkomplex oxidiert, wobei 0.7 g (46 %) des Produkts anfielen.
   MS: m/e = 460 (M⁺+H₂O)

### Beispiel 27

### 2-(4-(4-Methoxycarbonylbenzylpiperazin-1-yl)nikotinsäure-N-(3-phenylpropan-1-al-2-yl)amid

a) 2-(4-(4-Methoxycarbonylbenzylpiperazin-1-yl)nikotinsäure-N-(3-phenylpropan-1-ol-2-yl)amid
   2.5 g der Zwischenverbindung 27b wurden analog Beispiel 27c in 100 ml Ethanol vorgelegt und mit 1.7 g Brommethylbenzoesäuremethylester und 1.0 g Kaliumcarbonat versetzt, wobei 1.9 g (54 %) des Produkts anfielen.
b) 2-(4-(4-Methoxycarbonylbenzylpiperazin-1-yl)nikotinsäure-N-(3-phenylpropan-1-al-2-yl)amid
   0.9 g der Zwischenverbindung 31a wurden anlog Beispiel 1d in 5 ml DMSO in Gegenwart von 1.0 ml Triethylamin mit 0.6 g SO₃-Pyridinkomplex oxidiert, wobei 0.1 g (15 %) des Produkts anfielen.
   MS: m/e = 485 (M⁺-1)

### Analog Beispiel 1-27 wurden folgende Beispiele synthetisiert:

### Beispiel 28

### 2-(4-(Picol-3-yl)homopiperazin-1-yl)nikotinsäure-N-(3-phenylpropan-1-al-2-yl)amid

MS: m/e = 443 (M⁺)

### Beispiel 29

### 2-(4-Benzylpiperazin-1-yl)nikotinsäure-N-(1-carbamoyl-1-oxo-3-phenylpropan-2-yl)amid-Dihydrochlorid

MS: m/e = 471 (M⁺)

### Beispiel 30

### 2-(4-Benzylpiperazin-1-yl)benzoesäure-N-(1-carbamoyl-1-oxo-3-phenylpropan-2-yl)amid

MS: m/e = 470 (M⁺)

### Beispiel 31

### 2-(4-Benzylpiperazin-1-yl)nikotinsäure-N-(1-carbamoyl-1-oxohexan-2-yl)amid

MS: m/e = 437 (M⁺)

### Beispiel 32

### 2-(4-Benzylpiperazin-1-yl)pyridin-4-carbonsäure-N-(3-phenylpropan-1-al-2-yl)amid

MS: m/e = 428 (M⁺)

### Beispiel 33

### 2-(4-Benzylpiperazin-1-yl)nikotinsäure-N-(N(2-piperidin-1-yl-1-ethyl)-1-carbamoyl-1-oxo-3-phenylpropan-2-yl)amid

MS: m/e = 582 (M⁺)

### Beispiel 34

### 2-(4-Benzylpiperazin-1-yl)nikotinsäure-N-(N(2-piperidin-1-yl-1-ethyl)-1-carbamoyl-1-oxo-3-phenylpropan-2-yl)amid

MS: m/e = 584 (M⁺)

### Beispiel 35

### 2-(4-Benzylpiperazin-1-yl)nikotinsäure-N-(N(2-pyrid-2yl-1-ethyl)-1-carbamoyl-1-oxo-3-phenylpropan-2-yl)amid

HS: m/e = 576 (M⁺)

### Beispiel 36

### 2-(4-Benzylpiperazin-1-yl)nikotinsäure-N-(N(3(4-methyl-piperazin-1-yl)-1-propyl)-1-carbamoyl-1-oxo-3-phenylpropan-2-yl)amid

MS: m/e = 611 (M⁺)

### Beispiel 37

### 2-(4-Benzylpiperazin-1-yl)nikotinsäure-N-(N(3(N,N-diethylamino)-1-propyl)-1-carbamoyl-1-oxo-3-phenylpropan-2-yl)amid

MS: m/e = 584 (M⁺)

### Beispiel 38

### 2-((3-N,N-Dimethylaminomethylpyrid-2-yl)piperazin-1-yl)nikotinsäure-N-(3-phenylpropan-1-al-2-yl)amid x Trifumarsäure

MS: m/e = 500 (M⁺+1)

### Beispiel 39

### 2-(4-Phenylpiperazin-1-yl)benzoesäure-N-(1-carbamoyl-1-oxo-3-phenylpropan-2-yl)amid

MS: m/e = 456 (M⁺)

### Beispiel 40

### 5-Nitro-2-(4-phenylpiperazin-1-yl)benzoesäure-N-(1-carbamoyl-1-oxo-3-phenylpropan-2-yl)amid

MS: m/e = 501 (M⁺)

### Beispiel 41

### 2-(4-Benzylpiperazin-1-yl)-5-nitrobenzoesäure-N-(1-carbamoyl-1-oxo-3-phenylpropan-2-yl)amid

MS: m/e = 515 (M⁺)

### Beispiel 42

### 2-(3-Phenylpyrrolidin-1-yl)nikotinsäure-N-(1-carbamoyl-1-oxo-3-phenylpropan-2-yl)amid

¹H-NMR (CF₃COOD): δ= 2,0-2,7 (2H); 3,0 (1H); 3,3-4,0 (6H); 5,9 (1H); 6,9 (1H), 7.0-7.4 (10H) und 7.9 (2H) ppm

### Beispiel 43

### 2(4(4(N,N-Dimethylamino)benzyl-piperazin-1-yl))-nikotinsäure-N-(3-phenylpropan-1-al-2-yl)amid

MS: m/e = 471

### Beispiel 44

### 2-(4(3(2-(N,N-Diethylamino)-1-ethyl-pyrid-2-yl)piperazin-1-yl)-nikotinsäure-N-(1-carbamoyl-1-oxo-3-phenyl-propan-2-yl)amid

¹H-NMR (Methanol-D₄): δ= 1,4(6H); 3,0-4,0 (17H); 4,5 (2H); 7,0-7,5 (7H); 8,0-9,0 (5H)

### Beispiel 45

### 4-(4-Henzylpiperazin-1-yl)nikotinsäure-N-(1-carbamoyl-1-oxo-3-phenylpropan-2-yl)amid

MS: m/e = 471

### Beispiel 46

### 2-(4-Pyrid-2-yl)piperazin-1-yl)nikotinsäure-N-(3-phenylpropan-1-al-2-yl)amid

MS: m/e = 415

### Beispiel 47

### 2-(4-(Pyrid-2-yl)piperazin-1-yl)nikotinsäure-N-(1-carbamoyl-1-oxo-3-phenylpropan-2-yl)amid

¹H-NMR (DMSO-D₆): δ= 2,9-4,1 (10H); 5,5 (1H); 6,7-8,2 (14H); 9,1 (1H)

### Beispiel 48

### 2-(4-Benzyl-piperazin-1-yl)nikotinsäure-N-(pentan-1-al-2-yl)amid

¹H-NMR (CDCl₃): δ= 1,0-2,0 (7H); 2,7-3,8 (8H); 4,8 (1H); 7,3-8,4 (8H); 9,7 (2H)

### Beispiel 49

### 2-(4-Benzyl-piperazin-1-yl)nikotinsäure-N-(3-(indol-3-yl)propan-1-al-2-yl)amid

¹H-NMR (CDCl₃): δ= 2,0-3,5 (12H); 5,0 (1H); 7,3-8,0 (13H); 8,4 (2H); 9,6-9,8 (2H)

### Beispiel 50

### 2-(4-(Picol-2-yl)homopiperazin-1-yl)nikotinsäure-N-(3-(indol-3-yl)propan-1-al-2-yl)amid

¹H-NMR (CDCl₃): δ= 1,7 (2H); 2,6-3,7 (12H); 5,0 (1H); 6,8-8,6 (14H); 9,8 (1H)

### Beispiel 51

### 2-(4-Benzylpiperazin-1-yl)pyridin-4-carbonsäure-N(1-carbamoyl-1-oxo-3-phenylpropan-2-yl)amid

MS: m/e = 471

### Beispiel 52

### 2-(4-Methylpiperazin-1-yl)chinolin-4-carbonsäure-N-(1-carbamoyl-1-oxo-3-phenylpropan-2-yl)amid

¹H-NMR (D₂O): δ= 2,8 (1H); 3,0 (3H); 3,2-4,5 (9H); 6,7-7,8 (10H)

### Beispiel 53

### 2-(4-Benzylhomopiperazin-1-yl)-pyridin-4-carbonsäure-N-(3-phenylpropan-1-al-2-yl)amid

MS: m/e = 442

**Tabelle**

| Nr. | 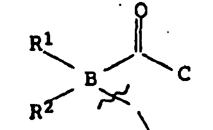 | 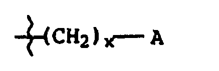 | R⁵ | R³ | R⁴ |
|---|---|---|---|---|---|
| 1 | 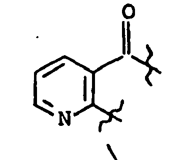 | 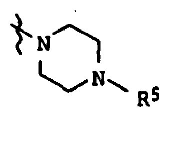 | 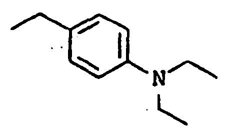 | 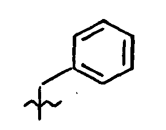 | H |
| 2 | 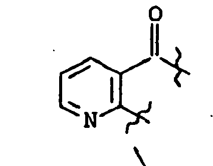 | 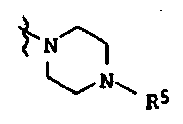 | 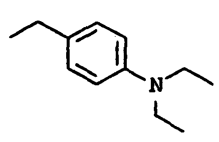 | 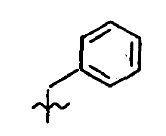 | CONH₂ |
| 3 | 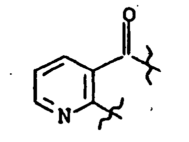 | 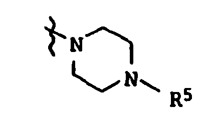 | 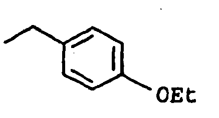 | 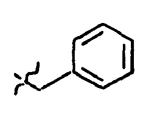 | H |
| 4 | 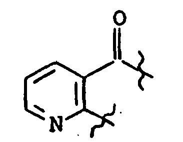 | 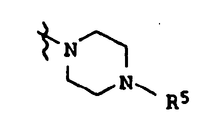 | 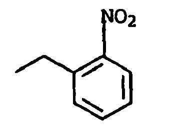 | 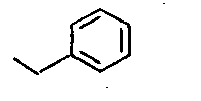 | H |
| 5 | 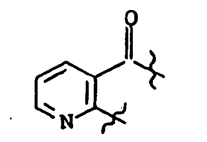 | 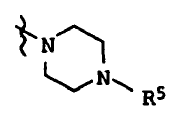 | 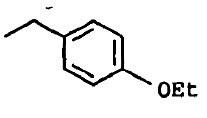 | 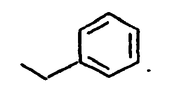 | CONH₂ |
| 6 | 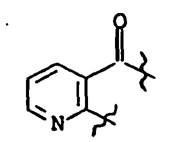 | 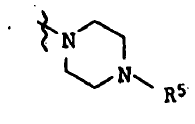 | 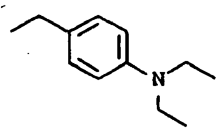 | 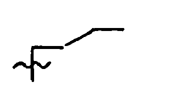 | H |
| 7 | 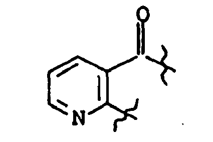 | 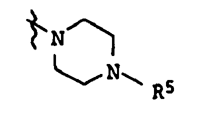 | 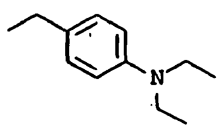 | 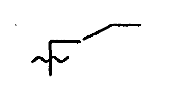 | CONH₂ |
| 8 | 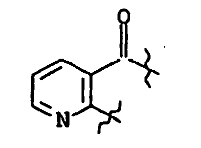 | 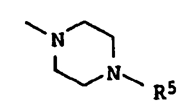 | 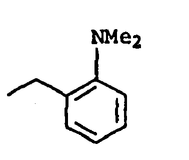 | 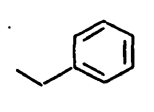 | H |
| 9 | 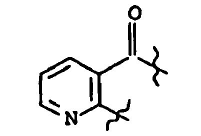 | 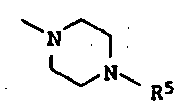 | 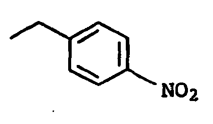 | 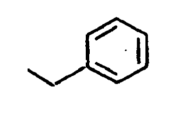 | H |
| 10 | 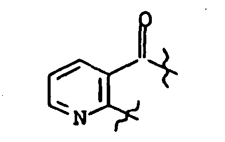 | 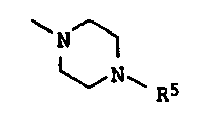 | 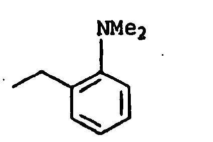 | 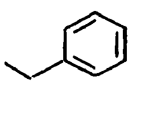 | CONH₂ |
| 11 | 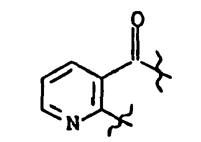 | 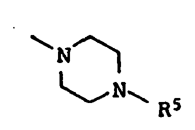 | 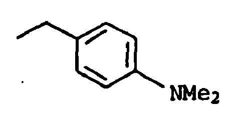 | 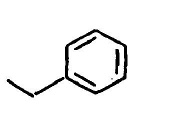 | CONH₂ |
| 12 | 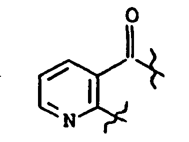 | 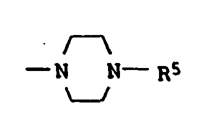 | 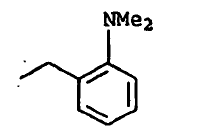 | 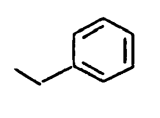 | H |
| 13 | 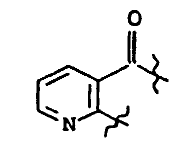 | 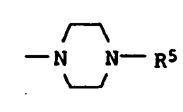 | 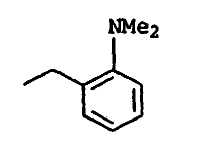 | 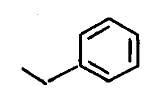 | CONH₂ |
| 14 | 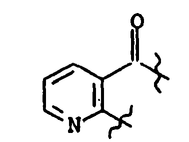 | 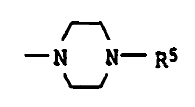 | 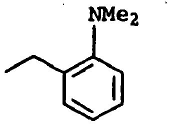 | 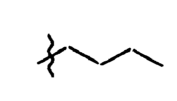 | H |
| 15 | 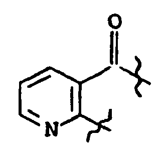 | 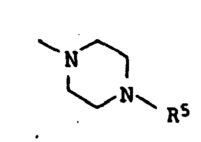 | 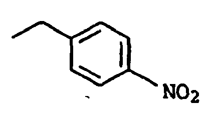 | 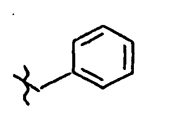 | CONH₂ |
| 16 | 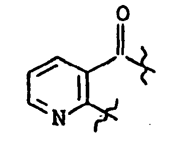 | 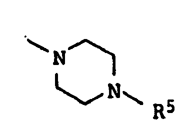 | 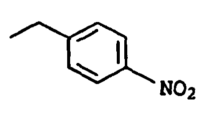 | 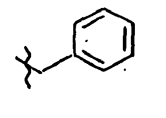 | CONH₂ |
| 17 | 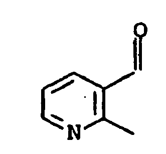 | 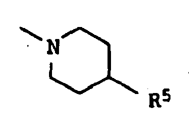 | 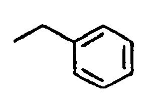 | 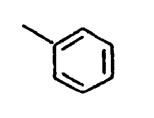 | H |
| 18 | 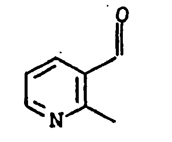 | 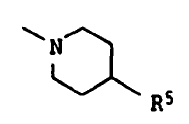 | 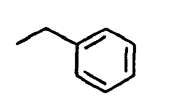 | 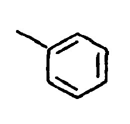 | CONH₂ |
| 19 | 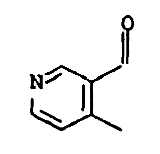 | 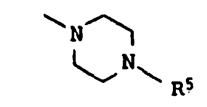 | 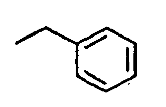 | 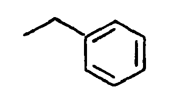 | H |
| 20 | 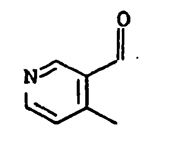 | 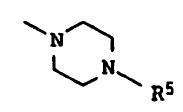 | 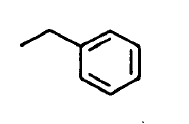 | 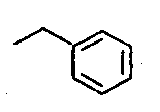 | CONH₂ |
| 21 | 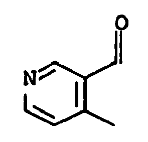 | 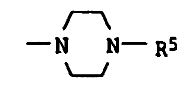 | 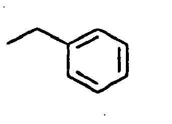 | 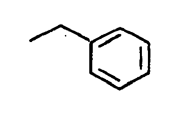 | H |
| 22 | 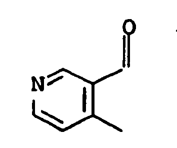 | 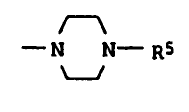 | 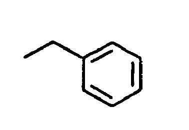 | 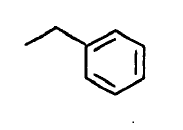 | CONH₂ |
| 23 | 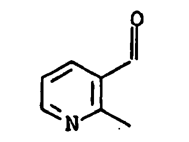 | 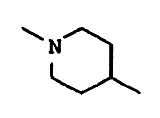 | 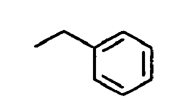 | 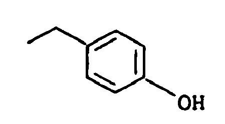 | H |
| 24 | 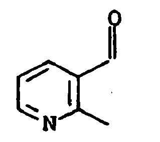 | 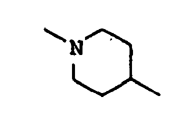 | 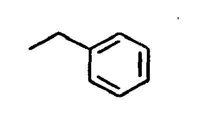 | 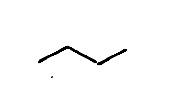 | H |
| 25 | 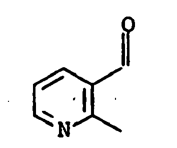 | 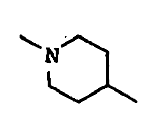 | 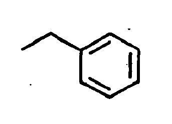 | 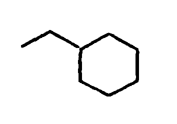 | H |
| 26 | 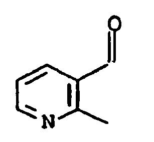 | 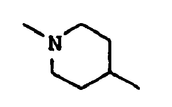 | 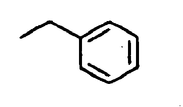 | 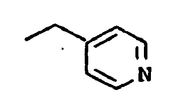 | H |
| 27 | 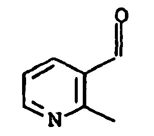 | 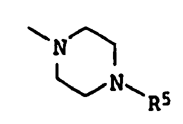 | 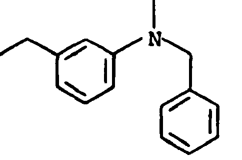 | 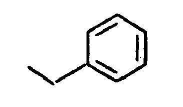 | H |
| 28 | 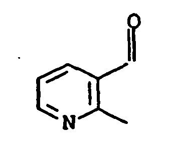 | 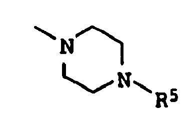 | 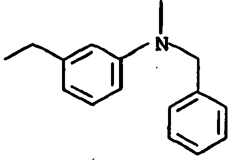 | 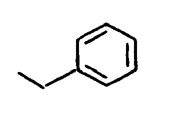 | H |
| 29 | 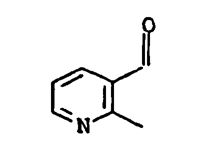 | 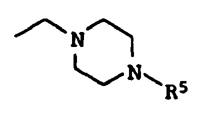 | 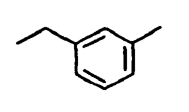 | 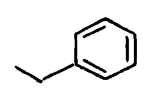 | CONH₂ |
| 30 | 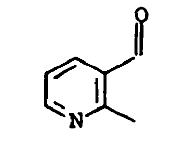 | 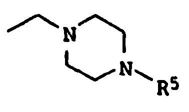 | 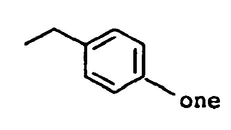 | 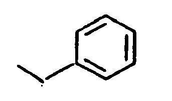 | CONH₂ |
| 31 | 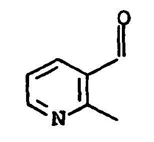 | 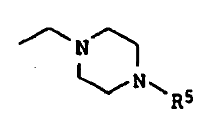 | 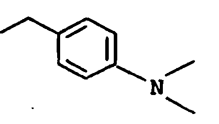 | 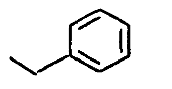 | CONH₂ |
| 32 | 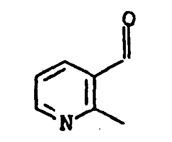 | 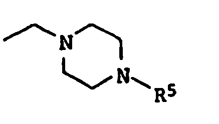 | 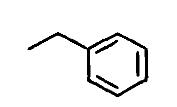 | 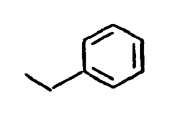 | CONH₂ |
| 33 | 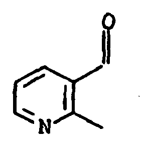 | 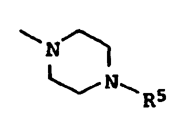 | 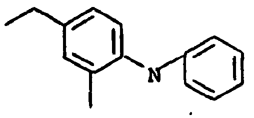 | 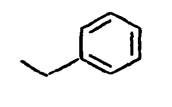 | H |
| 34 | 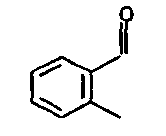 | 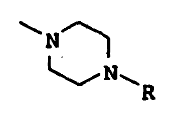 | 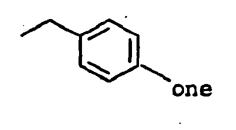 | 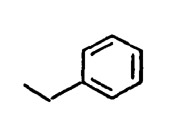 | H |
| 35 | 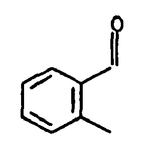 | 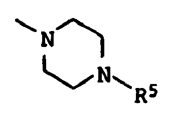 | 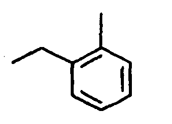 | 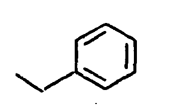 | H |
| 36 | 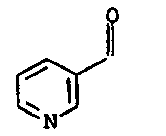 | 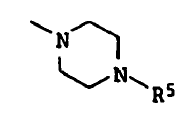 | 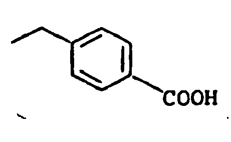 | Bn | H |
| 37 | 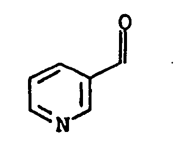 | 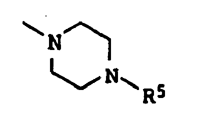 | 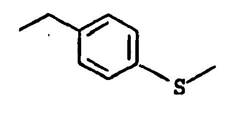 | Bn | H |
| 38 | 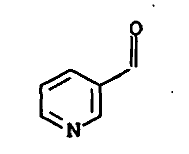 | 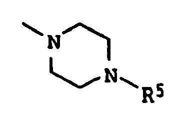 | 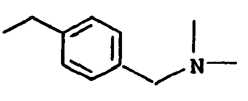 | Bn | H |
| 39 | 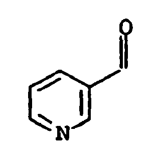 | 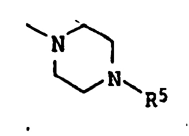 | 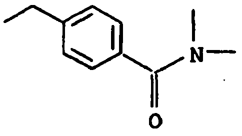 | Bn | H |
| 40 | 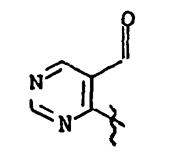 | 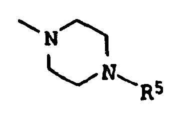 | 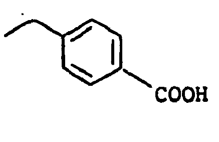 | Bn | H |
| 41 | 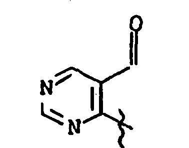 | 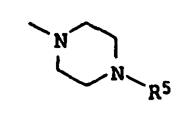 | 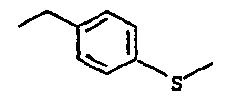 | Bn | H |
| 42 | 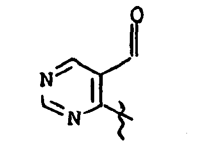 | 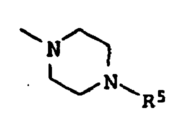 | 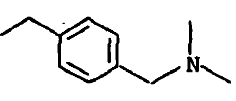 | Bn | H |
| 43 | 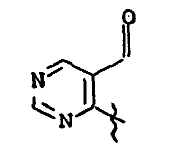 | 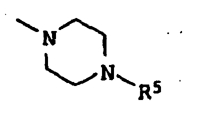 | 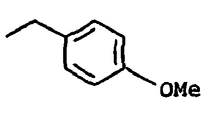 | Bn | H |
| 44 | 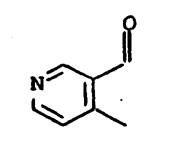 | 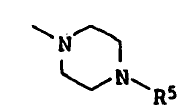 | 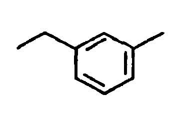 | Bn | H |
| 45 | 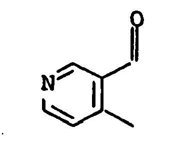 | 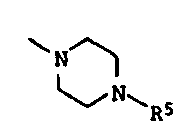 | 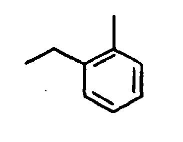 | Bn | H |
| 46 | 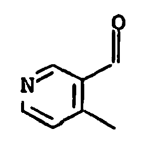 | 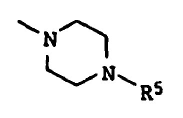 | 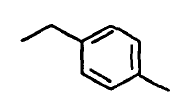 | Bn | H |
| 47 | 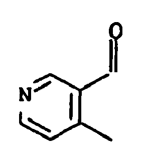 | 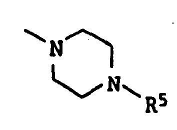 | 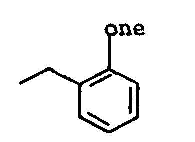 | Bn | H |
| 48 | 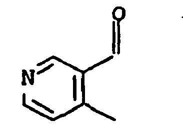 | 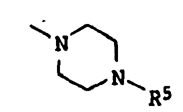 | 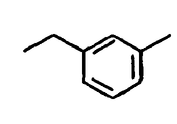 | Bn | H |
| 49 | 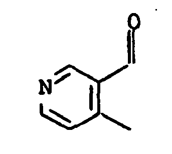 | 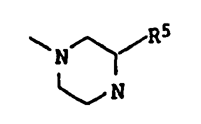 | 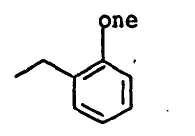 | Bn | H |
| 50 | 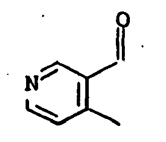 | 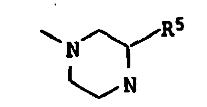 | 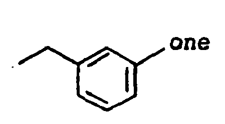 | Bn | H |
| 51 | 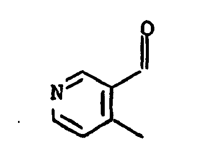 | 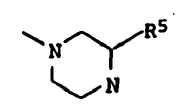 | 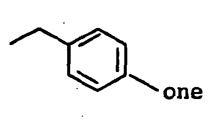 | Bn | H |
| 52 | 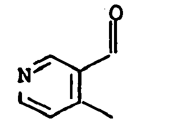 | 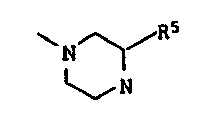 | 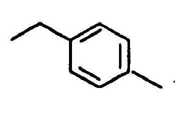 | Bn | H |
| 53 | 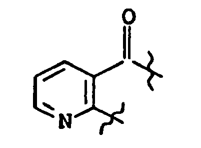 | 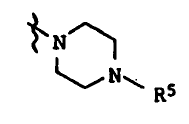 | 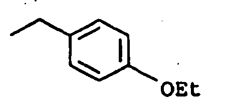 | 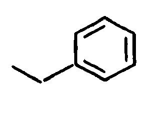 | CONH₂ |
| 54 | 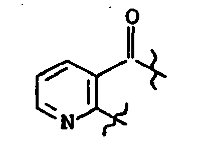 | 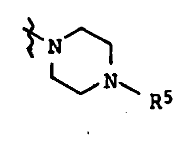 | 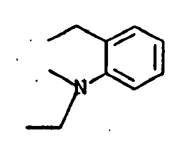 | 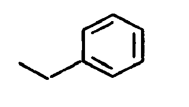 | CONH₂ |
| 55 | 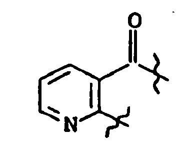 | 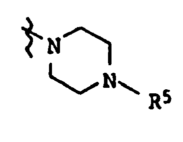 | 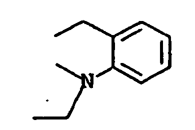 | 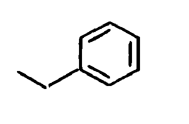 | H |
| 56 | 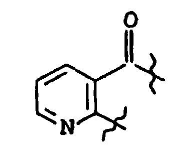 | 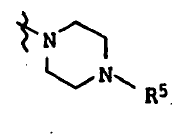 | 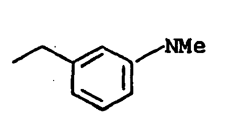 | 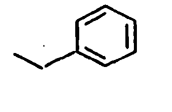 | CONH₂ |
| 57 | 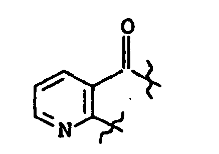 | 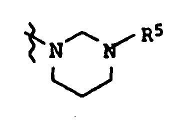 | 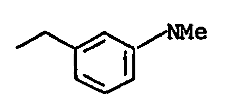 | 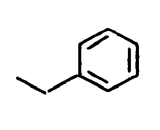 | H |
| 58 | 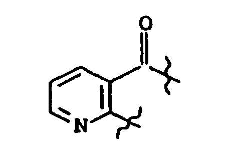 | 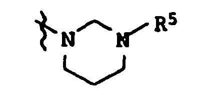 | 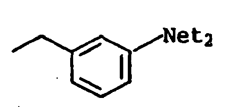 | 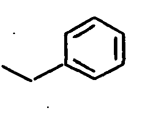 | H |
| 59 | 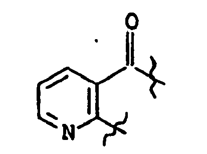 | 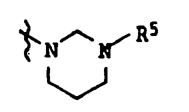 | 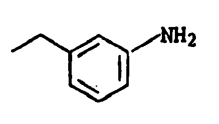 | 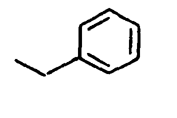 | CONH₂ |
| 60 | 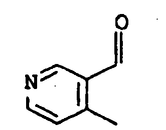 | 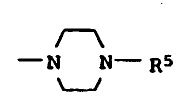 | 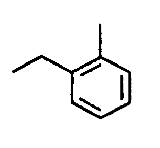 | 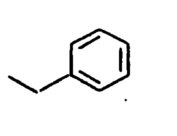 | H |
| 61 | 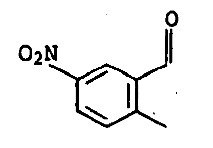 | 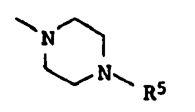 | 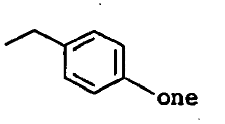 | 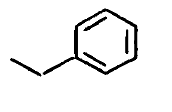 | H |
| 62 | 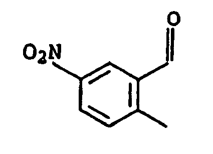 | 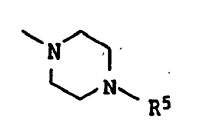 | 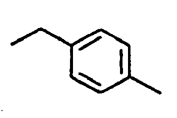 | 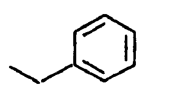 | H |
| 63 | 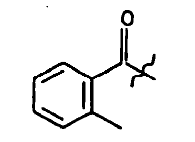 | 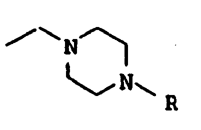 | 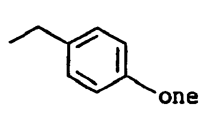 | 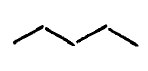 | H |
| 64 | 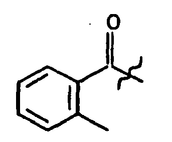 | 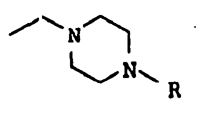 | 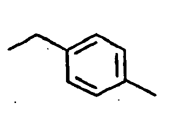 | 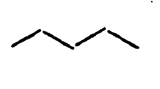 | H |
| 65 | 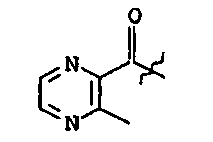 | 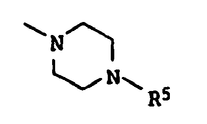 | 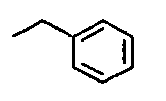 | 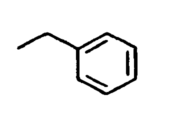 | H |
| 66 | 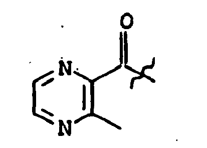 | 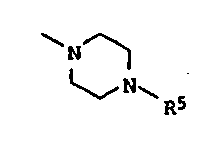 | 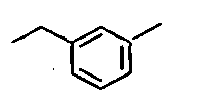 | 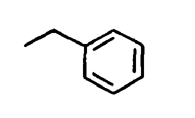 | H |
| 67 | 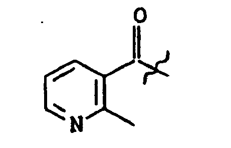 | 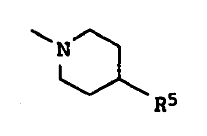 | 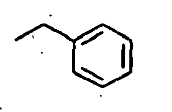 | 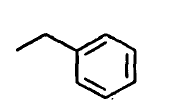 | H |
| 68 | 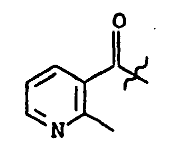 | 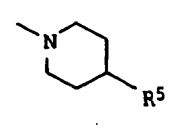 | 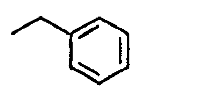 | 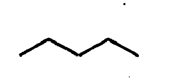 | H |
| 69 | 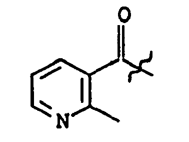 | 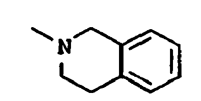 | H | 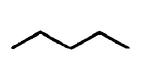 | H |
| 70 | 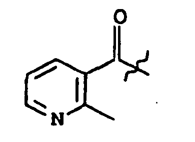 | 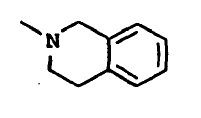 | H | 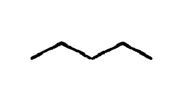 | CONH² |
| 71 | 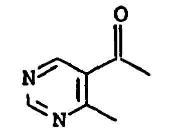 | 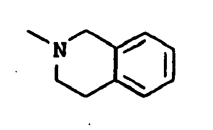 | H | 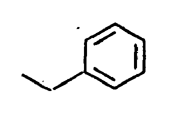 | CONH² |
| 72 | 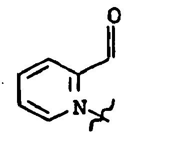 | 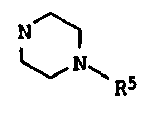 | 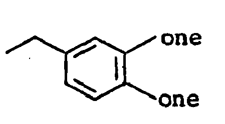 | 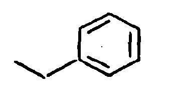 | H |
| 73 | 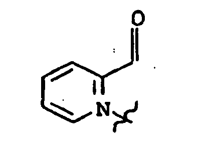 | 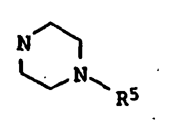 | 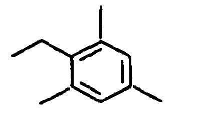 | 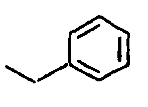 | H |
| 74 | 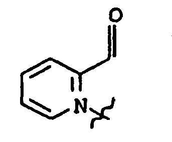 | 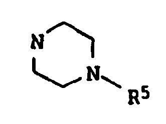 | 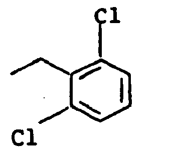 | 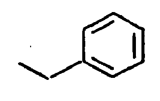 | H |
| 75 | 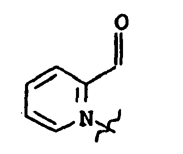 | 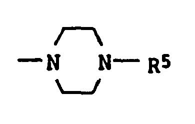 | 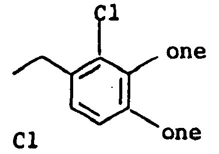 | 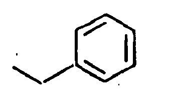 | H |
| 76 | 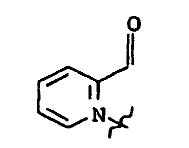 | 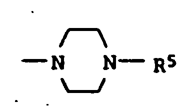 | 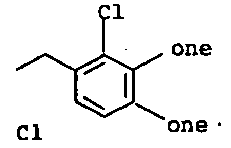 | 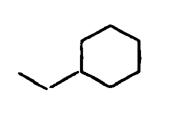 | H |
| 77 | 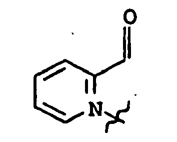 | 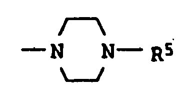 | 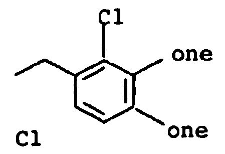 | 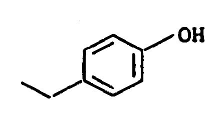 | H |
| 78 | 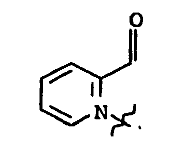 | 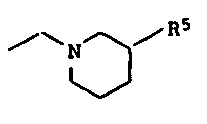 | 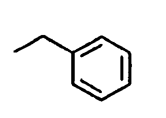 | 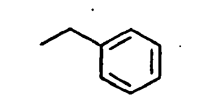 | H |
| 79 | 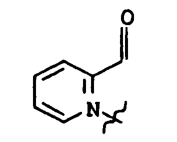 | 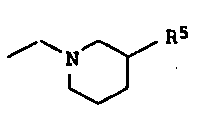 | 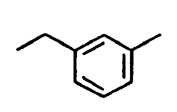 | 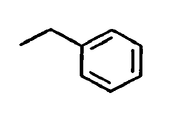 | H |
| 80 | 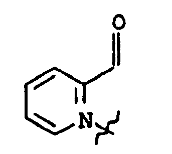 | 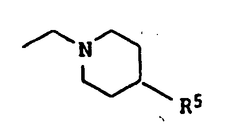 | 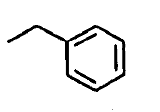 | 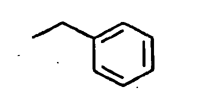 | H |
| 81 | 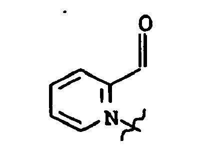 | 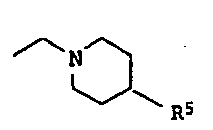 | 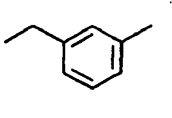 | 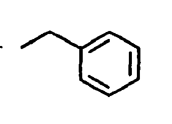 | H |
| 82 | 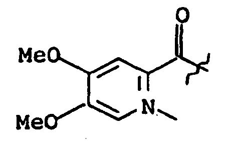 | 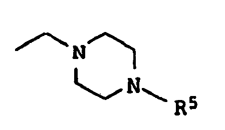 | 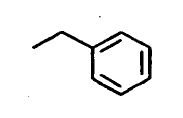 | 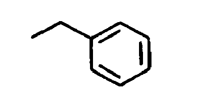 | H |
| 83 | 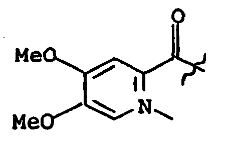 | 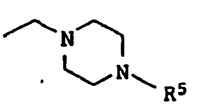 | 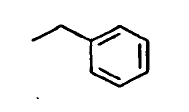 | 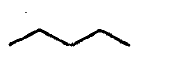 | H |
| 84 | 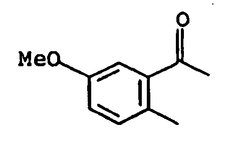 | 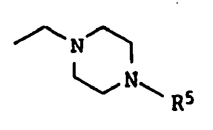 | 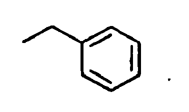 | 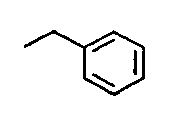 | H |
| 85 | 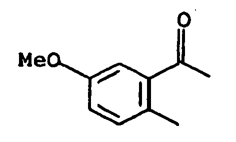 | 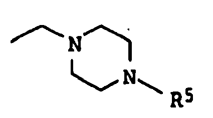 | 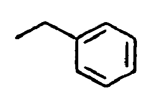 | 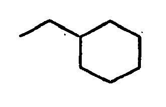 | H |
| 86 | 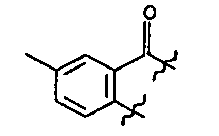 | 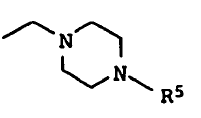 | 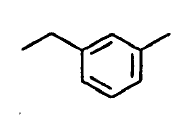 | 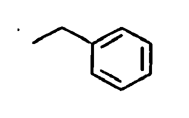 | H |
| 87 | 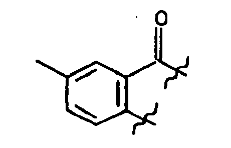 | 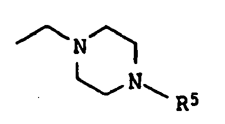 | 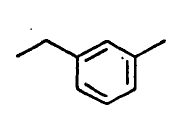 | 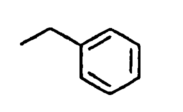 | CONH₂ |
| 88 | 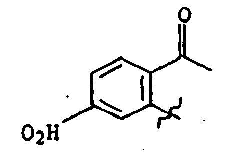 | 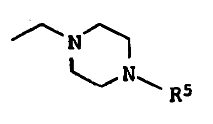 | 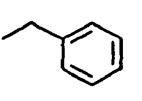 | 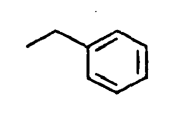 | H |
| 89 | 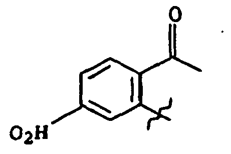 | 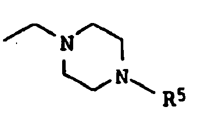 | 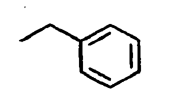 | 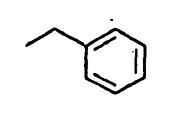 | CONH₂ |
| 90 | 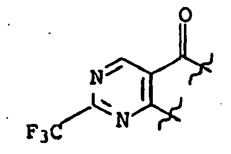 | 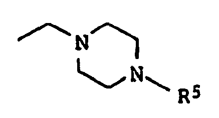 | 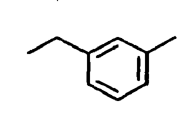 | 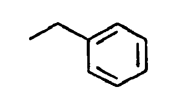 | H |
| 91 | 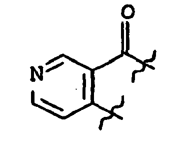 | 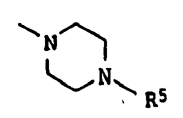 | 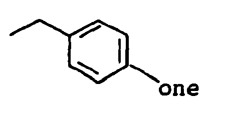 | Bn | CONH₂ |
| 92 | 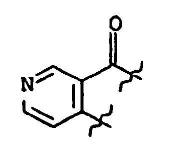 | 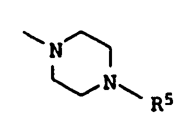 | 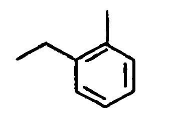 | Bn | CONH₂ |
| 93 | 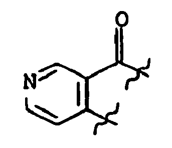 | 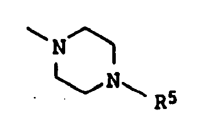 | 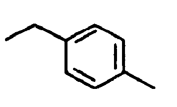 | Bn | CONH₂ |
| 94 | 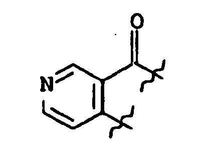 | 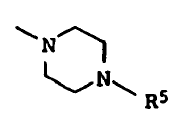 | 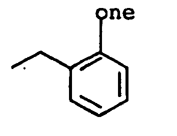 | Bn | CONH₂ |
| 95 | 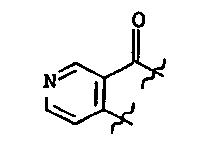 | 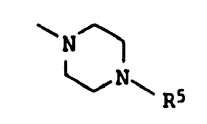 | 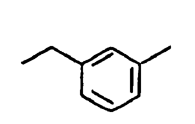 | Bn | CONH₂ |
| 96 | 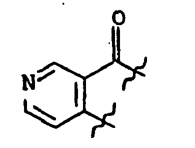 | 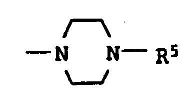 | 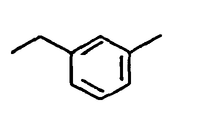 | Bn | CONH₂ |
| 97 | 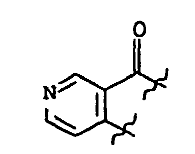 | 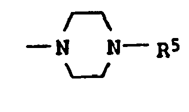 | 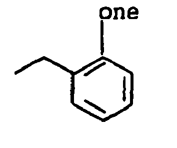 | Bn | CONH₂ |
| 98 | 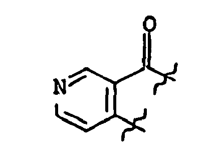 | 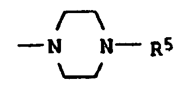 | 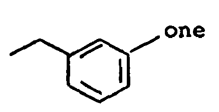 | Bn | CONH₂ |
| 99 | 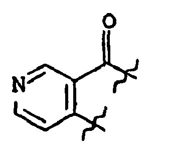 | 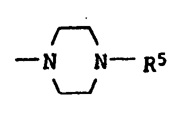 | 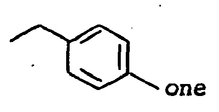 | Bn | CONH₂ |
| 100 | 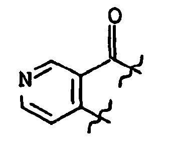 | 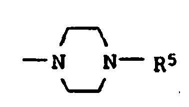 | 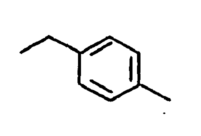 | Bn | CONH₂ |
| 101 | 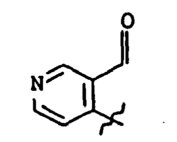 | 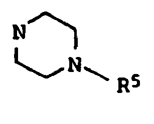 | 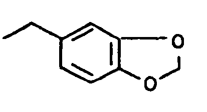 | 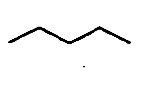 | H |
| 102 | 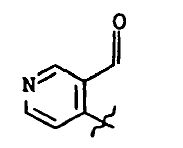 | 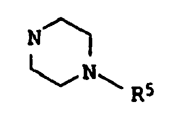 | 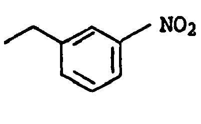 | 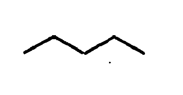 | H |
| 103 | 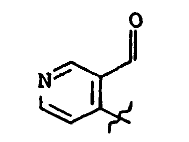 | 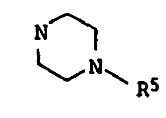 | 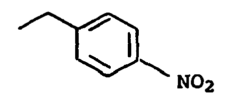 | 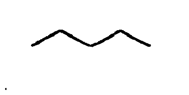 | H |
| 104 | 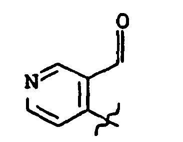 | 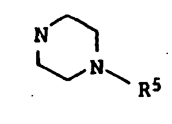 | 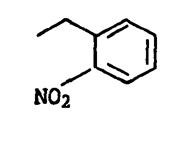 | 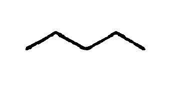 | H |
| 105 | 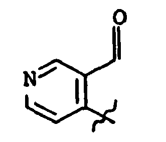 | 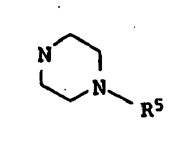 | 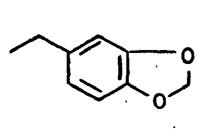 | 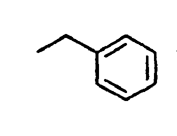 | CONH₂ |
| 106 | 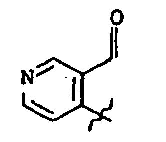 | 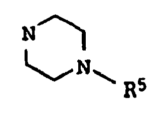 | 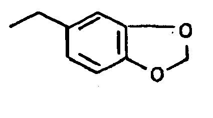 | 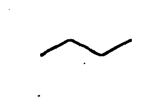 | H |
| 107 | 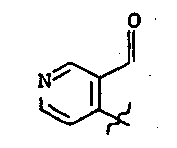 | 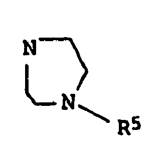 | 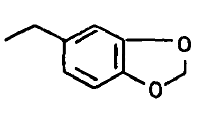 | 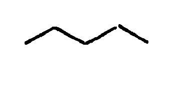 | CONH₂ |
| 108 | 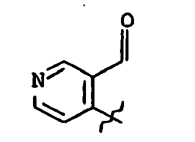 | 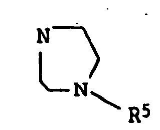 | 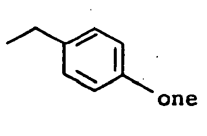 | 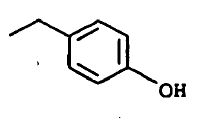 | CONH₂ |
| 109 | 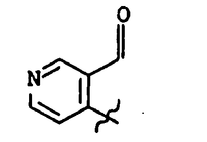 | 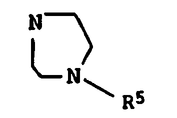 | 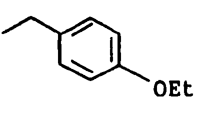 | 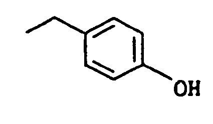 | CONH₂ |
| 110 | 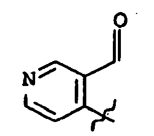 | 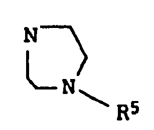 | 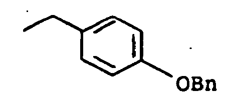 | 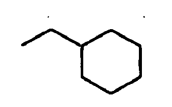 | CONH₂ |
| 111 | 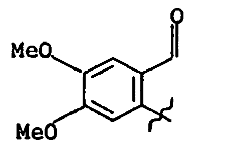 | 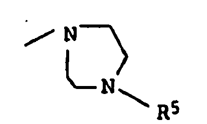 | 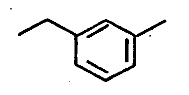 | 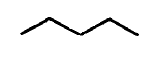 | 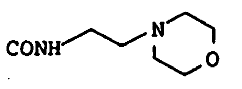 |
| 112 | 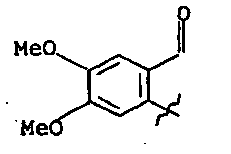 | 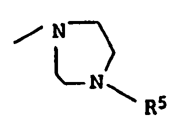 | 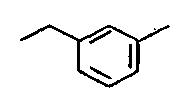 | 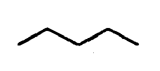 | 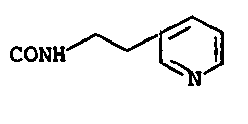 |
| 113 | 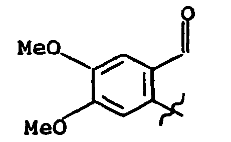 | 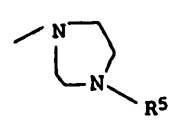 | 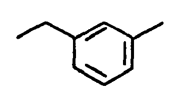 | 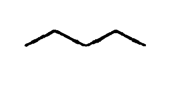 | 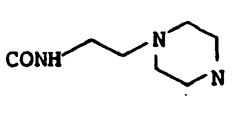 |
| 114 | 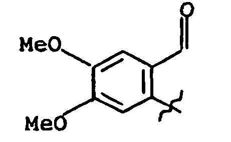 | 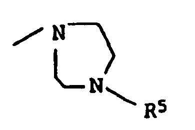 | 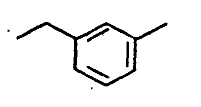 | 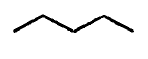 | 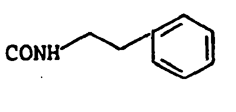 |
| 115 | 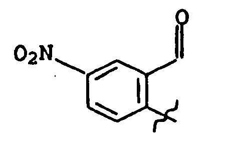 | 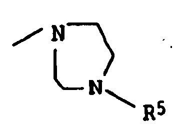 | 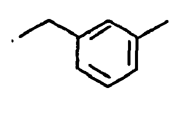 | 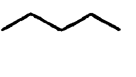 | 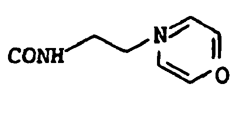 |
| 116 | 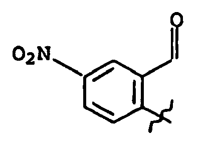 | 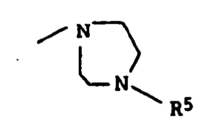 | 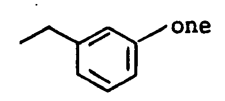 | 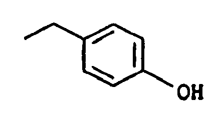 | CONH₂ |
| 117 | 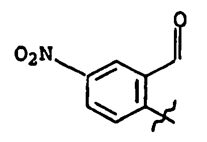 | 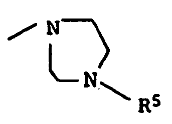 | 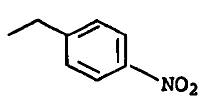 | 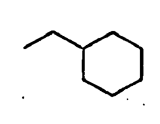 | CONH₂ |
| 118 | 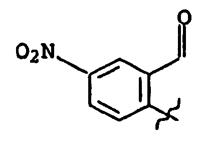 | 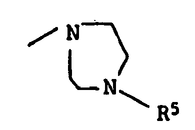 | 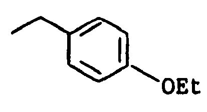 | 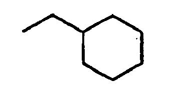 | CONH₂ |
| 119 | 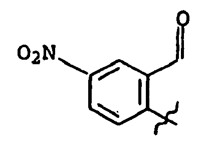 | 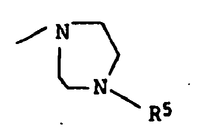 | 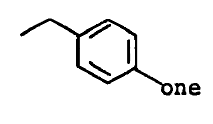 | 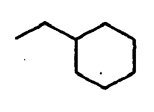 | 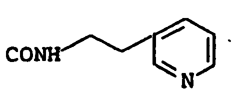 |
| 120 | 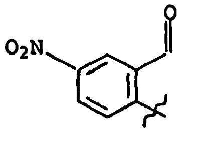 | 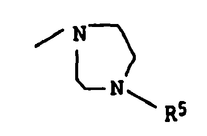 | 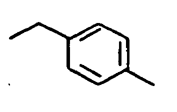 | 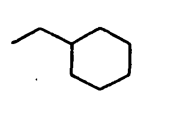 | 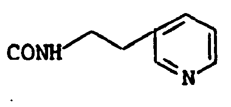 |
| 121 | 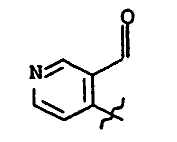 | 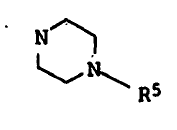 | 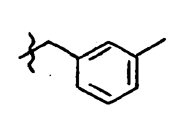 | 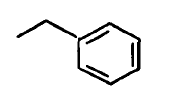 | H |
| 122 | 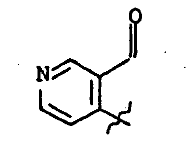 | 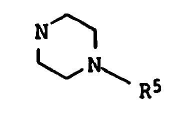 | 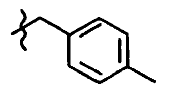 | 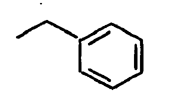 | H |
| 123 | 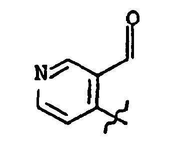 | 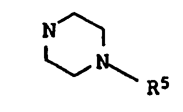 | 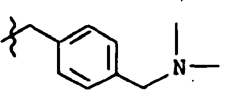 | 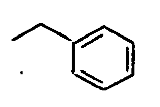 | H |
| 124 | 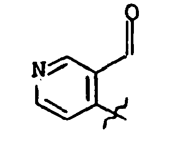 | 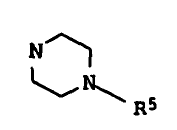 | 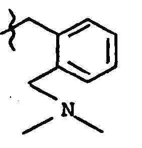 | 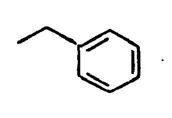 | H |
| 125 | 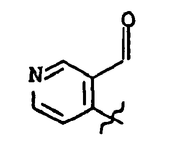 | 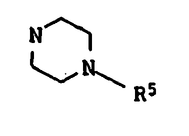 | 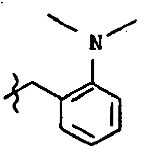 | 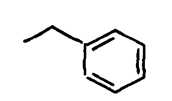 | H |
| 126 | 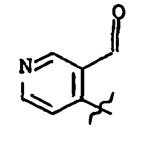 | 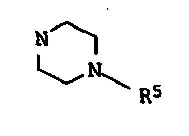 | 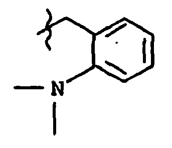 | 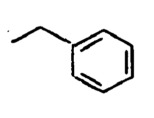 | H |
| 127 | 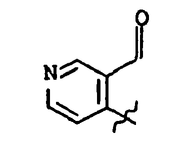 | 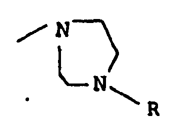 | 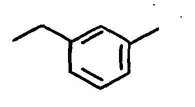 | 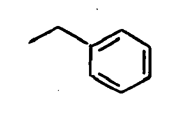 | H |
| 128 | 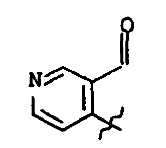 | 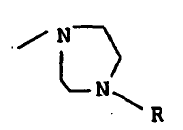 | 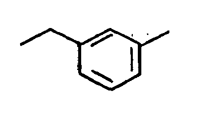 | 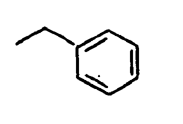 | CONH₂ |
| 129 | 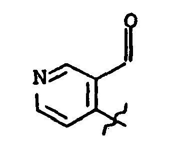 | 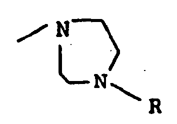 | 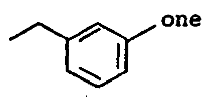 | 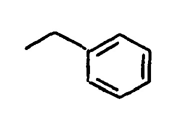 | H |
| 130 | 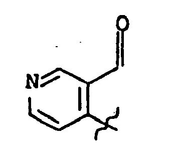 | 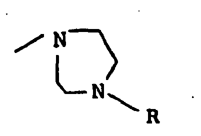 | 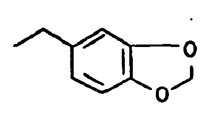 | 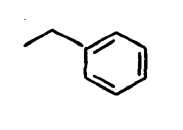 | H |
| 131 | 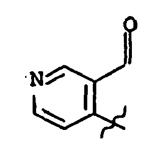 | 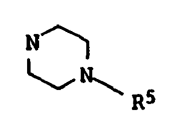 | 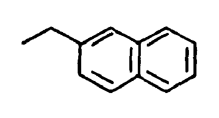 | 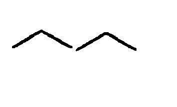 | CONH₂ |
| 132 | 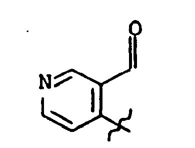 | 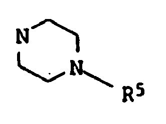 | 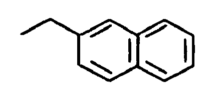 | 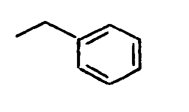 | CONH₂ |
| 133 | 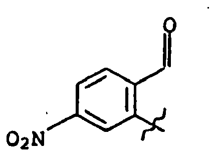 | 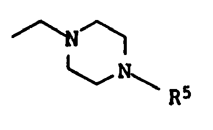 | 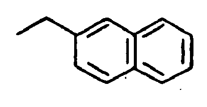 | 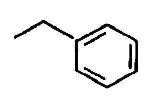 | CONH₂ |
| 134 | 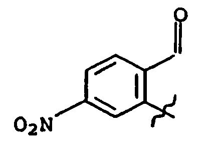 | 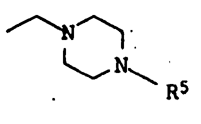 | 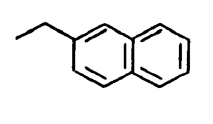 | 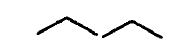 | CONH₂ |
| 135 | 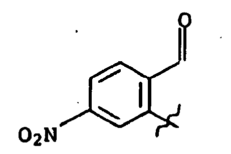 | 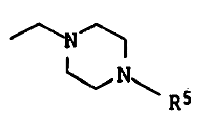 | 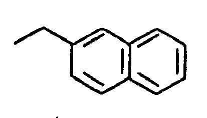 | 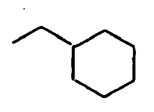 | CONH₂ |
| 136 | 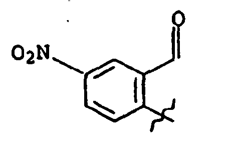 | 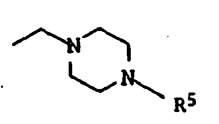 | 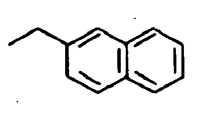 | 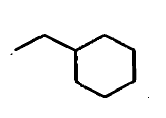 | CONH₂ |
| 137 | 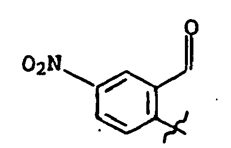 | 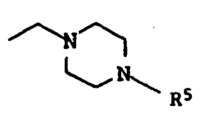 | 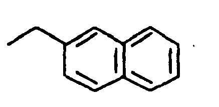 | 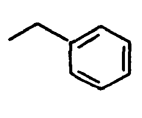 | CONH₂ |
| 138 | 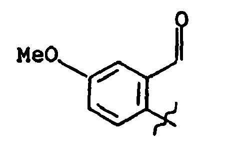 | 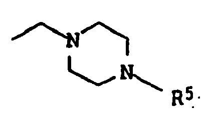 | 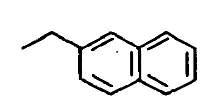 | 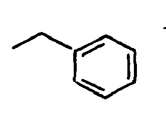 | CONH₂ |
| 139 | 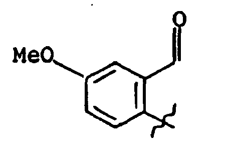 | 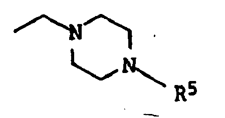 | 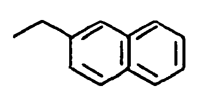 | 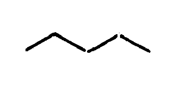 | CONH₂ |
| 140 | 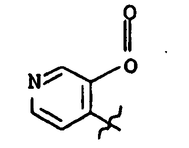 | 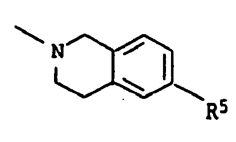 | | 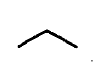 | H |
| 141 | | | H | 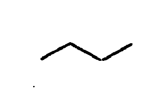 | H |
| 142 | 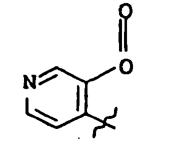 | 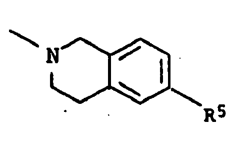 | H | | |
| 143 | 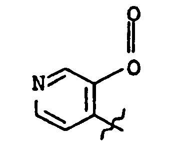 | 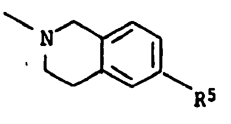 | H | 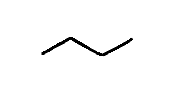 | CONH₂ |
| 144 | 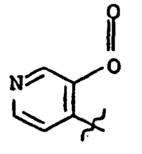 | 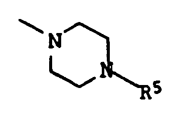 | 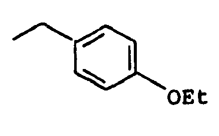 | 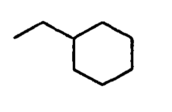 | CONH₂ |
| 145 | 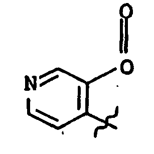 | 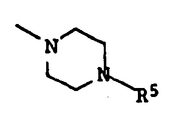 | 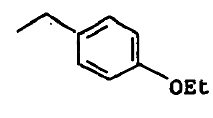 | 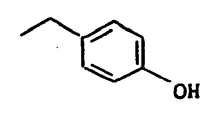 | CONH₂ |
| 146 | 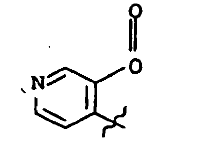 | 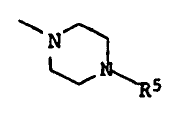 | 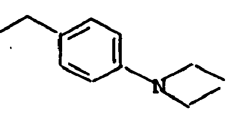 | 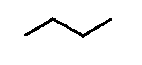 | CONH₂ |
| 147 | 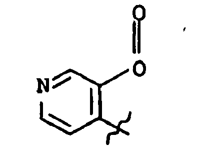 | 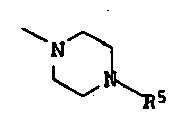 | 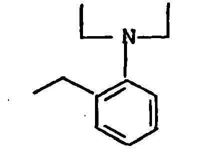 | 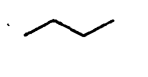 | CONH₂ |
| 148 | 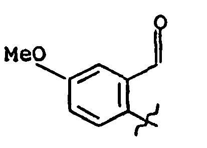 | 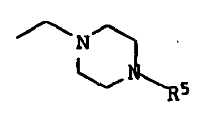 | 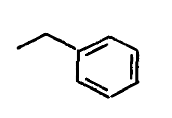 | 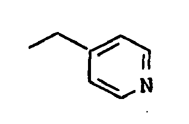 | H |
| 149 | 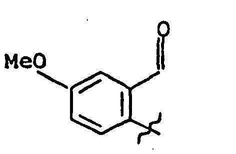 | 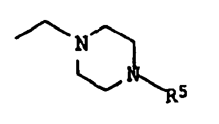 | 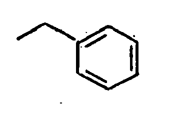 | 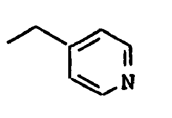 | CONH₂ |
| 150 | 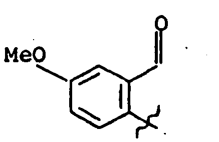 | 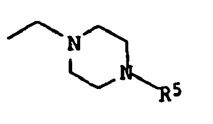 | 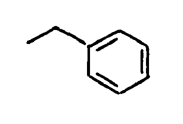 | 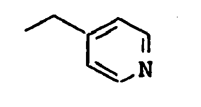 | 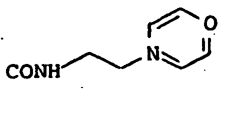 |
| 151 | 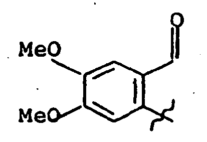 | 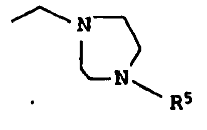 | 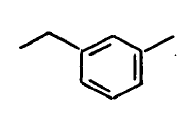 | 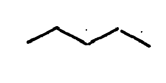 | H |
| 152 | 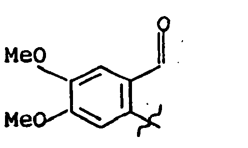 | 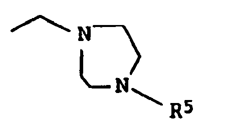 | 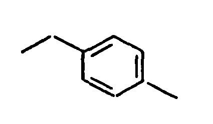 | 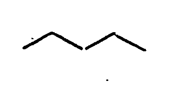 | H |
| 153 | 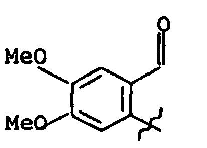 | 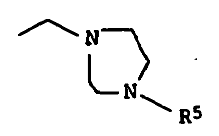 | 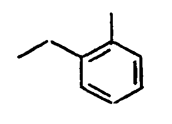 | 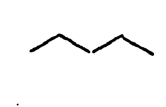 | H |
| 154 | 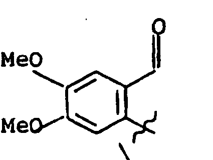 | 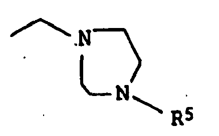 | 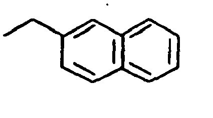 | 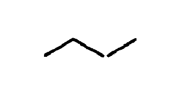 | H |
| 155 | 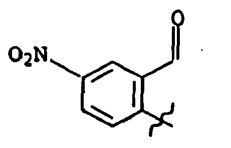 | 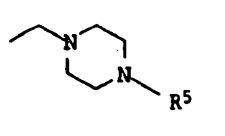 | 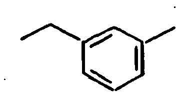 | 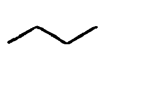 | H |
| 156 | 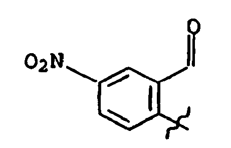 | 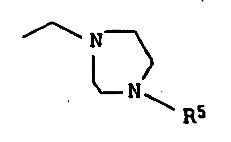 | 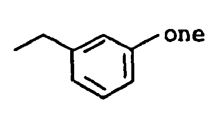 | 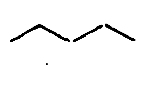 | H |
| 157 | 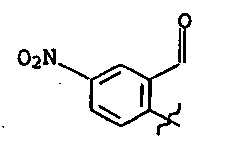 | 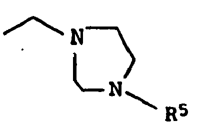 | 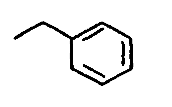 | 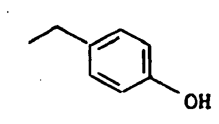 | H |
| 158 | 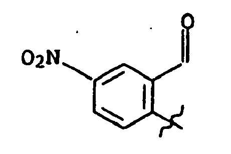 | 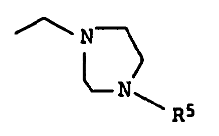 | 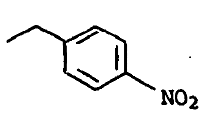 | 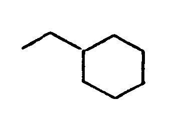 | H |
| 159 | 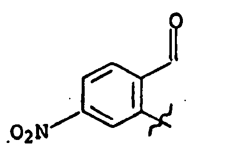 | 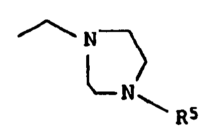 | 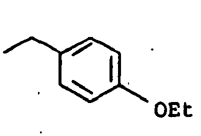 | 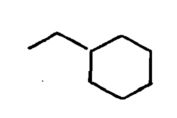 | H |
| 160 | 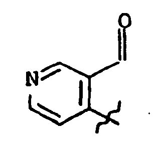 | 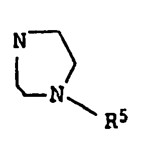 | 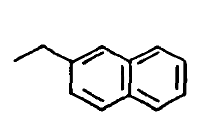 | 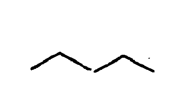 | H |
| 161 | 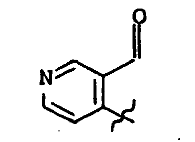 | 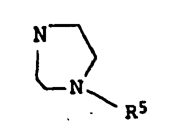 | 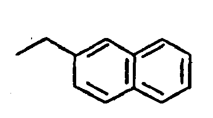 | 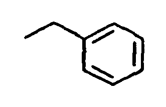 | H |
| 162 | 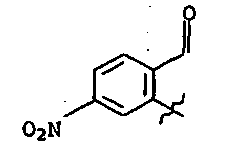 | 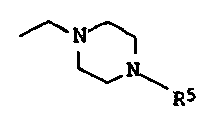 | 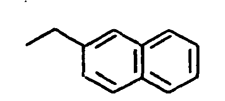 | 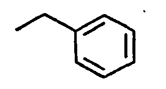 | H |
| 163 | 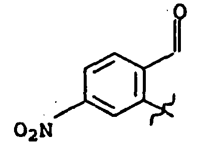 | 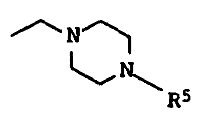 | 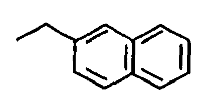 | 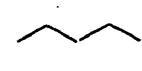 | H |
| 164 | 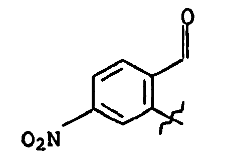 | 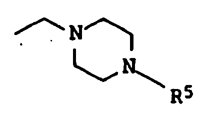 | 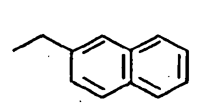 | 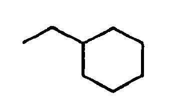 | H |
| 165 | 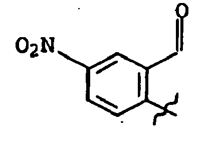 | 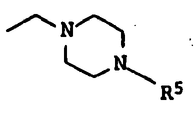 | 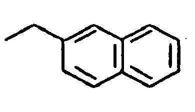 | 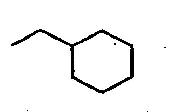 | H |
| 166 | 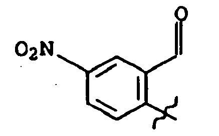 | 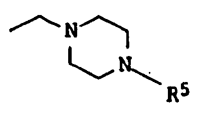 | 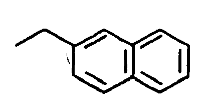 | 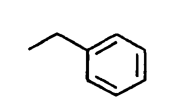 | H |
| 167 | 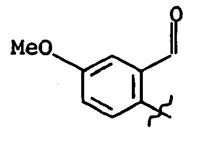 | 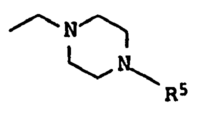 | 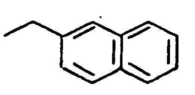 | 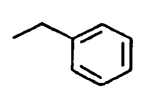 | H |
| 168 | 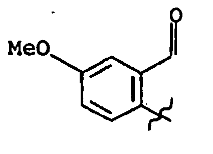 | 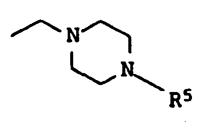 | 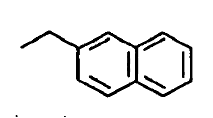 | 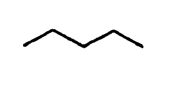 | H |
| 169 | 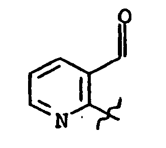 | 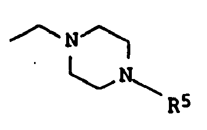 | 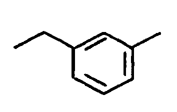 | 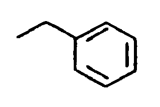 | 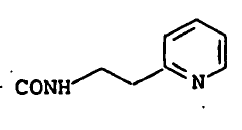 |
| 170 | 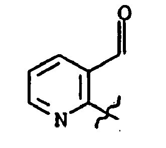 | 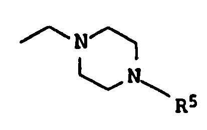 | 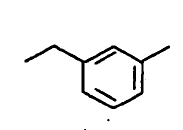 | 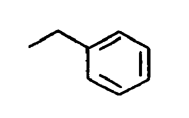 | 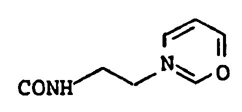 |
| 171 | 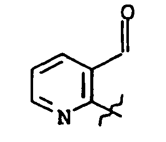 | 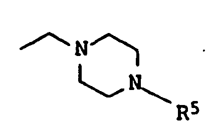 | 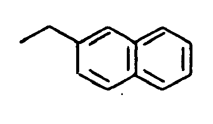 | 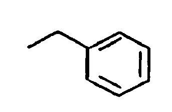 | 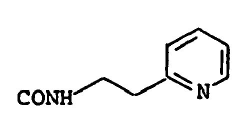 |
| 172 | 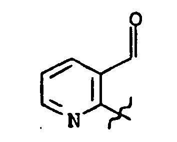 | 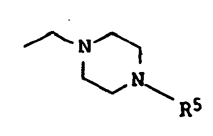 | 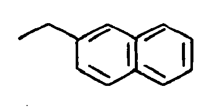 | 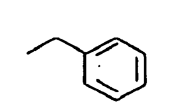 | 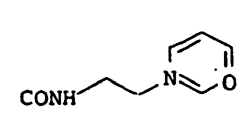 |
| 173 | 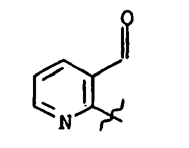 | 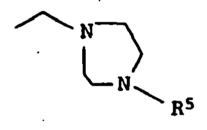 | 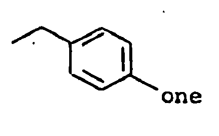 | 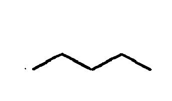 | 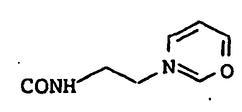 |
| 174 | 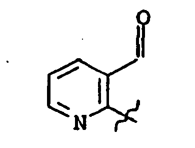 | 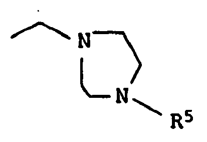 | 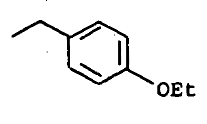 | 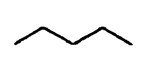 | 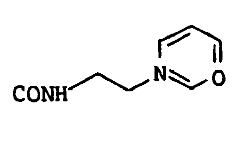 |
| 175 | 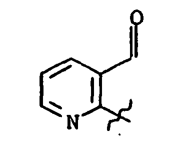 | 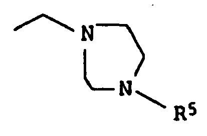 | 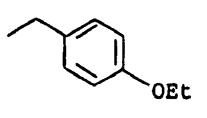 | 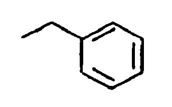 | 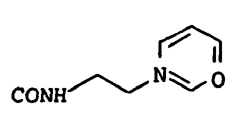 |
| 176 | 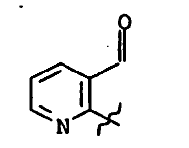 | 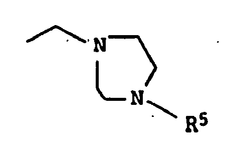 | 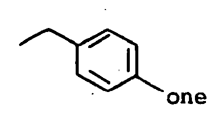 | 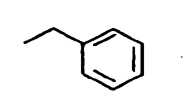 | 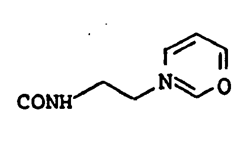 |
| 177 | 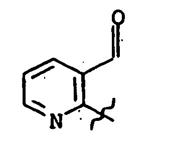 | 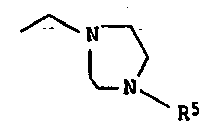 | 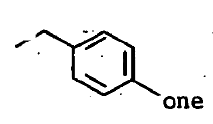 | 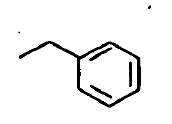 | 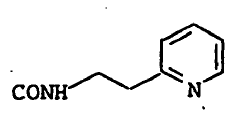 |
| 178 | 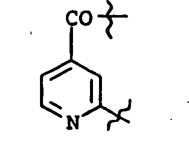 | 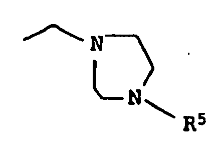 | Bu | Bu | 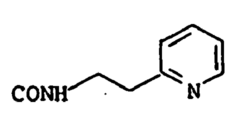 |
| 179 | 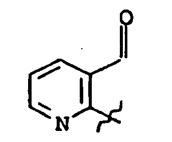 | 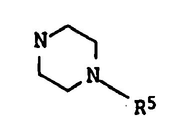 | 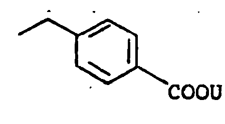 | Bn | CONH₂ |
| 180 | 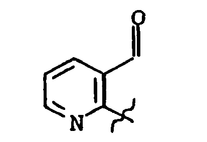 | 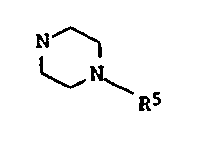 | 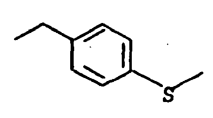 | Bn | CONH₂ |
| 181 | 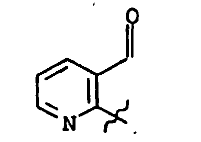 | 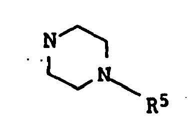 | 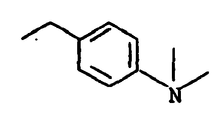 | Bn | CONH₂ |
| 182 | 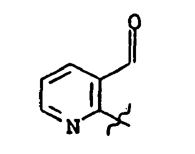 | 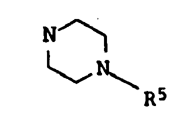 | 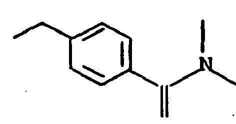 | Bn | CONH₂ |
| 183 | 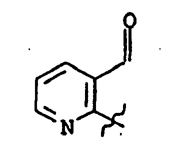 | 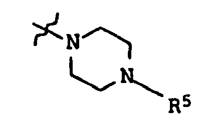 | 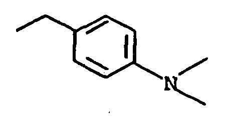 | Bn | H |
| 184 | 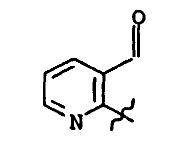 | 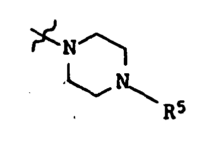 | 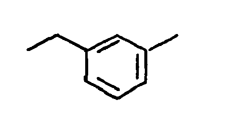 | Bn | CONH₂ |
| 185 | 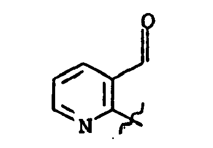 | 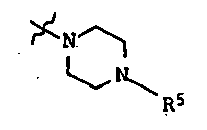 | 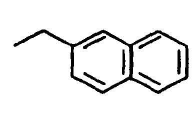 | Bn | CONH₂ |
| 186 | 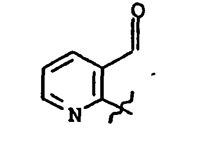 | 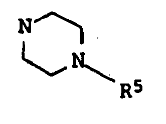 | 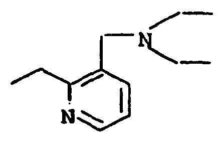 | Bn | CONH₂ |
| 187 | 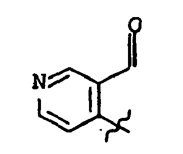 | 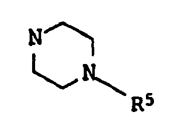 | 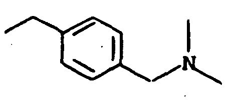 | Bn | CONH₂ |

## Patentansprüche

1. Heterocyclisch substituierte Amide der allgemeinen Formel I 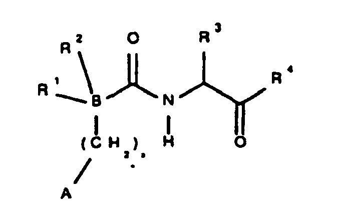 und ihre tautomeren und isomeren Formen, enantiomeren und diastereomeren Formen, sowie physiologisch verträgliche Salze, worin die Variablen folgende Bedeutung haben:
A Piperazin, Homopiperazin, Hexahydroazepin Piperidin und Pyrrolidin, die noch einen Rest R⁵ tragen können, bedeutet und
B einen Phenyl-, Pyridin-, Pyrimidin-, Pyrazin- oder Pyridazin-Ring darstellt und
R¹ und R² unabhängig voneinander Wasserstoff, C₁-C₆-Alkyl, verzweigt oder unverzweigt, O-C₁-C₆-Alkyl, verzweigt oder unverzweigt, OH, Cl, F, Br, J, CF₃, NO₂, NH₂, CN, COOH, COO-C₁-C₄-Alkyl, NHCO-C₁-C₄-Alkyl, NHCO-Phenyl, CONHR⁹, NHSO₂-C₁-C₄-Alkyl, NHSO₂-Phenyl, SO₂-C₁-C₄-Alkyl und SO₂-Phenyl bedeuten und R¹ und R² eine Kette -CH=CH-CH=CH- sein kann, die noch ein oder zwei Substituenten R⁶ tragen kann, und
R³ C₁-C₆-Alkyl, verzweigt oder unverzweigt, das noch einen S-CH₃-Rest, Cyclohexyl, Cyclopentyl, Cycloheptyl, Phenyl-, Pyridyl-, Pyrimidyl-, Pyridazyl-, Pyrazyl-, Indolyl-, Thienyl oder Naphthyl-Ring tragen kann, wobei die Ringe mit maximal zwei Resten R⁷ substituiert sind und R⁷ Wasserstoff, C₁-C₄-Alkyl, verzweigt oder unverzweigt, O-C₁-C₄-Alkyl, OH, Cl, F, Br, J, CF₃, NO₂, NH₂, CN, COOH, COO-C₁-C₄-Alkyl, CONHR⁹, NHCO-C₁-C₄-Alkyl, NHCO-Phenyl, NHSO₂-C₁-C₄-Alkyl, NHSO₂-Phenyl, SO₂-C₁-C₄-Alkyl und SO₂-Phenyl bedeutet, und
R⁴ Wasserstoff, -COR⁸ bedeutet, wobei R⁸ -OR⁹ , und -NR⁹R¹⁰ sein kann und
R⁵ Wasserstoff, C₁-C₆-Alkyl, verzweigt oder unverzweigt, das noch einen Substituenten R¹¹ tragen kann, oder R⁵ Phenyl-, Pyridyl-, Pyrimidyl-, Pyridazyl-, Pyrazinyl-, Pyrazyl-, Naphtyl, Thienyl, Piperidinyl, Pyrrolidinyl, Imidazyl-Ring sein kann, der noch einen oder zwei Substituenten R⁶ tragen kann, und
R⁶ Wasserstoff, C₁-C₆-Alkyl, verzweigt oder unverzweigt, O-C₁-C₆-Alkyl, verzweigt oder unverzweigt, OH, Cl, F, Br, J, CF₃, NO₂, NH₂, CN, COOH, COO-C₁-C₄-Alkyl und C₁-C₄-Alkyl-NR⁹R¹³ bedeutet oder zwei Reste R⁶ eine Brücke OC(R⁹)₂O darstellen kann und
R⁹ Wasserstoff, C₁-C₆-Alkyl, verzweigt und unverzweigt, bedeutet und
R¹⁰ Wasserstoff, C₁-C₆-Alkyl, verzweigt oder unverzweigt, bedeutet, das noch mit einen Phenylring, der noch einen Rest R¹² tragen kann, und mit 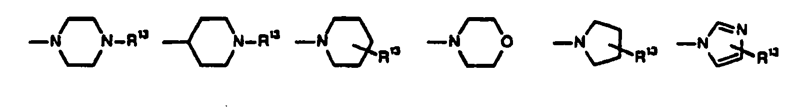 substituiert sein kann, und
R¹¹ ein Phenyl-, Pyridyl-, Pyrimidyl-, Naphtyl, Thienyl, Furyl, Pyridazyl-, Pyrazinyl-, Pyrazyl-, Pyrrolyl-, Imidazyl-Ring sein kann, der noch einen oder zwei Substituenten R⁶ tragen kann, und
R¹² Wasserstoff, C₁-C₆-Alkyl, verzweigt oder unverzweigt, O-C₁-C₆-Alkyl, verzweigt oder unverzweigt, OH, Cl, F, Br, J, CF₃, NO₂, NH₂, CN, COOH, COO-C₁-C₄-Alkyl bedeutet und
R¹³ Wasserstoff, eine C₁-C₄-Alkylkette und C₀-C₄-Alkylphenyl-, wobei der Phenylring noch ein oder zwei Resten R¹² tragen kann, bedeutet und
x eine Zahl 0, 1 und 2 bedeutet.

2. Heterocyclisch substituierte Amide der Formel I gemäß dem Anspruch 1, wobei
R⁴ Wasserstoff bedeutet.

3. Heterocyclisch substituierte Amide der Formel I gemäß dem Anspruch 1, wobei
R⁴ CONR⁹R¹⁰ bedeutet.

4. Heterocyclisch substituierte Amide der Formel I gemäß dem Anspruch 1, wobei
B Pyridin oder Phenyl und
R⁴ Wasserstoff bedeutet.

5. Heterocyclisch substituierte Amide der Formel I gemäß dem Anspruch 1, wobei
B Pyridin oder Phenyl und
R⁴ CONR⁹R¹⁰ bedeutet.

6. Heterocyclisch substituierte Amide der Formel I gemäß dem Anspruch 1, wobei
A Piperazin und
B Pyridin oder Phenyl und
R⁴ Wasserstoff bedeutet.

7. Heterocyclisch substituierte Amide der Formel I gemäß dem Anspruch 1, wobei
A Piperazin und
B Pyridin oder Phenyl und
R⁴ CONR⁹R¹⁰ bedeutet.

8. Heterocyclisch substituierte Amide der Formel I gemäß dem Anspruch 1, wobei
A Piperazin und
B ortho-substituiertes Pyridin oder Phenyl und
R⁴ Wasserstoff bedeutet.

9. Heterocyclisch subsütuierte Amide der Formel I gemäß dem Anspruch 1, wobei
A Piperazin und
B ortho-substituiertes Pyridin oder Phenyl und
R⁴ CONR⁹R¹⁰ bedeutet.

10. Heterocyclisch substituierte Amide der Formel I gemäß den Ansprüchen 1-8 als Arzneimittel.

11. Heterocyclisch substituierte Amide der Formel I gemäß den Ansprüchen 1-8 zur Verwendung als Inhibitoren von Cysteinproteasen.

12. Heterocyclisch substituierte Amide nach Anspruch 11 als Inhibitoren von Cysteinproteasen wie Calpaine, Cathepsine B und L.

13. Heterocyclisch substituierte Amide der Formel I gemäß den Ansprüchen 1-8 zur Verwendung in der Behandlung von Krankheiten, bei denen erhöhe Calpain-Aktivitäten auftreten.

14. Heterocyclisch substituierte Amide der Formel I gemäß den Ansprüchen 1-8 zur Verwendung in der Behandlung von neuro-degenerativen Krankheiten und neuronalen Schädigungen.

15. Heterocyclisch substituierte Amide nach Anspruch 14 zur Behandlung von solchen neuro-degenerativen Krankheiten und neuronalen Schädigungen, die durch Ischämie, Trauma oder Massenblutungen ausgelöst werden.

16. Heterocyclisch substituierte Amide nach Anspruch 14 zur Behandlung von Hirnschlag und Schädel-Himtrauma.

17. Heterocyclisch substituierte Amide nach Anspruch 14 zur Behandlung der Alzheimerschen Krankheit und der Huntington-Krankheit.

18. Heterocyclisch substituierte Amide der Formel I gemäß den Ansprüchen 1-8 zur Verwendung in der Behandlung von Schädigungen des Herzens nach cardialen Ischämien, Schädigungen und Reperfusion nach Gefäßverschlüssen, Schädigungen der Nieren nach renalen Ischämien, Skelettmuskelschädigungen, Muskeldystrophien, Schädigungen, die durch Proliferation der glatten Muskelzellen entstehen, coronarer Vasospasmus, cerebraler Vasospasmus, Katarakten der Augen und Restenose der Blutbahnen nach Angioplastie.

19. Heterocyclisch substituierte Amide der Formel I gemäß den Ansprüchen 1-8 zur Verwendung in der Behandlung von Tumoren und deren Metastasierung.

20. Heterocyclisch substituierte Amide der Formel I gemäß den Ansprüchen 1-8 zur Verwendung in der Behandlung von Krankheiten, bei denen erhöhte Interleukin-1-Spiegel auftreten.

21. Heterocyclisch substituierte Amide der Formel I gemäß den Ansprüchen 1-8 zur Verwendung in der Behandlung von Entzündungen und rheumatischen Erkrankungen.

22. Arzneimittelzubereitungen zur peroralen, parenteralen und intraperitonalen Anwendung, enthaltend pro Einzeldosis, neben den üblichen Arzneimittelhilftstoffen, mindestens eines heterocyclisch substituierten Amides I gemäß den Ansprüchen 1-8.

## Claims

1. Heterocyclically substituted amides of the general formula I 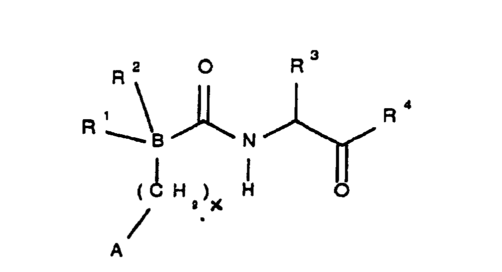 and their tautomeric and isomeric forms, enantiomeric and diastereomeric forms, as well as physiologically acceptable salts, wherein the variables have the following meaning:
A signifies piperazine, homopiperazine, hexahydroazepine piperidine and pyrrolidine, which can also carry a group R⁵, and
B represents a phenyl, pyridine, pyrimidine, pyrazine or pyridazine ring and
R¹ and R² independently of one another signify hydrogen, C₁-C₆ alkyl, branched or unbranched, O-C₁-C₆ alkyl, branched or unbranched, OH, Cl, F, Br, I, CF₃, NO₂, NH₂, CN, COOH, COO-C₁-C₄ alkyl, NHCO-C₁-C₄ alkyl, NHCO-phenyl, CONHR⁹, NHSO₂-C₁-C₄ alkyl, NHSO₂-phenyl, SO₂-C₁-C₄ alkyl and SO₂-phenyl, and R¹ and R² can be a -CH=CH-CH=CH- chain which can also carry one or two substituents R⁶, and
R³ signifies C₁-C₆ alkyl, branched or unbranched, which can also carry an S-CH₃ group, cyclohexyl, cyclopentyl, cycloheptyl, phenyl, pyridyl, pyrimidyl, pyridazyl, pyrazyl, indolyl, thienyl or naphthyl ring, wherein the rings are substituted with a maximum of two groups R⁷, and R⁷ signifies hydrogen, C₁-C₄ alkyl, branched or unbranched, O-C₁-C₄ alkyl, OH, Cl, F, Br, I, CF₃, NO₂, NH₂, CN, COOH, COO-C₁-C₄ alkyl, CONHR⁹, NHCO-C₁-C₄ alkyl, NHCO-phenyl, NHSO₂-C₁-C₄ alkyl, NHSO₂-phenyl, SO₂-C₁-C₄ alkyl and SO₂-phenyl, and
R⁴ signifies hydrogen, -COR⁸, wherein R⁸ can be -OR⁹, and NR⁹R¹⁰, and
R⁵ can be hydrogen, C₁-C₆ alkyl, branched or unbranched, which can also carry a substituent R¹¹, or R⁵ can be phenyl, pyridyl, pyrimidyl, pyridazyl, pyrazinyl, pyrazyl, naphthyl, thienyl, piperidinyl, pyrrolidinyl, imidazyl ring, which can also carry one or two substituents R⁶, and
R⁶ signifies hydrogen, C₁-C₆ alkyl, branched or unbranched, O-C₁-C₆ alkyl, branched or unbranched, OH, Cl, F, Br, I, CF₃, NO₂, NH₂, CN, COOH, COO-C₁-C₄ alkyl and C₁-C₄ alkyl-NR⁹R¹³ or two groups R⁶ can represent a bridge OC(R⁹)₂O, and
R⁹ signifies hydrogen, C₁-C₆ alkyl, branched and unbranched, and
R¹⁰ signifies hydrogen, C₁-C₆ alkyl, branched or unbranched, which can also be substituted with a phenyl ring, which can also carry a group R¹², and with 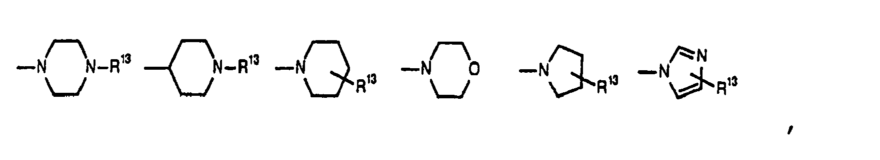 and
R¹¹ can be a phenyl, pyridyl, pyrimidyl, naphthyl, thienyl, furyl, pyridazyl, pyrazinyl, pyrazyl, pyrrolyl, imidazyl ring, which can also carry one or two substituents R⁶, and
R¹² signifies hydrogen, C₁-C₆ alkyl, branched or unbranched, O-C₁-C₆ alkyl, branched or unbranched, OH, Cl, F, Br, I, CF₃, NO₂, NH₂, CN, COOH, COO-C₁-C₄ alkyl, and
R¹³ signifies hydrogen, a C₁-C₄ alkyl chain and C₀-C₄ alkylphenyl, wherein the phenyl ring can also carry one or two groups R¹², and
x signifies a number 0, 1 and 2.

2. Heterocyclically substituted amides of formula I according to claim 1, wherein
R⁴ signifies hydrogen.

3. Heterocyclically substituted amides of formula I according to claim 1, wherein
R⁴ signifies CONR⁹R¹⁰.

4. Heterocyclically substituted amides of formula I according to claim 1, wherein
B signifies pyridine or phenyl and
R⁴ signifies hydrogen.

5. Heterocyclically substituted amides of formula I according to claim 1, wherein
B signifies pyridine or phenyl and
R⁴ signifies CONR⁹R¹⁰.

6. Heterocyclically substituted amides of formula I according to claim 1, wherein
A signifies piperazine and
B signifies pyridine or phenyl and
R⁴ signifies hydrogen.

7. Heterocyclically substituted amides of formula I according to claim 1, wherein
A signifies piperazine and
B signifies pyridine or phenyl and
R⁴ signifies CONR⁹R¹⁰.

8. Heterocyclically substituted amides of formula I according to claim 1, wherein
A signifies piperazine and
B signifies ortho-substituted pyridine or phenyl and
R⁴ signifies hydrogen.

9. Heterocyclically substituted amides of formula I according to claim 1, wherein
A signifies piperazine and
B signifies ortho-substituted pyridine or phenyl and
R⁴ signifies CONR⁹R¹⁰.

10. Heterocyclically substituted amides of formula I according to claims 1 - 8 as medicaments.

11. Heterocyclically substituted amides of formula I according to claims 1 - 8 for use as inhibitors of cysteine proteases.

12. Heterocyclically substituted amides according to claim 11 as inhibitors of cysteine proteases such as calpains and cathepsins B and L.

13. Heterocyclically substituted amides of formula I according to claims 1 - 8 for use in the treatment of diseases in which increased calpain activities occur.

14. Heterocyclically substituted amides of formula I according to claims 1 - 8 for use in the treatment of neurodegenerative diseases and neuronal damage.

15. Heterocyclically substituted amides according to claim 14 for the treatment of neurodegenerative diseases and neuronal damage caused by ischaemia, trauma or massive haemorrhages.

16. Heterocyclically substituted amides according to claim 14 for the treatment of stroke and craniocerebral trauma.

17. Heterocyclically substituted amides according to claim 14 for the treatment of Alzheimer's disease and Huntington's disease.

18. Heterocyclically substituted amides of formula I according to claims 1 - 8 for use in the treatment of damage to the heart following cardiac ischaemias, damage and reperfusion following vascular obliterations, damage to the kidneys following renal ischaemias, skeletal muscle damage, muscular dystrophies, damage caused by proliferation of the smooth muscle cells, coronary vasospasm, cerebral vasospasm, cataracts of the eyes and restenosis of the blood vessels after angioplasty.

19. Heterocyclically substituted amides of formula I according to claims 1 - 8 for use in the treatment of tumours and the metastases thereof.

20. Heterocyclically substituted amides of formula I according to claims 1 - 8 for use in the treatment of diseases in which increased interleukin-1 levels occur.

21. Heterocyclically substituted amides of formula I according to claims 1 - 8 for use in the treatment of inflammations and rheumatic diseases.

22. Medicament preparations for peroral, parenteral and intraperitonal use, containing per individual dose, in addition to the conventional medicament auxiliaries, at least one heterocyclically substituted amide I according to claims 1 - 8.

## Revendications

1. Amides substitués par voie hétérocyclique de la formule générale I 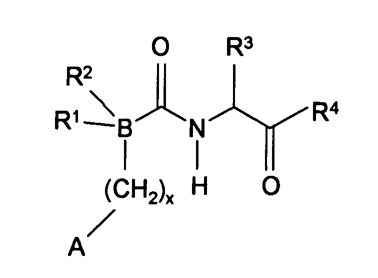 et leurs formes tautomères et isomères, formes énantiomères et diastéréomères ainsi que sels physiologiquement compatibles, dans lesquels les variables ont les significations suivantes :
A représente la pipérazine, l'homopipérazine, l'hexahydroazépine, la pipéridine et la pyrrolidine, lesquels peuvent encore porter un reste R⁵ et
B représente un noyau phényle, pyridine, pyrimidine, pyrazine ou pyridazine et
R¹ et R² représentent indépendamment l'un de l'autre un atome d'hydrogène, un groupe alkyle en C₁-C₆, ramifié ou non ramifié, O-(alkyle en C₁-C₆), ramifié ou non ramifié, OH, Cl, F, Br, J, CF₃, NO₂, NH₂, CN, COOH, COO-(alkyle en C₁-C₄), NHCO-(alkyle en C₁-C₄), NHCO-phényle, CONHR⁹, NHSO₂-(alkyle en C₁-C₄), NHSO₂-phényle, SO₂-(alkyle en C₁-C₄) et SO₂-phényle et R¹ et R² peuvent être une chaîne -CH=CH-CH=CH-, laquelle peut encore porter un ou deux substituants R⁶, et
R³ représente un groupe alkyle en C₁-C₆, ramifié ou non ramifié, lequel peut encore porter un reste S-CH₃, cyclohexyle, cyclopentyle, cycloheptyle, un noyau phényle, pyridyle, pyrimidyle, pyridazyle, pyrazyle, indolyle, thiényle ou naphtyle, les noyaux pouvant être substitués avec au maximum deux restes R⁷ et R⁷ représente un atome d'hydrogène, un groupe alkyle en C₁-C₄, ramifié ou non ramifié, O-(alkyle en C₁-C₄), OH, Cl, F, Br, I, CF₃, NO₂, NH₂, CN, COOH, COO-(alkyle en C₁-C₄), CONHR⁹, NHCO-(alkyle en C₁-C₄), NHCO-phényle, NHSO₂-(alkyle en C₁-C₄), NHSO₂-phényle, SO₂-(alkyle en C₁-C₄) et SO₂-phényle, et
R⁴ représente un atome d'hydrogène, -COR⁸, R⁸ pouvant être -OR⁹ et -NR⁹R¹⁰ et
R⁵ est un atome d'hydrogène, un groupe alkyle en C₁-C₆, ramifié ou non ramifié, lequel peut encore porter un substituant R¹¹ ou R⁵ peut être un noyau phényle, pyridyle, pyrimidyle, pyridazyle, pyrazinyle, pyrazyle, naphtyle, thiényle, pipéridinyle, pyrrolidinyle, imidazyle, lequel peut encore porter un ou deux substituants R⁶, et
R⁶ représente un atome d'hydrogène, un groupe alkyle en C₁-C₆, ramifié ou non ramifié, O-(alkyle en C₁-C₆), ramifié ou non ramifié, OH, Cl, F, Br, I, CF₃, NO₂, NH₂, CN, COOH, COO-(alkyle en C₁-C₄), et (alkyle en C₁-C₄)-NR⁹R¹³ ou deux restes R⁶ peuvent représenter un pont OC(R⁹)₂O et
R⁹ représente un atome d'hydrogène, un groupe alkyle en C₁-C₆, ramifié et non ramifié et
R¹⁰ est un atome d'hydrogène, un groupe alkyle en C₁-C₆, ramifié ou non ramifié, lequel peut encore être substitué avec un noyau phényle, qui peut encore porter un reste R¹², et avec 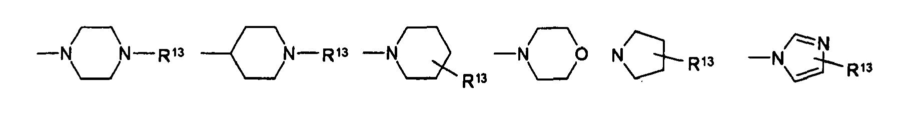 et
R¹¹ peut être un noyau phényle, pyridyle, pyrimidyle, naphtyle, thiényle, furyle, pyridazyle, pyrazinyle, pyrazyle, pyrrolyle, imidazyle, lequel peut encore porter un ou deux substituants R⁶, et
R¹² représente un atome d'hydrogène, un groupe alkyle en C₁-C₆, ramifié ou non ramifié, O-(alkyle en C₁-C₆), ramifié ou non ramifié, OH, Cl, F, Br, I, CF₃, NO₂, NH₂, CN, COOH, COO-(alkyle en C₁-C₄), et
R¹³ représente un atome d'hydrogène, une chaîne alkyle en C₁-C₄ et (alkyle en C₀-C₄)phényle, le noyau phényle pouvant encore porter un ou deux restes R¹² et
X est un nombre égal à 0, 1 et 2.

2. Amides substitués par voie hétérocyclique de la formule I selon la revendication 1, dans lesquels
R⁴ est un atome d'hydrogène.

3. Amides substitués par voie hétérocyclique de la formule I selon la revendication 1, dans lesquels
R⁴ représente CONR⁹R¹⁰,

4. Amides substitués par voie hétérocyclique de la formule I selon la revendication 1, dans lesquels
B représente le groupe pyridine ou phényle et
R⁴ représente un atome d'hydrogène.

5. Amides substitués par voie hétérocyclique de la formule I selon la revendication 1, dans lesquels
B représente le groupe pyridine ou phényle et
R⁴ représente CONR⁹R¹⁰.

6. Amides substitués par voie hétérocyclique de la formule I selon la revendication 1, dans lesquels
A représente le groupe pipérazine et
B représente le groupe pyridine ou phényle et
R⁴ représente un atome d'hydrogène.

7. Amides substitués par voie hétérocyclique de la formule I selon la revendication 1, dans lesquels
A représente le groupe pipérazine et
B représente le groupe pyridine ou phényle et
R⁴ représente CONR⁹R¹⁰.

8. Amides substitués par voie hétérocyclique de la formule I selon la revendication 1, dans lesquels
A représente le groupe pipérazine et
B représente le groupe pyridine ou phényle ortho-substitué et
R⁴ représente un atome d'hydrogène.

9. Amides substitués par voie hétérocyclique de la formule I selon la revendication 1, dans lesquels
A représente le groupe pipérazine et
B représente le groupe pyridine ou phényle ortho-substitué et
R⁴ représente CONR⁹R¹⁰.

10. Amides substitués par voie hétérocyclique de la formule I selon les revendications 1-8 en tant que médicaments.

11. Amides substitués par voie hétérocyclique de la formule I selon les revendications 1-8 pour une utilisation comme inhibiteurs de cystéineprotéases.

12. Amides substitués par voie hétérocyclique selon la revendication 11 en tant qu'inhibiteurs de cystéineprotéases comme la calpaïne, la cathepsine B et L.

13. Amides substitués par voie hétérocyclique de la formule I selon les revendications 1-8 pour une utilisation dans le traitement de maladies dans lesquelles apparaissent des activités élevées de calpaïne.

14. Amides substitués par voie hétérocyclique de la formule I selon les revendications 1-8 pour une utilisation dans le traitement de maladies neurodégénératives et de lésions neuronales.

15. Amides substitués par voie hétérocyclique selon la revendication 14 pour un traitement de maladies neurodégénératives et de lésions neuronales qui sont provoquées par des ischémies, des traumatismes ou des hémorragies.

16. Amides substitués par voie hétérocyclique selon la revendication 14 pour un traitement de l'attaque cérébrale et de traumatismes encéphalocraniens.

17. Amides substitués par voie hétérocyclique selon la revendication 14 pour le traitement de la maladie d'Alzheimer et de la maladie de Huntington.

18. Amides substitués par voie hétérocyclique de la formule I selon les revendications 1-8 pour une utilisation dans le traitement de lésions cardiaques après des ischémies cardiales, de lésions et de reperfusion après des obstructions vasculaires, de lésions rénales après des ischémies rénales, de lésions musculaires du squelette, de dystrophies musculaires, de lésions qui sont produites par la prolifération des cellules musculaires lisses, de vasospasmes coronaires, de vasospasmes cérébraux, de cataractes des yeux et de resténoses des circuits sanguins après une angioplastie.

19. Amides substitués par voie hétérocyclique de la formule I selon les revendications 1-8 pour une utilisation dans le traitement de tumeurs et de leurs métastases.

20. Amides substitués par voie hétérocyclique de la formule I selon les revendications 1-8 pour une utilisation dans le traitement de maladies dans lesquelles apparaissent des teneurs élevées en Interleukine-1.

21. Amides substitués par voie hétérocyclique de la formule I selon les revendications 1-8 pour une utilisation dans le traitement d'inflammations et de maladies rhumatismales.

22. Préparations de médicaments destinés à une application perorale, parentérale et intrapéritonale contenant, par dose individuelle, en plus des auxiliaires classiques de médicaments au moins un amide I substitué par voie hétérocyclique selon les revendications 1-8.
